(19) Europäisches Patentamt
European Patent Office
Office européen des brevets

(11) Veröffentlichungsnummer: **0 015 505**
**B1**

(12) # EUROPÄISCHE PATENTSCHRIFT

(45) Veröffentlichungstag der Patentschrift: **08.08.84**

(21) Anmeldenummer: **80100991.1**

(22) Anmeldetag: **28.02.80**

(51) Int. Cl.³: **C 07 C 103/26,** C 07 C 103/85, C 07 C 103/38, C 07 C 125/065, C 07 C 127/15, C 07 C 127/19, C 07 C 147/06, C 07 C 147/14, C 07 C 149/18, C 07 D 207/325, C 07 D 209/08

(54) **Derivate des 3-Amino-1,2-propandiols, Verfahren zu ihrer Herstellung und pharmazeutische Präparate enthaltend solche Verbindungen und Verwendung von letzteren.**

(30) Priorität: **01.03.79 CH 2037/79**

(43) Veröffentlichungstag der Anmeldung: **17.09.80 Patentblatt 80/19**

(45) Bekanntmachung des Hinweises auf die Patenterteilung: **08.08.84 Patentblatt 84/32**

(84) Benannte Vertragsstaaten: **AT BE CH DE FR GB IT LU NL SE**

(56) Entgegenhaltungen:
**EP - A - 0 014 951**
**DE - A - 2 357 849**
**DE - B - 2 032 642**

**Chemical Abstracts 75 (1971) 47094x**
***Arzneimittelforschung* 15 (1965), 1079-1080**

(73) Patentinhaber: **CIBA-GEIGY AG, Postfach, CH-4002 Basel (CH)**

(72) Erfinder: **Ostermayer, Franz, Dr., Am Hang 5, CH-4125 Riehen (CH)**
Erfinder: **Zimmermann, Markus, Dr., Steinbrecheweg 8, CH-4125 Riehen (CH)**

(74) Vertreter: **Zumstein, Fritz sen., Dr. et al, *Bräuhausstrasse 4, D-8000 München 2 (DE)***

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 015 505 B1

BUNDESDRUCKEREI BERLIN

**Beschreibung**

Die Erfindung betrifft neue Derivate des 3-Amino-1,2-propandiols, Verfahren zu ihrer Herstellung, pharmazeutische Präparate, die solche Verbindungen enthalten und ihre Verwendung zur Herstellung von pharmazeutischen Präparaten.

Die erfindungsgemäßen neuen Derivate des 3-Amino-1,2-propandiols entsprechen der Formel

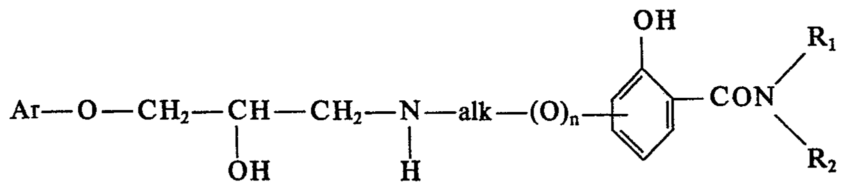

(I)

in welcher Ar gegebenenfalls substituiertes Aryl, n die Zahl 0 oder 1 und alk Alkylen mit 2—5 Kohlenstoffatomen bedeuten, wobei das Stickstoffatom und das Sauerstoffatom oder, falls n für Null steht, der Phenylrest, durch mindestens zwei Kohlenstoffatome voneinander getrennt sind, und $R_1$ und $R_2$ unabhängig voneinander je Wasserstoff oder Niederalkyl mit bis zu 7 Kohlenstoffatomen, oder zusammen Niederalkylen, Oxaniederalkylen, Thianiederalkylen oder Azaniederalkylen mit jeweils bis zu 7 Kohlenstoffatomen, oder N-Niederalkyl-azaniederalkylen mit jeweils bis zu 7 Kohlenstoffatomen im Niederalkyl- und Azaniederalkylenteil darstellen.

Zum Gegenstand der Erfindung gehören auch Salze dieser Verbindungen.

Ein Arylrest Ar ist ein carbocyclischer oder heterocyclischer Arylrest. Vorzugsweise ist Ar mono- oder bicyclisches carbocyclisches Aryl, oder mono- oder bicyclisches, über ein Ringkohlenstoffatom gebundenes, höchstens zwei Stickstoffatome und/oder ein Sauerstoff- oder Schwefelatom als Ringglieder enthaltendes Heteroaryl, welche Reste unsubstituiert oder ein- oder mehrfach, vorzugsweise höchstens dreifach, substituiert sein können, wobei in bicyclischen Resten Ar der nicht direkt mit der Äthergruppe verbundene Ring mindestens partiell hydriert sein und in diesem Fall insbesondere durch Oxo substituiert sein kann. Ar ist insbesondere Phenyl, ferner Naphthyl, wie 1-Naphthyl oder teilweise gesättigtes Naphthyl, wie 1,2,3,4-Tetrahydro-5-naphthyl, weiter als mono- oder bicyclisches Heteroaryl insbesondere Pyridyl, z. B. 2-, 3- und 4-Pyridyl, Pyridazinyl, z. B. 3-Pyridazinyl, Pyrimidinyl, z. B. 2- oder 4-Pyrimidinyl, Pyrazinyl, z. B. 2-Pyrazinyl, Thienyl, z. B. 3-Thienyl, Thiazolyl, z. B. 2-Thiazolyl, Thiadiazolyl, z. B. 1,2,4-Thiadiazol-3- oder 5-yl oder 1,2,5-Thiadiazol-3-yl, Indolyl, z. B. Indol-4-yl, Chinolinyl, z. B. 2-Chinolinyl, Isochinolinyl, z. B. 1-Isochinolinyl, 2-Oxo-4-benzimidazolyl, Naphthyridinyl, z. B. 1,8-Naphthyridin-2-yl, oder Benzofuranyl, z. B. 4- oder 5-Benzofuranyl.

Als Substituenten von Resten Ar, z. B. der oben näher definierten und der vorstehend als Beispiele genannten Reste, können beispielsweise gegebenenfalls, insbesondere in untenstehend angegebener Weise, substituiertes Niederalkyl, Niederalkenyl oder Niederalkoxy mit jeweils bis zu 7 Kohlenstoffatomen, weiter Niederalkenyloxy, Niederalkinyl, Niederalkinyloxy mit jeweils bis zu 7 Kohlenstoffatomen, Cyan und/oder Nitro und/oder direkt oder an vorgenanntes Niederalkyl, Niederalkenyl oder Niederalkoxy mit jeweils bis zu 7 Kohlenstoffatomen gebundenes Niederalkanoyl mit bis zu 7 Kohlenstoffatomen, verestertes oder amidiertes Carboxyl insbesondere Niederalkoxycarbonyl mit bis zu 7 Kohlenstoffatomen im Niederalkoxyteil, bzw. gegebenenfalls substituiertes Carbamoyl, z. B. Carbamoyl, Niederalkylcarbamoyl mit bis zu 7 Kohlenstoffatomen im Niederalkylteil, Diniederalkylcarbamoyl mit bis zu 7 Kohlenstoffatomen in jedem Niederalkylteil, oder (Hydroxyniederalkyl)-carbamoyl mit bis zu 7 Kohlenstoffatomen im (Hydroxyniederalkyl)-teil, Niederalkylsulfinyl, Niederalkylsulfonyl mit jeweils bis zu 7 Kohlenstoffatomen, Sulfamoyl oder Niederalkylsulfamoyl mit bis zu 7 Kohlenstoffatomen, oder direkt oder an vorgenanntes Niederalkyl mit bis zu 7 Kohlenstoffatomen, oder in höherer als der 1-Stellung an vorgenanntes Niederalkoxy mit bis zu 7 Kohlenstoffatomen gebundenes Halogen, gegebenenfalls veräthertes oder verestertes Hydroxy, wie Hydroxy oder, als nicht direkt gebundener Substituent wiederum Niederalkoxy mit bis zu 7 Kohlenstoffatomen, ferner Phenylniederalkoxy mit bis zu 7 Kohlenstoffatomen im Niederalkoxyteil, bzw. Niederalkanoyloxy mit bis zu 7 Kohlenstoffatomen, veräthertes Mercapto, wie Niederalkylthio mit bis zu 7 Kohlenstoffatomen, gegebenenfalls substituiertes Amino, wie Amino, Niederalkylamino mit bis zu 7 Kohlenstoffatomen, Diniederalkylamino mit bis zu 7 Kohlenstoffatomen in jedem Niederalkylteil, Alkylen- oder Oxaalkylenamino, Pyrrol-1-yl, Acylamino, wie Niederalkanoylamino mit bis zu 7 Kohlenstoffatomen, oder Niederalkoxycarbonylamino mit bis zu 7 Kohlenstoffatomen im Niederalkoxyteil, gegebenenfalls, insbesondere durch ein oder zwei Niederalkyl, durch Hydroxyniederalkyl mit jeweils bis zu 7 Kohlenstoffatomen oder Cyacloalkyl mit 5 bis 7 Ringgliedern substituiertes Ureido, oder Niederalkylsulfonylamino mit bis zu 7 Kohlenstoffatomen vorliegen.

Die im Zusammenhang mit der vorliegenden Beschreibung mit »nieder« bezeichneten Reste und Verbindungen enthalten bis 7 und in erster Linie bis 4 Kohlenstoffatome.

Die in der Aufzählung von Substituenten des Restes Ar verwendeten Allgemeinbegriffe können z. B. folgende spezifische Bedeutungen haben: Niederalkyl mit bis zu 7 Kohlenstoffatomen ist z. B. Methyl, Äthyl, n-Propyl, Isopropyl, n-Butyl, Isobutyl oder tert.-Butyl; substituiertes Niederalkyl mit bis zu 7

Kohlenstoffatomen ist insbesondere entsprechendes Methyl oder 1- oder 2-substituiertes Äthyl. Niederalkenyl mit bis zu 7 Kohlenstoffatomen ist z. B. Vinyl, Allyl, 2- oder 3-Methallyl oder 3,3-Dimethylallyl, und substituiertes Niederalkenyl mit bis zu 7 Kohlenstoffatomen insbesondere 2-substituiertes Vinyl oder 3-substituiertes Allyl. Niederalkoxy mit bis zu 7 Kohlenstoffatomen ist z. B. Methoxy, Äthoxy, n-Propyloxy, Isopropyloxy, n-Butyloxy oder Isobutyloxy, und substituiertes Niederalkoxy mit bis zu 7 Kohlenstoffatomen z. B. substituiertes Methoxy- oder 1- oder 2-substituiertes Äthoxy. Niederalkenyloxy mit bis zu 7 Kohlenstoffatomen ist z. B. Allyloxy, 2- oder 3-Methallyloxy oder 3,3-Dimethylallyloxy. Niederalkinyl mit bis zu 7 Kohlenstoffatomen ist z. B. Propargyl, und Niederalkinyloxy mit bis zu 7 Kohlenstoffatomen insbesondere Propargyloxy. Niederalkanoyl mit bis zu 7 Kohlenstoffatomen ist z. B. Acetyl, Propionyl oder Butyryl. Niederalkoxycarbonyl mit bis zu 7 Kohlenstoffatomen im Niederalkoxyteil ist z. B. Methoxycarbonyl oder Äthoxycarbonyl. Niederalkyl- oder Diniederalkylcarbamoyl mit bis zu 7 Kohlenstoffatomen in jedem Niederalkylteil ist z. B. Methylcarbamoyl, Dimethylcarbamoyl, Äthylcarbamoyl oder Diäthylcarbamoyl, und Hydroxyniederalkylcarbamoyl mit bis zu 7 Kohlenstoffatomen im Hydroxyniederalkylteil z. B. (2-Hydroxyäthyl)-carbamoyl. Niederalkylsulfinyl mit bis zu 7 Kohlenstoffatomen ist z. B. Methyl- oder Äthylsulfinyl, Niederalkylsulfonyl mit bis zu 7 Kohlenstoffatomen z. B. Methyl-, Äthyl- oder Propylsulfonyl, und Niederalkylsulfamoyl mit bis zu 7 Kohlenstoffatomen z. B. Methyl-, Äthyl- oder Isopropylsulfamoyl. Halogen ist Brom oder Jod und vorzugsweise Fluor oder Chlor. Phenylniederalkoxy mit bis zu 7 Kohlenstoffatomen im Niederalkoxyteil ist z. B. Benzyloxy oder 1- oder 2-Phenyläthoxy, und Niederalkanoyloxy mit bis zu 7 Kohlenstoffatomen z. B. Formyloxy, Acetoxy, Propionyloxy, Butyryloxy oder Isobutyryloxy. Niederalkylthio mit bis zu 7 Kohlenstoffatomen ist z. B. Methylthio, n-Propylthio oder Isopropylthio. Niederalkylamino und Diniederalkylamino mit bis zu 7 Kohlenstoffatomen in jedem Niederalkylteil ist z. B. Methylamino, Äthylamino, Dimethylamino oder Diäthylamino. Alkylenamino und Oxaalkylenamino ist z. B. Pyrrolidino oder Piperidino bzw. Morpholino. Niederalkanoylamino mit bis zu 7 Kohlenstoffatomen ist z. B. Acetylamino oder Butyrylamino, und Niederalkoxycarbonylamino mit bis zu 7 Kohlenstoffatomen im Niederalkoxyteil z. B. Methoxycarbonylamino oder Äthoxycarbonylamino. Durch ein oder zwei Niederalkyl mit bis zu 7 Kohlenstoffatomen, durch Hydroxyniederalkyl mit bis zu 7 Kohlenstoffatomen oder durch Cycloalkyl mit 5 bis 7 Ringgliedern substituiertes Ureido ist z. B. 3-Methylureido, 3,3-Dimethylureido, 3-(2-Hydroxyäthyl)-ureido bzw. 3-Cyclohexylureido. Niederalkylsulfonylamino mit bis zu 7 Kohlenstoffatomen ist z. B. Äthylsulfonylamino und insbesondere Methylsulfonylamino.

Wie oben angegeben, können Substituenten von Ar aus einem der vorgenannten Reste, der nicht direkt, sondern über Niederalkyl oder Niederalkoxy mit jeweils bis zu 7 Kohlenstoffatomen oder gegebenenfalls auch Niederalkenyl mit bis zu 7 Kohlenstoffatomen gebunden ist, bestehen. Nachstehend werden eine Auswahl solcher Substituenten allgemein und spezifisch als Beispiele genannt. Niederalkanoylalkyl mit jeweils bis zu 7 Kohlenstoffatomen im Niederalkanoyl- und Alkylteil ist z. B. 2-Oxopropyl (Acetonyl) oder 3-Oxobutyl, Niederalkanoylniederalkenyl mit jeweils bis zu 7 Kohlenstoffatomen im Niederalkanoyl- und Niederalkenylteil z. B. 3-Oxo-1-butenyl, und Niederalkanoylalkoxy mit jeweils bis zu 7 Kohlenstoffatomen im Niederalkanoyl- und Alkoxyteil z. B. 2-Oxopropoxy (Acetonyloxy) oder 3-Oxobutoxy. Gegebenenfalls substituiertes Carbamoylniederalkyl mit bis zu 7 Kohlenstoffatomen im Niederalkylteil ist z. B. Carbamoylmethyl, oder [(Hydroxyniederalkyl)-carbamoyl)]-niederalkyl mit jeweils bis zu 7 Kohlenstoffatomen im (Hydroxyniederalkyl)- und Niederalkylteil, wie [(2-Hydroxyäthyl)-carbamoyl]-methyl. Niederalkoxycarbonylniederalkoxy mit bis zu 7 Kohlenstoffatomen in jedem Niederalkoxyteil ist z. B. Äthoxycarbonylmethoxy. Gegebenenfalls substituiertes Carbamoylniederalkoxy mit bis zu 7 Kohlenstoffatomen im Niederalkoxyteil ist z. B. Carbamoylniederalkoxy mit bis zu 7 Kohlenstoffatomen im Niederalkoxyteil, wie Carbamoylmethoxy, oder [(Hydroxyniederalkyl)-carbamoyl])-niederalkoxy mit jeweils bis zu 7 Kohlenstoffatomen im (Hydroxyniederalkyl)- und Niederalkoxyteil, wie [(2-Hydroxyäthyl)-carbamoyl]-methoxy. Halogenniederalkyl mit bis zu 7 Kohlenstoffatomen ist insbesondere Halogenmethyl, z. B. Trifluormethyl. Hydroxyniederalkyl mit bis zu 7 Kohlenstoffatomen ist vorzugsweise Hydroxymethyl oder 1- und in erster Linie 2-Hydroxyäthyl. Niederalkoxyniederalkyl mit jeweils bis zu 7 Kohlenstoffatomen im Niederalkoxy- und Niederalkylteil ist vorzugsweise Niederalkoxymethyl mit bis zu 7 Kohlenstoffatomen im Niederalkoxyteil oder 1- und in erster Linie 2-Niederalkoxyäthyl mit bis zu 7 Kohlenstoffatomen im Niederalkoxyteil, z. B. Methoxymethyl, Äthoxymethyl, 2-Methoxyäthyl oder 2-Äthoxyäthyl. Niederalkoxyniederalkoxy mit jeweils bis zu 7 Kohlenstoffatomen im Niederalkoxyteil ist insbesondere 2-Niederalkoxyäthoxy mit bis zu 7 Kohlenstoffatomen im Niederalkoxyteil, wie 2-Methoxyäthoxy oder 2-Äthoxyäthoxy. Niederalkylthioniederalkoxy mit jeweils bis zu 7 Kohlenstoffatomen im Niederalkylthio- und Niederalkoxyteil ist z. B. 2-Methylthioäthoxy oder 2-Äthylthioäthoxy. Acylaminoniederalkyl mit bis zu 7 Kohlenstoffatomen im Niederalkylteil ist z. B. Niederalkanoylaminoniederalkyl mit jeweils bis zu 7 Kohlenstoffatomen im Niederalkanoyl- und Niederalkylteil, insbesondere Niederalkanoylaminomethyl mit bis zu 7 Kohlenstoffatomen im Niederalkanoylteil oder 1- und in erster Linie 2-Niederalkanoylamino-äthyl mit bis zu 7 Kohlenstoffatomen im Niederalkanoylteil, z. B. Acetylaminomethyl, 2-Acetylamino-äthyl oder 2-Propionylaminoäthyl, oder Niederalkoxycarbonylaminoniederalkyl mit jeweils bis zu 7 Kohlenstoffatomen im Niederalkoxy- und Niederalkylteil, insbesondere Niederalkoxycarbonylaminomethyl mit bis zu 7 Kohlenstoffatomen im Niederalkoxyteil oder 1- und in erster Linie 2-Niederalkoxycarbonylamino-äthyl mit bis zu 7 Kohlenstoffatomen im Niederalkoxyteil z. B. Methoxycarbonylaminomethyl, 2-Methoxycarbo-

nylamino-äthyl oder 2-Äthoxycarbonylamino-äthyl. Acylaminoniederalkoxy mit bis zu 7 Kohlenstoffatomen im Niederalkoxyteil ist z. B. Niederalkanoylaminoniederalkoxy mit jeweils bis zu 7 Kohlenstoffatomen im Niederalkanoyl- und Niederalkoxyteil, insbesondere 2-Niederalkanoylaminoäthoxy mit bis zu 7 Kohlenstoffatomen im Niederalkanoylteil, z. B. 2-(Acetylamino)-äthoxy oder Niederalkoxycarbonylaminoniederalkoxy mit bis zu 7 Kohlenstoffatomen in jedem Niederalkoxyteil, insbesondere 2-(Niederalkoxycarbonyl)-äthoxy mit bis zu 7 Kohlenstoffatomen im Niederalkoxyteil, z. B. 2-(Methoxycarbonylamino)-äthoxy oder 2-(Äthoxycarbonylamino)-äthoxy.

Alkylen alk kann geradkettig oder verzweigt sein und ist z. B. 1,2-Äthylen, 1,2-, 2,3-, oder 1,3-Propylen, 1,4- oder 2,4-Butylen, 2-Methyl-2,4-butylen, oder 1,1-Dimethyläthylen.

$R_1$ und $R_2$ in der Bedeutung von Niederalkyl sind beispielsweise Propyl, Isopropyl, Butyl, Isobutyl, Sek.-butyl, Pentyl, Isopentyl, Neopentyl, Hexyl oder Heptyl, und vor allem Methyl oder Äthyl. Zusammen mit dem Stickstoffatom der Amidgruppe sind $R_1$ und $R_2$ als Niederalkylen beispielsweise 1-Aziridinyl, 1-Azetidinyl, Pyrrolidino, Piperidino, Hexahydro-1H-azepin-1-yl, als Oxaniederalkylen z. B. Morpholino, als Thianiederalkylen z. B. Thiomorpholino, als Azaniederalkylen z. B. 1-Piperazinyl oder Hexahydro-1H-1,4-diazepin-1-yl, wobei die beiden letztgenannten Gruppen, entsprechend der Bedeutung N-Niederalkylazaniederalkylen für $R_1$ und $R_2$ in 4-Stellung, d. h. in der Iminogruppe, z. B. durch Niederalkyl, wie Methyl, Äthyl, Propyl, Isopropyl, Butyl oder Isobutyl, substituiert sein können.

Das die Amid- und die Hydroxygruppe tragende Phenyl kann in beliebiger Stellung mit dem restlichen Molekül verbunden sein, vorzugsweise ist letzteres in der 4-Stellung des genannten Phenyls, d. h. in der para-Stellung zur Amidgruppe, und vor allem in der 5-Stellung des genannten Phenyls, (d. h. in para-Stellung zur Hydroxygruppe) gebunden.

Die neuen Verbindungen können in Form ihrer Salze wie ihrer Säureadditionssalze und in erster Linie ihrer pharmazeutisch verwendbaren, nicht-toxischen Säureadditionssalze vorliegen. Geeignete Salze sind z. B. solche mit anorganischen Säuren, wie Halogenwasserstoffsäuren, z. B. Chlorwasserstoffsäure oder Bromwasserstoffsäure, Schwefelsäure, oder Phosphorsäure, oder mit organischen Säuren, wie aliphatischen, cycloaliphytischen, aromatischen oder heterocyclischen Carbon- oder Sulfonsäuren, z. B. Ameisen-, Essig-, Propion-, Bernstein-, Glykol-, Milch-, Äpfel-, Wein-, Zitronen-, Malein-, Hydroxymalein-, Brenztrauben-, Fumar-, Benzoe-, 4-Aminobenzoe-, Anthranil-, 4-Hydroxybenzoe-, Salicyl-, Embon-, Methansulfon-, Äthansulfon-, 2-Hydroxyäthansulfon-, Äthylensulfon, Toluolsulfon-, Naphthalinsulfon- oder Sulfanilsäure, oder mit anderen sauren organischen Stoffen, wie Ascorbinsäure.

Die neuen Verbindungen besitzen wertvolle pharmakologische Eigenschaften. Insbesondere wirken sie in spezifischer Weise auf $\beta$-adrenerge Rezeptoren. Dieser Wirkung liegt als gemeinsame Eigenschaft der Verbindungen der Formel I die Affinität zu diesen Rezeptoren zugrunde, die sich bei fehlender oder sehr geringer stimulierender Eigenwirkung als reine Blockade, bei geringer bis mäßiger stimulierender Eigenwirkung als Blockade mit gleichzeitiger ISA, d. h. intrinsic sympathomimetic activity, und bei stärkerer Eigenwirkung als eigentliche Stimulierung der $\beta$-adrenergen Rezeptoren äußert. Die Grenzen zwischen $\beta$-Rezeptoren-Blockern ohne oder mit mehr oder weniger ausgeprägter ISA sind fließend, ebenso die therapeutischen Anwendungsbereiche dieser Verbindungstypen, denen keine besondern Strukturmerkmale zugeordnet werden können, während ausgeprägte $\beta$-Rezeptoren-stimulierende und zugleich cardioselektive Wirksamkeit insbesondere bei solchen Verbindungen der Formel I festgestellt wird, in denen Ar einen Hydroxyphenylrest darstellt. Von den übrigen Verbindungen der Formel I, den $\beta$-Rezeptoren-Blockern ohne oder mit ISA, zeigen solche mit einem Substituenten in p-Stellung, wie insbesondere 1-[2-(3-Carbamoyl-4-hydroxy-phenoxy)-äthylamino]-3-[4-(2-methoxyäthoxy)-phenoxy]-2-propanol eine mehr oder weniger ausgeprägte Cardioselektivität. Diese ist dagegen weniger deutlich oder fehlend bei Verbindungen mit ortho-Substitution im weiten Sinne, d. h. Vorliegen eines einzelnen Substituenten oder eines ankondensierten Ringes in ortho-Stellung zur Äthergruppe. Andererseits besitzen solche Verbindungen zusätzlich $\alpha$-Rezeptoren-blockierende Eigenschaften. Als Beispiel einer solchen Verbindung sei insbesondere das 1-(2-Allyloxy-phenoxy)-3-[2-(3-carbamoyl-4-hydroxyphenoxy)-äthylamino]-2-propanol genannt.

Die vorstehenden Angaben betreffend pharmakologische Eigenschaften beruhen auf den Resultaten von entsprechenden pharmakologischen Versuchen in üblichen Testverfahren. So zeigen die neuen $\beta$-blockierenden Verbindungen eine Hemmung der durch Isoproterenol induzierten Tachykardie am isolierten Meerschweinchenherzen in einem Konzentrationsbereich von etwa 0,001 µg/ml bis etwa 1 µg/ml und an der narkotisierten Katze in einem Dosenbereich von etwa 0,001 mg/kg bis etwa 1 mg/kg bei intravenöser Verabreichung. Im gleichen Dosenbereich hemmen die $\beta$-blockierenden Verbindungen der Formel I an der narkotisierten Katze auch die durch elektrische Reizung der sympathischen Nerven induzierte Zunahme der Herzfrequenz. Die Hemmung der durch Isoproterenol induzierten Vasodilatation an der narkotisierten Katze mit Perfusion der Arteria femoralis ist mit cardioselektiven Verbindungen der Formel I bei intravenöser Verabreichung in einem Dosenbereich von etwa 0,1 mg/kg bis etwa 10 mg/kg und mit nicht-cardioselektiven Verbindungen in einem Dosenbereich von 0,001 bis 1 mg/kg nachweisbar. Die ISA der $\beta$-blockierenden Verbindungen der Formel I ergibt sich aus der Zunahme der basalen Herzfrequenz an der narkotisierten, mit Reserpin vorbehandelten Katze bei intravenöser Verabreichung in einem Dosenbereich von 0,001 bis 1 mg/kg. Die neuen $\beta$-blockierenden Verbindungen bewirken ebenfalls in einem Dosenbereich von etwa 0,01 mg/kg bis etwa 10 mg/kg i. v.

eine Senkung des arteriellen Blutdrucks bei der narkotisierten Katze. Die zusätzliche $\alpha$-blockierende Wirksamkeit der nicht-cardioselektiven $\beta$-Rezeptoren-Blocker, welche z. B. eine blutdrucksenkende Wirkung begünstigen kann, geht z. B. aus der Antagonisierung der durch Noradrenalin induzierten Kontraktion des isolierten Vas deferens der Ratte durch solche Verbindungen in einer Konzentration von 0,01—3 µg/ml hervor. Die neuen $\beta$-blockierenden Verbindungen der Formel I können als, gegebenenfalls cardioselektive, $\beta$-Rezeptoren-Blocker, z. B. zur Behandlung von Angina pectoris und Herzrhythmusstörungen, sowie als blutdrucksenkende Mittel verwendet werden.

Die $\beta$-stimulierenden Verbindungen der Formel I, wie das 1-(4-Hydroxy-phenoxy)-3-[2-(carbamoyl-4-hydroxyphenoxy)-1-methyläthylamino]-2-propanol, z. B. dasjenige Racemat, dessen neutrales Fumarat bei 195—198° schmilzt, bewirken eine Zunahme von Herzfrequenz und myocardialer Kontraktionskraft am isolierten Meerschweinchenvorhof in einem Konzentrationsbereich von etwa 0,0001 bis 0,1 µg/ml und eine Zunahme von Herzfrequenz und maximaler Druckanstiegsgeschwindigkeit im linken Ventrikel (dp/dt max.) an der narkotisierten Katze in einem Dosenbereich von etwa 0,0001 bis etwa 0,1 mg/kg i. v.. Die zur Senkung des arteriellen Blutdrucks an der narkotisierten Katze erforderlichen Dosen liegen demgegenüber, entsprechend einem Dosenbereich von etwa 0,001 bis etwa 1 mg/kg i. v., höher, d. h. die neuen Verbindungen stimulieren spezifisch die cardialen $\beta$-Rezeptoren ($\beta_1$-Rezeptoren) im Vergleich zu den $\beta$-Rezeptoren in den Blutgefäßen ($\beta_2$-Rezeptoren) und unterscheiden sich dadurch qualitativ deutlich von Isoproterenol, welches die $\beta$-Rezeptoren des Herzens und der Blutgefäße etwa gleich stark stimuliert. Die neuen Verbindungen können somit als positiv inotrop wirkende Mittel, insbesondere als Cardiotonica zur Behandlung der Herzmuskelinsuffizienz, allein oder in Kombination mit anderen Präparaten, wie Herzglykosiden, ferner zur Behandlung von bestimmten Störungen des Herzrhythmus verwendet werden.

Die Verbindungen der allgemeinen Formel I können auch als wertvolle Zwischenprodukte für die Herstellung anderer wertvoller, insbesondere pharmazeutisch wirksamer, Verbindungen verwendet werden.

Die Erfindung betrifft insbesondere Verbindungen der Formel I, worin Ar mono- oder bicyclisches carbocyclisches Aryl oder mono- oder bicyclisches, über ein Ringkohlenstoffatom gebundenes, höchstens zwei Stickstoffatome und/oder ein Sauerstoff- oder Schwefelatom als Ringglieder enthaltendes Heteroaryl bedeutet, welche Reste unsubstituiert oder ein- oder mehrfach, vorzugsweise höchstens dreifach, substituiert sein können, wobei als Substituenten gegebenenfalls, insbesondere in untenstehend angegebener Weise, gegebenenfalls substituiertes Niederalkyl, Niederalkenyl oder Niederalkoxy mit jeweils bis zu 7 Kohlenstoffatomen, weiter Niederalkenyloxy, Niederalkinyl, Niederalkinyloxy mit jeweils bis zu 7 Kohlenstoffatomen, Cyan und/oder Nitro, und/oder direkt oder an vorgenanntes Niederalkyl, Niederalkenyl oder Niederalkoxy mit jeweils bis zu 7 Kohlenstoffatomen gebundenes Niederalkanoyl mit bis zu 7 Kohlenstoffatomen, verestertes oder amidiertes Carboxyl, insbesondere Niederalkoxycarbonyl mit bis zu 7 Kohlenstoffatomen im Niederalkoxyteil, Carbamoyl, Niederalkylcarbamoyl mit bis zu 7 Kohlenstoffatomen im Niederalkylteil, oder (Hydroxyniederalkyl)-carbamoyl mit bis zu 7 Kohlenstoffatomen im (Hydroxyniederalkyl)-teil, Niederalkylsulfinyl, Niederalkylsulfonyl mit jeweils bis zu 7 Kohlenstoffatomen, Sulfamoyl oder Niederalkylsulfamoyl mit bis zu 7 Kohlenstoffatomen, und/oder direkt oder an vorgenanntes Niederalkyl mit bis zu 7 Kohlenstoffatomen oder in höherer als der 1-Stellung an vorgenanntes Niederalkoxy mit bis zu 7 Kohlenstoffatomen gebundenes Halogen, gegebenenfalls veräthertes oder verestertes Hydroxy, wie Hydroxy, oder als nicht direkt gebundener Substituent, wiederum Niederalkoxy mit bis zu 7 Kohlenstoffatomen, ferner Phenylniederalkoxy mit bis zu 7 Kohlenstoffatomen im Niederalkoxyteil, z. B. Benzyloxy, oder Niederalkenyloxy mit bis zu 7 Kohlenstoffatomen, veräthertes Mercapto, wie Niederalkylthio mit bis zu 7 Kohlenstoffatomen, gegebenenfalls substituiertes Amino, wie Amino, Niederalkylamino mit bis zu 7 Kohlenstoffatomen, Diniederalkylamino mit bis zu 7 Kohlenstoffatomen in jedem Niederalkylteil, Alkylen- oder Oxaalkylenamino, z. B. Pyrrolidino, Piperidino oder Morpholino, Pyrrol-1-yl, Acylamino, wie Niederalkanoylamino mit bis zu 7 Kohlenstoffatomen oder Niederalkoxycarbonylamino mit bis zu 7 Kohlenstoffatomen im Niederalkoxyteil, gegebenenfalls durch Niederalkyl oder Hydroxyniederalkyl mit jeweils bis zu 7 Kohlenstoffatomen, oder Cycloalkyl mit 5 bis 7 Ringgliedern substituiertes Ureido, oder Niederalkylsulfonylamino mit bis zu 7 Kohlenstoffatomen vorliegen können, und in bicyclischen Resten Ar der nicht direkt mit der Äthergruppe verbundene Ring auch mindestens partiell hydriert und in diesem Fall auch durch Oxo substituiert sein kann, und n für die Zahl 0 oder 1 steht, und alk einen Alkylenrest mit 2 bis 4 Kohlenstoffatomen bedeutet, wobei das Stickstoffatom und das Sauerstoffatom oder, falls n für 0 steht, der Phenylrest durch 2 bis 3 Kohlenstoffatome voneinander getrennt sind, und $R_1$ und $R_2$ die unter der Formel I angegebene Bedeutung haben, jedoch vorzugsweise für Wasserstoff oder Niederalkyl mit bis zu 7 Kohlenstoffatomen, z. B. Methyl oder Äthyl, stehen, oder zusammen mit dem Stickstoffatom der Amidgruppe Morpholino oder Alkylenamino mit 5 bis 6 Ringgliedern bilden, wie Pyrrolidino oder Piperidino, und Salze insbesondere Säureadditionssalze, vor allem pharmazeutisch verwendbare, nicht-toxische Säureadditionssalze davon.

Die Erfindung betrifft vorzugsweise Verbindungen der Formel I, worin Ar mono- oder bicyclisches carbocyclisches Aryl oder mono- oder bicyclisches, über ein Ringkohlenstoffatom gebundenes, höchstens zwei Stickstoffatome und/oder ein Sauerstoff- oder Schwefelatom als Ringglieder enthaltendes Heteroaryl bedeutet, welche Reste unsubstituiert oder ein- bis dreifach substituiert sein können,

wobei als Substituenten gegebenenfalls in untenstehend angegebener Weise substituiertes Niederalkyl, Niederalkenyl- oder Niederalkoxy mit jeweils bis zu 7 Kohlenstoffatomen, weiter Niederalkenyloxy, Niederalkinyl, Niederalkinyloxy mit jeweils bis zu 7 Kohlenstoffatomen, Cyan und/oder Nitro und/oder direkt oder an vorgenanntes Niederalkyl, Niederalkenyl oder Niederalkoxy mit jeweils bis zu 7 Kohlenstoffatomen gebundenes Niederalkanoyl mit bis zu 7 Kohlenstoffatomen, Niederalkoxycarbonyl mit bis zu 7 Kohlenstoffatomen im Niederalkoxyteil, Carbamoyl, Niederalkylcarbamoyl oder (Hydroxyniederalkyl)-carbamoyl mit jeweils bis zu 7 Kohlenstoffatomen im Niederalkyl- bzw. (Hydroxyniederalkyl)-teil, Niederalkylsulfinyl oder Niederalkylsulfonyl mit jeweils bis zu 7 Kohlenstoffatomen, Sulfamoyl oder Niederalkylsulfamoyl mit bis zu 7 Kohlenstoffatomen, und/oder direkt oder an vorgenanntes Niederalkyl mit bis zu 7 Kohlenstoffatomen, oder in höherer als der 1-Stellung an vorgenanntes Niederalkoxy mit bis zu 7 Kohlenstoffatomen gebundenes Halogen, Hydroxy oder, als nicht direkt gebundener Substituent, wiederum Niederalkoxy mit bis zu 7 Kohlenstoffatomen, Phenylniederalkoxy mit bis zu 7 Kohlenstoffatomen im Niederalkoxyteil, z. B. Benzyloxy, Niederalkanoyloxy oder Niederalkylthio mit jeweils bis zu 7 Kohlenstoffatomen, Amino, Niederalkylamino mit bis zu 7 Kohlenstoffatomen, Diniederalkylamino mit jeweils bis zu 7 Kohlenstoffatomen in den Niederalkylteilen, Alkylenamino oder Oxaalkylenamino, z. B. Pyrrolidino, Piperidino oder Morpholino, Pyrrol-1-yl, Niederalkanoylamino mit bis zu 7 Kohlenstoffatomen, oder Niederalkoxycarbonylamino mit bis zu 7 Kohlenstoffatomen im Niederalkoxyteil, gegebenenfalls durch Niederalkyl oder Hydroxyniederalkyl mit jeweils bis zu 7 Kohlenstoffatomen, oder Cycloalkyl mit 5 bis 7 Ringgliedern substituiertes Ureido, oder Niederalkylsulfonylamino mit bis zu 7 Kohlenstoffatomen vorliegen können, und in bicyclischen Resten Ar der nicht direkt mit der Äthergruppe verbundene Ring auch mindestens partiell hydriert und in diesem Fall auch durch Oxo substituiert sein kann, und n die Zahl 0 oder 1, und alk einen Alkylenrest mit 2 bis 4 Kohlenstoffatomen bedeutet, wobei das Stickstoffatom und das Sauerstoffatom, oder, falls n für 0 steht, der Phenylrest durch 2 bis 3 Kohlenstoffatome voneinander getrennt sind, und $R_1$ und $R_2$ die unter Formel I angegebene Bedeutung haben, jedoch vorzugsweise für Wasserstoff oder Niederalkyl mit bis zu 7 Kohlenstoffatomen, insbesondere Methyl oder Äthyl stehen, oder zusammen mit dem Stickstoffatom der Amidgruppe Pyrrolidino, Piperidino oder Morpholino darstellen, und Salze, insbesondere Säureadditionssalze, vor allem pharmazeutisch verwendbare nicht-toxische Säureadditionssalze davon.

Die Erfindung betrifft besonders Verbindungen der Formel I, worin Ar Phenyl, Naphthyl oder 1,2,3,4-Tetrahydro-5-naphthyl bedeutet, welche Reste unsubstituiert oder ein- bis dreifach substituiert sein können, wobei als Substituenten gegebenenfalls in untenstehend angegebener Weise substituiertes Niederalkyl, Niederalkenyl oder Niederalkoxy mit jeweils bis zu 7 Kohlenstoffatomen, weiter Niederalkenyloxy, Niederalkinyloxy mit jeweils bis zu 7 Kohlenstoffatomen und/oder Cyan und/oder direkt oder an vorgenanntes Niederalkyl, Niederalkenyl oder Niederalkoxy mit jeweils bis zu 7 Kohlenstoffatomen gebundenes Niederalkanoyl mit bis zu 7 Kohlenstoffatomen, Niederalkoxycarbonyl mit bis zu 7 Kohlenstoffatomen im Niederalkoxyteil, Carbamoyl, Niederalkylcarbamoyl oder (Hydroxyniederalkyl)-carbamoyl mit jeweils bis zu 7 Kohlenstoffatomen im Niederalkyl bzw. (Hydroxyniederalkyl)-teil, Niederalkylsulfinyl oder Niederalkylsulfonyl mit jeweils bis zu 7 Kohlenstoffatomen, Sulfamoyl oder Niederalkylsulfamoyl mit bis zu 7 Kohlenstoffatomen, und/oder direkt oder an vorgenanntes Niederalkyl mit bis zu 7 Kohlenstoffatomen oder in höherer als der 1-Stellung an vorgenanntes Niederalkoxy mit bis zu 7 Kohlenstoffatomen gebundenes Halogen, Hydroxy oder, als nicht direkt gebundener Substituent, wiederum Niederalkoxy mit bis zu 7 Kohlenstoffatomen, Phenylniederalkoxy mit bis zu 7 Kohlenstoffatomen im Niederalkoxyteil, z. B. Benzyloxy, Niederalkanoyloxy oder Niederalkylthio mit jeweils bis zu 7 Kohlenstoffatomen, Amino, Niederalkylamino mit bis zu 7 Kohlenstoffatomen, Diniederalkylamino mit jeweils bis zu 7 Kohlenstoffatomen in den Niederalkylteilen, Alkylenamino oder Oxaalkylenamino mit jeweils 5 bis 6 Ringgliedern, z. B. Pyrrolidino, Piperidino oder Morpholino, Pyrrol-1-yl, Niederalkanoylamino mit bis zu 7 Kohlenstoffatomen, Niederalkoxycarbonylamino mit bis zu 7 Kohlenstoffatomen im Niederalkoxyteil, gegebenenfalls durch Niederalkyl mit bis zu 7 Kohlenstoffatomen oder Cycloalkyl mit 5 bis 7 Ringgliedern substituiertes Ureido, oder Niederalkylsulfonylamino mit bis zu 7 Kohlenstoffatomen vorliegen können, und n die Zahl 0 oder 1 und Alk einen Alkylenrest mit 2 bis 4 Kohlenstoffatomen bedeutet, wobei das Stickstoffatom und das Sauerstoffatom, oder, falls n für 0 steht, der Phenylrest durch 2 bis 3 Kohlenstoffatome voneinander getrennt sind, und $R_1$ und $R_2$ unabhängig voneinander je Wasserstoff oder Niederalkyl mit bis zu 7 Kohlenstoffatomen, insbesondere Methyl oder Äthyl, darstellen, oder zusammen mit dem Stickstoffatom der Amidgruppe Morpholino bilden, und Salze, insbesondere Säureadditionssalze, in erster Linie pharmazeutisch verwendbare, nicht-toxische Säureadditionssalze davon.

Die Erfindung betrifft vor allem Verbindungen der Formel I, worin Ar Phenyl, welches unsubstituiert oder ein- bis dreifach substituiert sein kann, wobei als Substituenten gegebenenfalls in untenstehend angegebener Weise substituiertes Niederalkyl oder Niederalkoxy mit jeweils bis zu 7 Kohlenstoffatomen, weiter Niederalkenyl, Niederalkenyloxy oder Niederalkinyloxy mit jeweils bis zu 7 Kohlenstoffatomen, und/oder Cyan, und/oder direkt oder an vorgenanntes Niederalkyl oder Niederalkoxy mit jeweils bis zu 7 Kohlenstoffatomen gebundenes Niederalkanoyl mit bis zu 7 Kohlenstoffatomen, Niederalkoxycarbonyl mit bis zu 7 Kohlenstoffatomen im Niederalkoxyteil, Carbamoyl, Niederalkylcarbamoyl oder (Hydroxyniederalkyl)-carbamoyl mit jeweils bis zu 7 Kohlenstoffatomen im Niederalkyl-

bzw. (Hydroxyniederalkyl)-teil, Niederalkylsulfinyl oder Niederalkylsulfonyl mit jeweils bis zu 7 Kohlenstoffatomen, und/oder direkt oder an vorgenanntes Niederalkyl mit bis zu 7 Kohlenstoffatomen oder in höherer als der 1-Stellung an vorgenanntes Niederalkoxy mit bis zu 7 Kohlenstoffatomen gebundenes Halogen, Hydroxy oder, als nicht direkt gebundener Substituent, wiederum Niederalkoxy mit bis zu 7 Kohlenstoffatomen, ferner Phenylniederalkoxy mit bis zu 7 Kohlenstoffatomen im Niederalkoxyteil, z. B. Benzyloxy, Niederalkylthio mit bis zu 7 Kohlenstoffatomen, Alkylen- oder Oxaalkylenamino z. B. Pyrrolidino, Piperidino oder Morpholino, Pyrrol-1-yl, Niederalkanoylamino mit bis zu 7 Kohlenstoffatomen, Niederalkoxycarbonylamino mit bis zu 7 Kohlenstoffatomen im Niederalkoxyteil, oder gegebenenfalls durch Niederalkyl mit bis zu 7 Kohlenstoffatomen substituiertes Ureido vorliegen können, und n die Zahl 0 oder 1, und alk einen Alkylenrest mit 2 bis 4 Kohlenstoffatomen bedeutet, wobei das Stickstoffatom und das Sauerstoffatom oder, falls n für 0 steht, der Phenylrest durch 2 bis 3 Kohlenstoffatome voneinander getrennt sind, und $R_1$ und $R_2$ unabhängig je Wasserstoff oder Niederalkyl mit bis zu 7 Kohlenstoffatomen, jedoch vorzugsweise Wasserstoff oder Methyl darstellen; und Salze, insbesondere Säureadditionssalze, in erster Linie pharmazeutisch verwendbare, nicht-toxische Säureadditionssalze davon.

Die Erfindung betrifft in erster Linie Verbindungen der Formel I, worin Ar Phenyl, welches unsubstituiert oder ein- bis dreifach, substituiert sein kann, wobei als Substituenten gegebenenfalls in untenstehend angegebener Weise substituiertes Niederalkyl oder Niederalkoxy mit jeweils bis zu 7 Kohlenstoffatomen, weiter Niederalkenyl, Niederalkenyloxy oder Niederalkinyloxy mit jeweils bis zu 7 Kohlenstoffatomen, und/oder Cyan, und/oder direkt oder an vorgenanntes Niederalkyl oder Niederalkoxy mit jeweils bis zu 7 Kohlenstoffatomen gebundenes Niederalkanoyl mit bis zu 7 Kohlenstoffatomen, Carbamoyl, Niederalkylcarbamoyl oder (Hydroxyniederalkyl)-carbamoyl mit jeweils bis zu 7 Kohlenstoffatomen im Niederalkyl- bzw. (Hydroxyniederalkyl)-teil, Niederalkylsulfinyl oder Niederalkylsulfonyl mit jeweils bis zu 7 Kohlenstoffatomen, und/oder direkt oder an vorgenanntes Niederalkyl mit bis zu 7 Kohlenstoffatomen, oder in höherer als der 1-Stellung an vorgenanntes Niederalkoxy mit bis zu 7 Kohlenstoffatomen gebundenes Fluor oder Chlor, Hydroxy, oder, als nicht direkt gebundener Substituent wiederum Niederalkoxy mit bis zu 7 Kohlenstoffatomen, ferner Phenylniederalkoxy mit bis zu 7 Kohlenstoffatomen im Niederalkoxyteil, z. B. Benzyloxy, Niederalkylthio mit bis zu 7 Kohlenstoffatomen, Pyrrol-1-yl, Niederalkanoylamino mit bis zu 7 Kohlenstoffatomen, Niederalkoxycarbonylamino mit bis zu 7 Kohlenstoffatomen im Niederalkoxyteil, oder Ureido vorliegen können, und n die Zahl 1, und alk einen Alkylenrest mit 2 bis 4 Kohlenstoffatomen bedeutet, wobei das Stickstoffatom und das Sauerstoffatom durch 2 Kohlenstoffatome voneinander getrennt sind, $R_1$ und $R_2$ Wasserstoff darstellen, und der die Amid- und die Hydroxygruppe tragende Phenylrest vorzugsweise in seiner 4-Stellung und vor allem in seiner 5-Stellung mit dem restlichen Molekül verbunden ist, und Salze, insbesondere Säureadditionssalze, in erster Linie pharmazeutisch annehmbare, nicht-toxische Säureadditionssalze davon.

Die neuen Verbindungen der Formel I werden in an sich bekannter Weise hergestellt. Man kann sie z. B. erhalten, indem man eine Verbindung der Formel

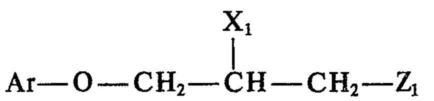

(II)

mit einer Verbindung der Formel

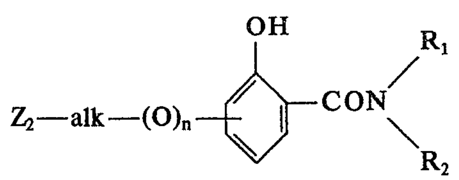

(III)

worin eine der Gruppen $Z_1$ und $Z_2$ eine reaktionsfähige veresterte Hydroxygruppe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, und Ar, n, alk, $R_1$ und $R_2$ obige Bedeutung haben, umsetzt und, wenn erwünscht, eine so erhältliche Verbindung in eine andere Verbindung der Formel I umwandelt und/oder, wenn erwünscht, eine erhaltene freie Verbindung in ein Salz oder ein erhaltenes Salz in eine freie Verbindung überführt, und/oder, wenn erwünscht, ein erhaltenes Isomerengemisch in die Isomeren oder ein erhaltenes Racemat in die Antipoden auftrennt.

Eine reaktionsfähige veresterte Hydroxygruppe $Z_1$ bzw. $Z_2$ ist eine, durch eine starke Säure, insbesondere eine starke anorganische Säure, wie eine Halogenwasserstoffsäure, insbesondere Chlor-, Brom- oder Jodwasserstoffsäure, oder Schwefelsäure, oder eine starke organische Säure, insbesondere eine starke organische Sulfonsäure, wie eine aliphatische oder aromatische Sulfonsäure, z. B. Methansulfonsäure, 4-Methylphenylsulfonsäure oder 4-Bromphenylsulfonsäure, veresterte Hydroxygruppe, und stellt in erster Linie Halogen, z. B. Chlor, Brom oder Jod, oder aliphatisch oder aromatisch

substituiertes Sulfonyloxy, z. B. Methylsulfonyloxy oder 4-Methylphenylsulfonyloxy dar.

Die obige Reaktion wird in an sich bekannter Weise durchgeführt, wobei man, besonders bei Verwendung eines Ausgangsmaterials mit einer reaktionsfähigen veresterten Hydroxygruppe, vorteilhafterweise in Gegenwart eines basischen Mittels, wie einer anorganischen Base, z. B. eines Alkalimetall- oder Erdalkalimetallcarbonats oder -hydroxyds, oder eines organischen basischen Mittels, wie eines Alkalimetall-niederalkanolats, und/oder eines Überschusses des basischen Reaktionsteilnehmers und üblicherweise in Gegenwart eines Lösungsmittels oder Llösungsmittelgemisches und, wenn notwendig, unter Kühlen oder Erwärmen, z. B. in einem Temperaturbereich von etwa — 20° C bis etwa + 150° C, in einem offenen oder geschlossenen Gefäß und/oder in einer Inertgasatmosphäre, z. B. in einer Stickstoffatmosphäre, arbeitet.

Ausgangsstoffe der Formel II sind bekannt oder können in an sich bekannter Weise hergestellt werden.

Ausgangsstoffe der Formel III können z. B. durch Umsetzung eines Hydroxysalicylamids mit einem der Bedeutung von alk entsprechenden Dihalogenalkan, etwa einem Chlorbrom- oder Dibromalkan in Gegenwart eines alkalischen Kondensationsmittels, wie einem Alkalicarbonat, erhalten werden. Diese Umsetzungen werden in üblicher Weise vorgenommen, wobei an den Hydroxygruppen stehende Schutzgruppen gleichzeitig oder, wie nachfolgend beschrieben, anschließend abgespalten werden.

Die Verbindungen der Formel I können weiterhin hergestellt werden, indem man in einer Verbindung der Formel

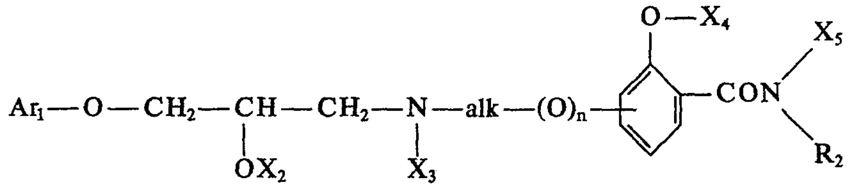

(IV)

worin $Ar_1$ die Bedeutung von Ar hat oder einen Rest Ar bedeutet, der durch 1 bis 2 in Hydroxy überführbare Gruppen substituiert ist, $X_2$, $X_3$ und $X_4$ jeweils Wasserstoff oder einen durch Wasserstoff ersetzbaren Substituenten bedeuten und $X_5$ für $R_1$ steht, oder $X_2$ und $X_3$ und/oder $X_4$ und $X_5$ zusammen einen zweiwertigen, durch zwei Wasserstoffatome ersetzbaren Rest bedeuten, mit der Maßgabe, daß mindestens einer der Reste $X_2$, $X_3$ und $X_4$ von Wasserstoff verschieden ist, oder mindestens $Ar_1$ einen Rest Ar bedeutet, der durch 1 bis 2 in Hydroxy überführbare Gruppen substituiert ist, oder mindestens $X_2$ und $X_3$ zusammen oder $X_4$ und $X_5$ zusammen einen zweiwertigen, durch zwei Wasserstoffatome ersetzbaren Rest darstellen, oder in einem Salz davon die von Wasserstoff verschiedenen der Gruppe $X_2$, $X_3$, und $X_4$, bzw. $X_2$ und $X_3$ zusammen oder $X_4$ und $X_5$ zusammen durch Wasserstoffatome ersetzt, und/oder in einem Rest $Ar_1$ vorhandenes substituiertes Hydroxy in freies Hydroxy überführt, und wenn erwünscht, die anschließend an das erste Verfahren genannten zusätzlichen Verfahrensschritte durchführt.

Die Abspaltung der Gruppen $X_2$, $X_3$ oder $X_4$ oder jeweils $X_2$ und $X_3$ oder $X_4$ und $X_5$ zusammen, sowie der in einem Rest $Ar_1$ vorhandenen Hydroxy-Substituenten wird mittels Solvolyse, wie Hydrolyse, Alkoholyse oder Acidolyse, oder mittels Reduktion, einschließlich Hydrogenolyse vorgenommen.

Eine besonders geeignete, abspaltbare Gruppe $X_3$ oder $X_4$, oder eine Hydroxy-Schutzgruppe in einem Rest $Ar_1$ ist in erster Linie eine hydrogenolytisch abspaltbare $\alpha$-Arylniederalkylgruppe, wie eine gegebenenfalls substituierte 1-Polyphenylniederalkyl- oder 1-Phenylniederalkylgruppe, worin Substituenten, insbesondere des Phenylteils, z. B. Niederalkyl, wie Methyl, oder Niederalkoxy wie Methoxy sein können, und in erster Linie Benzyl. Eine Gruppe $X_3$ und insbesondere $X_2$ und $X_4$ sowie Hydroxy-Schutzgruppen in einem Rest $Ar_1$ können auch einen solvolytisch, wie hydrolytisch oder acidolytisch, ferner einen reduktiv, einschließlich hydrogenolytisch, abspaltbaren Rest, insbesondere einen entsprechenden Acylrest, wie den Acylrest einer organischen Carbonsäure, z. B. Niederalkanoyl, wie Acetyl, oder Aroyl, wie Benzoyl, ferner den Acylrest eines Halbesters der Kohlensäure, wie Niederalkoxycarbonyl, z. B. Methoxycarbonyl, Äthoxycarbonyl oder tert.-Butyloxycarbonyl, 2-Halogenniederalkoxycarbonyl, z. B. 2,2,2-Trichloräthoxycarbonyl oder 2-Jodäthoxycarbonyl, gegebenenfalls substituiertes 1-Phenylniederalkoxycarbonyl, z. B. Benzyloxycarbonyl oder Diphenylmethoxycarbonyl, oder Aroylmethoxycarbonyl, z. B. Phenacycloxycarbonyl, ferner eine gegebenenfalls substituierte 1-Polyphenyl-niederalkylgruppe, worin Substituenten, in erster Linie des Phenylteils, z. B. die oben gegebene Bedeutung haben, und in erster Linie Trityl darstellen.

Ein durch $X_2$ und $X_3$ und/oder $X_4$ und $X_5$ zusammen gebildeter, abspaltbarer Rest ist in erster Linie eine hydrogenolytisch abspaltbare Gruppe, wie gegebenenfalls substituiertes 1-Phenyl-niederalkyliden, worin Substituenten, insbesondere des Phenylteils, z. B. Niederalkyl oder Niederalkoxy sein können, und insbesondere Benzyliden, sowie solvolytisch, insbesondere hydrolytisch spaltbare Gruppen, wie Niederalkyliden, z. B. Methylen oder Isopropyliden, oder 1-Phenyl-niederalkyliden, dessen Phenylteil gegebenenfalls durch Niederalkyl, wie Methyl oder Niederalkoxy, wie Methoxy, substituiert ist, insbesondere Benzyliden, oder Cycloalkyliden, z. B. Cyclopentyliden oder Cyclohexyliden.

In der Form von Salzen verwendbare Ausgangsstoffe werden in erster Linie in der Form von Säureadditionssalzen, z. B. mit Mineralsäuren, sowie mit organischen Säuren verwendet.

Hydrogenolytisch abspaltbare Reste $X_2$, $X_3$ und/oder $X_4$, insbesondere gegebenenfalls substituierte 1-Phenylniederalkylgruppen, ferner auch geeignete Acylgruppen, wie gegebenenfalls substituiertes 1-Phenylniederalkoxycarbonyl, sowie durch die Gruppen $X_2$ und $X_3$ sowie $X_4$ und $X_5$ zusammen gebildete, gegebenenfalls substituierte 1-Phenylniederalkylidengruppen, sowie in einem Rest $Ar_1$ vorhandene Hydroxy-Schutzgruppen dieser Art können durch Behandeln mit katalytisch aktiviertem Wasserstoff, z. B. mit Wasserstoff in Gegenwart eines Nickelkatalysators, wie Raney-Nickel, oder eines geeigneten Edelmetallkatalysators abgespalten werden.

Hydrolytisch abspaltbare Gruppen $X_2$, $X_3$ und/oder $X_4$, wie Acylreste von organischen Carbonsäuren, z. B. Niederalkanoyl, und von Halbestern der Kohlensäure, z. B. Niederalkoxycarbonyl, ferner z. B. Tritylreste, sowie durch die Reste $X_2$ und $X_3$ und/oder $X_4$ und $X_5$ zusammen gebildete Niederalkyliden-, 1-Phenyl-niederalkyliden- oder Cycloalkylidengruppen sowie in einem Rest $Ar_1$ vorhandene Hydroxy-Schutzgruppen dieser Art können je nach Art solcher Reste durch Behandeln mit Wasser unter sauren oder basischen Bedingungen, z. B. in Gegenwart einer Mineralsäure, wie Chlorwasserstoff- oder Schwefelsäure, oder eines Alkalimetall- oder Erdalkalimetallhydroxids oder -carbonats oder eines Amins, wie Isopropylamin, abgespalten werden.

Acidolytisch abspaltbare Reste $X_2$, $X_3$ und/oder $X_4$ und/oder Hydroxy-Schutzgruppen in einem Rest $Ar_1$ sind insbesondere gewisse Acylreste von Halbestern der Kohlensäure, wie z. B. tert.-Niederalkoxycarbonyl oder gegebenenfalls substituierte Diphenylmethoxycarbonylreste, ferner auch der tert.-Niederalkylrest; solche Reste können durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig, in Gegenwart eines aktivierenden Mittels, wie Anisol), sowie mit Ameisensäure abgespalten werden.

Unter reduktiv abspaltbaren Resten $X_2$, $X_3$ und/oder $X_4$ und/oder Hydroxy-Schutzgruppen in einem Rest $Ar_1$ werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl oder Arylmethoxycarbonyl, die z. B. beim Behandeln mit einem reduzierenden Schwermetall, wie Zink, oder mit einem reduzierenden Schwermetallsalz, wie einem Chrom(II)salz, z. B. -chlorid oder -acetat, üblicherweise in Gegenwart einer organischen Carbonsäure, wie Ameisensäure oder Essigsäure, und von Wasser abgespalten werden können.

Schutzgruppen, welche an in einem Rest $Ar_1$ gegebenenfalls vorhandenen Hydroxygruppen stehen, entsprechen den vorhin genannten und mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmaßnahme abgespalten werden.

Die obigen Reaktionen werden üblicherweise in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z. B. in einem offenen oder geschlossenen Gefäß und/oder in der Atmosphäre eines Inertgases, z. B. Stickstoff.

Die Ausgangsstoffe der Formel IV lassen sich analog den oben beschriebenen Verfahrensmodifikationen erhalten, z. B. durch Behandeln einer Verbindung der Formel $Ar_1OH$ oder einem Salz davon mit einer Verbindung der Formel

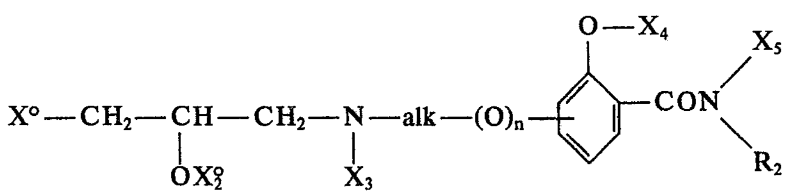

(IVa)

worin $X_2^o$ für die Gruppe $X_2$ steht, wobei mindestens eine der Gruppen $X_3$ und $X_2^o$ von Wasserstoff verschieden ist, und $X^o$ eine reaktionsfähige veresterte Hydroxygruppe bedeutet, oder $X_2^o$ und $X^o$ zusammen eine Kohlenstoff-Sauerstoff-Bindung darstellen, oder worin $X_3$ und $X_2^o$ zusammen einen abspaltbaren, durch zwei, mit dem Sauerstoff- bzw. Stickstoffatom verbundene Wasserstoffatome ersetzbaren Rest darstellen und $X^o$ eine reaktionsfähige veresterte Hydroxygruppe bedeutet, oder durch Behandeln einer Verbindung der Formel

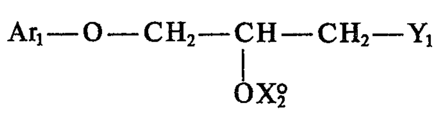

(IVb)

mit einer Verbindung der Formel

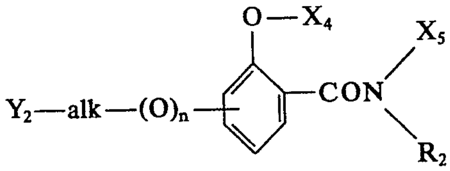

(IVc)

worin $X_2^\circ$ die oben für $X_2$ angegebene Bedeutung hat, und eine der Gruppen $Y_1$ und $Y_2$ eine reaktionsfähige veresterte Hydroxygruppe darstellt, und die andere für die Gruppe der Formel $-NH(X_3)$ steht, worin $X_3$ die oben gegebene Bedeutung hat, mit der Maßgabe, daß mindestens eine der Gruppen $X_3$ und $X_2$ von Wasserstoff verschieden ist, oder worin $X_2^\circ$ und $Y_1$ eine Sauerstoff-Kohlenstoff-Bindung bilden und $Y_2$ für die Gruppe der Formel $-NH(X_3)$ steht und $X_3$ von Wasserstoff verschieden ist. Die obigen Reaktionen werden in an sich bekannter Weise, z. B. wie unter dem ersten erfindungsgemäßen Verfahren beschrieben, durchgeführt.

Man kann ferner z. B. die durch Umsetzung einer Verbindung der Formel

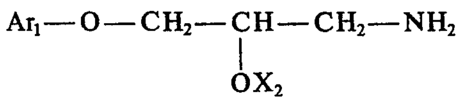

(IVd)

mit einer Carbonylverbindung der Formel

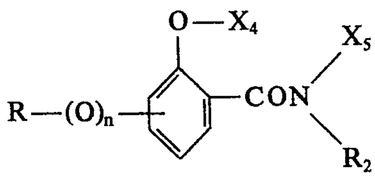

(IVe)

worin R einen Alkylenrest alk entsprechenden, eine vom Sauerstoffatom bzw. Phenylrest durch mindestens ein Kohlenstoffatom getrennte Carbonylgruppierung enthaltenden Alkylrest bedeutet, und $X_4$ oder $X_4$ und $X_5$ zusammen eine der angegebenen Schutzgruppen bedeuten, gebildete Schiff'sche Base mit einem Borhydrid, etwa Natriumborhydrid zur Verbindung der Formel IV reduzieren. Die Reduktion kann auch mittels aktiviertem Wasserstoff in Gegenwart eines Hydrierkatalysators, z. B. eines Platin-auf-Kohle Katalysators erfolgen.

Carbonylverbindungen der Formel [IVe), in der n 1 ist, wiederum können durch Umsetzung einer Verbindung der Formel

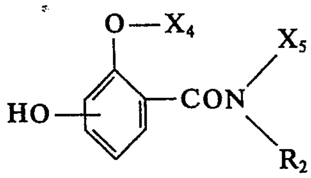

(IVf)

mit einer Verbindung der Formel R-Hal (IVg), worin R obige Bedeutung hat, z. B. ein Halogenketon, z. B. Chloraceton darstellt, in üblicher Weise erhalten werden.

Die neuen Verbindungen der Formel I können ebenfalls erhalten werden, indem man in einer Verbindung der Formel

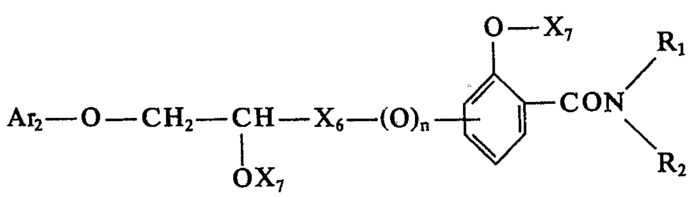

(V)

worin $X_6$ eine reduzierbare Gruppe der Formeln

$-CH = N - alk-$ (Va)

bzw.

$-CH_2 - N = alk_1-$ (Vb)

ist, wobei $alk_1$ für einen, dem Rest alk entsprechenden Alkylylidenrest steht und $X_7$ Wasserstoff oder einen unter den Bedingungen für die Reduktion von $X_6$ durch Wasserstoff ersetzbaren Rest darstellt, $Ar_2$ einem Rest Ar entspricht, jedoch gegebenenfalls anstelle von einem oder zwei Hydroxy eine oder zwei Gruppen $OX_7$, in welchen $X_7$ die vorstehend angegebene Bedeutung hat, trägt, und n Null oder 1 bedeutet, wobei stets $X_6$ eine reduzierbare Gruppe Va oder Vb ist, diese Gruppe reduziert und gleichzeitig die von Wasserstoff verschiedene Gruppe $X_7$ durch Wasserstoff ersetzt, und, wenn erwünscht, die anschließend an das erste Verfahren genannten zusätzlichen Verfahrensschritte durchführt.

Eine hydrogenolytisch abspaltbare Gruppe $X_7$ ist in erster Linie eine $\alpha$-Arylniederalkylgruppe, wie eine gegebenenfalls substituierte 1-Phenylniederalkylgruppe, worin Substituenten z. B. Niederalkoxy, wie Methoxy sein können, und ganz besonders Benzyl.

Schutzgruppen, die an den den Rest $Ar_2$ gegebenenfalls substituierenden Hydroxygruppen stehen, entsprechen den vorhin für $X_7$ genannten und mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmaßnahme abgespalten werden.

Ausgangsstoffe der Formel V mit einer Gruppe der Formel Vb können auch in der isomeren Form von Ring-Tautomeren der Formel

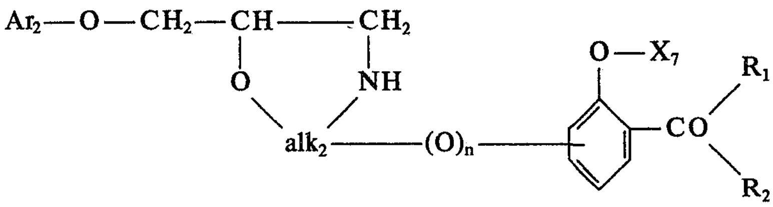

(Vd)

worin $alk_2$ der Bedeutung von $alk_1$ entspricht und das Sauerstoff- und Stickstoffatom des Ringes an das gleiche Kohlenstoffatome gebunden sind, vorliegen.

Eine Alkylylidengruppe $alk_1$ ist z. B. Methin oder Äthylyliden, während eine Alkylidengruppe $alk_2$ z. B. Methylen, Äthyliden oder 1-Methyläthyliden darstellt.

Die Reduktion der Stickstoff-Kohlenstoff-Doppelbindung in Ausgangsstoffen der Formel V, die als $X_6$ eine Gruppe Va oder Vb enthalten, während $Ar_2$, $X_7$ und n die unter der Formel V angegebene Bedeutung haben, zur Stickstoff-Kohlenstoff-Einfachbindung kann in an sich bekannter Weise, z. B. durch Behandeln mit katalytisch aktiviertem Wasserstoff, wie Wasserstoff in Gegenwart eines geeigneten Hydrierkatalysators, z. B. eines Nickel-, Platin- oder Palladiumkatalysators, erfolgen, wobei hydrogenolytisch abspaltbare Gruppen $X_7$ zugleich abgespalten und durch Wasserstoff ersetzt werden; oder man arbeitet mit einem geeigneten Hydridreduktionsmittel, wie einem Alkalimetallborhydrid, z. B. Natriumborhydrid. Bei Anwendung von Hydridreduktionsmittel können auch an Sauerstoff gebundene Acylreste von Carbonsäuren, wie z. B. der Essigsäure, als Reste $X_7$ vorliegen und im gleichen Arbeitsgang abgespalten werden.

Ein Ausgangsmaterial der Formel V kann in an sich bekannter Weise, gegebenenfalls in situ, d. h. unter den Bedingungen des beschriebenen Verfahrens, hergestellt werden. So kann man eine Verbindung der Formel

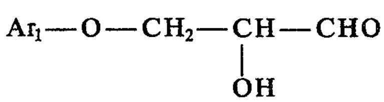

(Ve)

mit einem Amin der Formel

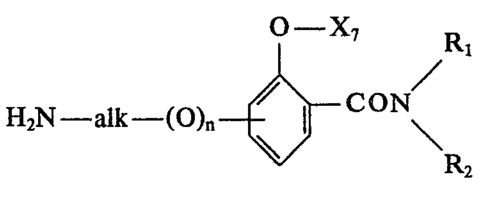

(Vf)

zu einem Ausgangspunkt der Formel V mit der Gruppe $X_6$ der Formel Va umgesetzt werden.

Durch Umsetzung einer Verbindung der Formel

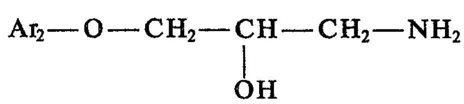

(Vh)

mit einer Carbonylverbindung der Formel

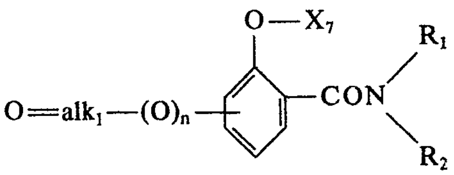

(Vi)

kann man zu Ausgangsstoffen der Formel V mit einer Gruppe $X_6$ der Formel (Vb) gelangen. Eine Modifizierung dieser Umsetzungen besteht darin, daß man einer Verbindung der Formel (Vh) entsprechende Dibenzylaminoverbindung, mit der Oxoverbindung der Formel (Vi) unter den reduzierenden Bedingungen des Verfahrens umsetzt. Hierbei verwendet man als Reduktionsmittel in erster Linie katalytisch aktivierten Wasserstoff, z. B. Wasserstoff in Gegenwart eines Schwermetallhydrierkatalysators oder eines Gemisches davon, wie eines Palladium- und/oder Platinkatalysators. Unter solchen Reaktionsbedingungen werden hydrogenolytisch abspaltbare Gruppen $X_7$, z. B. Benzylgruppen, abgespalten, und gleichzeitig die Stickstoff-Kohlenstoff-Doppelbindung zur entsprechenden Stickstoff-Kohlenstoff-Einfachbindung reduziert.

Oxoverbindungen der Formel (Vi) wiederum, worin n für 1 steht, sind z. B. durch Umsetzung einer Dihydroxyverbindung der Formel

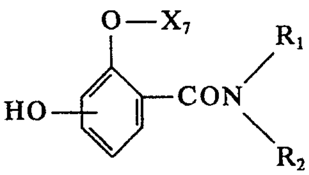

(Vk)

mit einer Halogenalkanon-Verbindung der oben erläuterten Formel R — Hal (IVf), z. B. Chloraceton, in Gegenwart eines alkalischen Kondensationsmittels, etwa Kaliumcarbonat, oder einer organischen Base, wie Triäthylamin, erhältlich.

Die neuen Verbindungen der Formel I können ebenfalls erhalten werden, indem man eine Verbindung der Formel

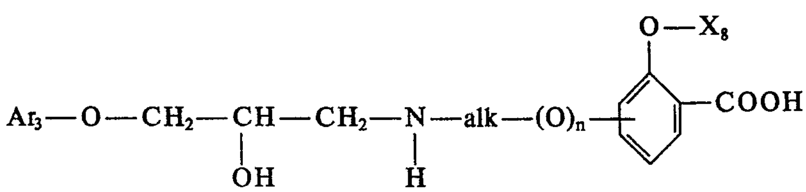

(VI)

worin $Ar_3$ die Bedeutung von Ar hat, oder einen Rest Ar darstellt, der durch 1 bis 2 mittels Aminolyse in Hydroxy überführbare Gruppen substituiert ist, $X_8$ Wasserstoff oder eine mittels Aminolyse abspaltbare Gruppe bedeutet, oder ein reaktionsfähiges Derivat einer der in Formel VI definierten Carbonsäuren, mit einer Verbindung der Formel

$$HNR_1R_2 \qquad \text{(VII)}$$

umsetzt, und gleichzeitig gegebenenfalls vorhandene Reste $X_8$ abspaltet und durch Wasserstoff ersetzt, und wenn erwünscht die anschließend an das erste Verfahren genannten zusätzlichen Verfahrensschritte durchführt.

Aminolytisch und insbesondere ammonolytisch abspaltbare Reste $X_8$ sind Acylreste von organischen Carbonsäuren, z. B. Aroyl, wie Benzoyl, oder Niederalkanoyl, wie Acetyl.

Reaktionsfähige Derivate der in Formel VI definierten Carbonsäuren sind z. B. die Halogenide, wie die Chloride oder Bromide, ferner die Azide, sowie Säureanhydride insbesondere gemischte Säureanhydride mit z. B. Niederalkancarbonsäuren, wie Essigsäure oder Propionsäure, Niederalkoxyalkancarbonsäuren, wie 2-Methoxyessigsäure. Reaktionsfähige Derivate von Carbonsäuren der Formel VI sind insbesondere Ester, z. B. mit Niederalkanolen, wie Methanol, Äthanol, Isopropanol, tert.-Butanol, ferner mit Arylniederalkanolen, etwa gegebenenfalls durch Niederalkyl, z. B. Methyl oder Niederalkoxy z. B. Methoxy, substituierter Benzylalkohol, oder Phenolen, die gegebenenfalls durch geeignete Substituenten aktiviert sind, z. B. durch Halogen, etwa 4-Halogen, wie 4-Chlor, Niederalkoxy etwa 4-Niederalkoxy wie 4-Methoxy, 4-Nitro oder 2,4-Dinitro, wie etwa 4-Chlorphenol, 4-Methoxyphenol, 4-Nitro- oder 2,4-Dinitrophenol, ferner mit Cycloalkanolen, wie etwa Cyclopentanol oder Cyclohexanol, die gegebenenfalls durch Niederalkyl, z. B. Methyl, substituiert sein können. Die Umsetzung wird in an sich bekannter Weise, üblicherweise in Gegenwart eines inerten Lösungsmittels z. B. in einem Temperaturbereich von etwa $-10°$ bis $+50°$ C in einem geschlossenen Gefäß durchgeführt.

Die Ausgangsstoffe der Formel VI lassen sich in an sich bekannter Weise erhalten, indem man eine Verbindung der Formel (II), in der $X_1$ und $Z_1$ zusammen die Epoxygruppe bedeuten, mit einer Aminoverbindung der Formel

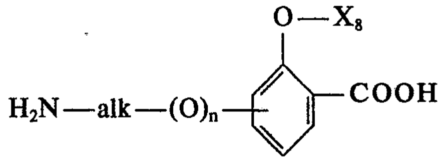

(VIa)

worin $X_8$ die angegebene Bedeutung hat, oder einem reaktionsfähigen Derivat davon, umsetzt.

Man kann ferner die durch Umsetzung einer Verbindung der Formel

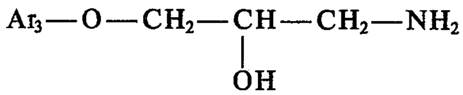

(VIb)

mit einer Carbonylverbindung der Formel

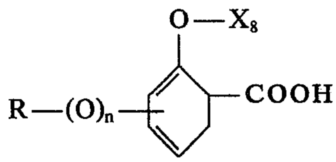

(VIc)

worin R den einem Alkylenrest Alk entsprechenden, an der Stelle von dessen freier Valenz einen Oxorest enthaltenden Alkylrest bedeutet, gebildete Schiff'sche Base mit einem Borhydrid, etwa Natriumborhydrid reduzieren. Die Reduktion kann auch mittels aktiviertem Wasserstoff in Gegenwart eines Hydrierkatalysators, z. B. eines Platin-auf-Kohle-Katalysators erfolgen.

Carbonylverbindungen der Formel (VIc) wiederum, worin n 1 bedeutet, können durch Umsetzung einer Verbindung der Formel

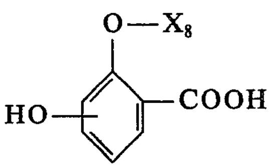

(IVh)

mit einer Verbindung der Formel

R—Hal (IVg)

worin Hal für Halogen, insbesondere Chlor steht, in an sich bekannter Weise erhalten werden.

Die neuen Verbindungen der Formel I können ebenfalls erhalten werden, indem man in einer Verbindung der Formel

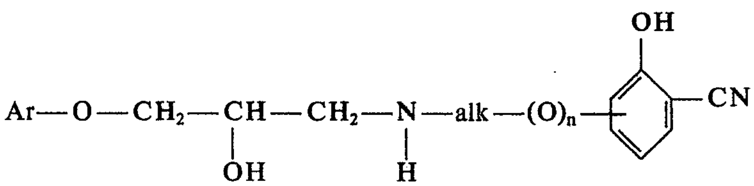

(VIII)

worin eine oder beide Hydroxygruppen und/oder im Rest Ar vorhandene Hydroxygruppen gegebenenfalls durch solche mittels Hydrolyse abspaltbaren und durch Wasserstoff ersetzbaren Gruppen geschützt sind, die unter den Bedingungen des Verfahrens abgespalten und durch Wasserstoff ersetzt werden, die Gruppe —CN mittels Hydrolyse in die Gruppe $-CONH_2$ und gleichzeitig gegebenenfalls geschützte Hydroxygruppen in freie Hydroxygruppen umwandelt, und, wenn erwünscht, die anschließend an das erste Verfahren genannten zusätzlichen Verfahrensschritte durchführt.

Die obigen Reaktionen werden in an sich bekannter Weise durchgeführt. Die Hydrolyse wird in einem basischen oder vorteilhafterweise in einem sauren Medium, insbesondere in Gegenwart konzentrierter wäßriger Mineralsäure, wie z. B. konz. Salzsäure, und wenn notwendig, unter Kühlen oder Erwärmen, z. B. in einem Temperaturbereich von etwa 0 bis 60°, vorzugsweise von etwa 40—50°, in einem offenen oder geschlossenen Gefäß und/oder in einer Inertgasatmosphäre, z. B. in einer Stickstoffatmosphäre durchgeführt.

Die Ausgangsstoffe der Formel VIII lassen sich z. B. durch Umsetzen einer Verbindung der Formel

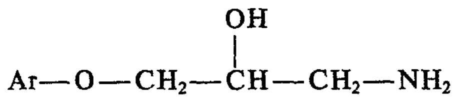

(VIIIa)

mit einer Verbindung der Formel

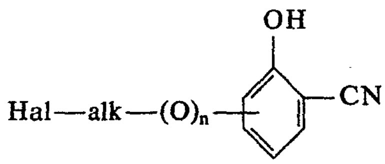

(VIIIb)

worin Hal Chlor, Brom oder Jod darstellt, erhalten. Die Umsetzung wird vorteilhafterweise in Gegenwart eines basischen Mittels in an sich bekannter Weise durchgeführt.

Die Verbindung VIIIb wiederum kann durch Einwirkung von Essigsäureanhydrid auf das dem Cyanid entsprechende Oxim erhalten werden. Dies geschieht zweckmäßigerweise durch Kochen unter Rückfluß. Das Oxim kann seinerseits aus dem entsprechenden Aldehyd durch Kochen mit Hydroxylaminhydrochlorid in Gegenwart von alkoholischer Sodalösung unter Rückfluß hergestellt werden. Der entsprechende Aldehyd wiederum kann durch Umsetzung von 2,4-Dihydroxybenzaldehyd mit einem $\alpha,\omega$-Dihalogenniederalkan, vorzugsweise in Gegenwart eines basischen Mittels hergestellt werden. Man kann aber auch in analoger Weise ein Hydroxysalicylonitril, z. B. das 2,4-Dihydroxybenzonitril [Chem. Ber. 24, 3657 (1891)] oder das 2,5-Dihydroxybenzonitril [Helv. Chim. Acta 30, 149, 153 (1947)] mit einem nicht-geminalen Dihalogenniederalkan zu einer Verbindung der Formel VIIIb umsetzen.

Bei der Auswahl des geeigneten obigen Verfahrens zur Herstellung von Verbindungen der Formel I muß darauf geachtet werden, daß vorhandene Substituenten, in erster Linie der Reste Ar, nicht umgewandelt oder abgespalten werden, falls solche Umwandlungen bzw. Abspaltungen nicht erwünscht sind. So können insbesondere funktionell abgewandelte Carboxylgruppen, wie veresterte oder amidierte Carboxylgruppen, sowie Cyangruppen, als Substituenten der Reste Ar während Solvolysen, insbesondere Hydrolysen, ferner auch bei Reduktionen an der Reaktion beteiligt sein und umgewandelt werden. Andererseits können gleichzeitige Umwandlungen von Substituenten erwünscht sein; z. B. können ungesättigte Substituenten, wie Niederalkenyl, unter den Bedingungen eines erfindungsgemäß eingesetzten Reduktionsverfahrens, z. B. zu Niederalkyl, reduziert werden.

In erhaltenen Verbindungen kann man im Rahmen der Definition der Verbindungen der Formel I in üblicher Weise verfahrensgemäß erhaltene Verbindungen in andere Endstoffe überführen, z. B. indem man geeignete Substituenten abwandelt, einführt oder abspaltet.

So kann man in erhaltenen Verbindungen ungesättigte Substituenten, wie Niederalkenyl, z. B. durch Behandeln mit katalytisch aktiviertem Wasserstoff, reduzieren.

Ferner kann man in erhaltenen Verbindungen mit halogensubstituierten Resten aromatischen Charakters das Halogen, z. B. durch Behandeln mit Wasserstoff in Gegenwart eines üblichen Hydrierkatalysators, wie Raney-Nickel oder Palladium auf Kohle, durch Wasserstoff ersetzen.

Freie Carboxylgruppen in den Resten Ar lassen sich in üblicher Weise verestern, beispielsweise durch Umsetzen mit einem entsprechenden Alkohol, vorteilhaft in Gegenwart einer Säure, wie einer Mineralsäure, z. B. Schwefelsäure oder Chlorwasserstoffsäure, oder in Gegenwart eines wasserbindenden Mittels, wie Dicyclohexylcarbodiimid, oder durch Umsetzen mit einer entsprechenden Diazoverbindung, z. B. Diazomethan. Die Veresterung kann auch durch Umsetzen eines Salzes, vorzugsweise eines Alkalimetallsalzes der Säure mit einem reaktionsfähigen veresterten Alkohol, z. B. einem entsprechenden Halogenid, wie Chlorid, durchgeführt werden.

Freie Carboxylgruppen lassen sich in üblicher Weise amidieren, beispielsweise durch Umsetzen mit Ammoniak, oder mit einem primären oder sekundären Amin, vorteilhaft in Gegenwart eines wasserbindenden Mittels, wie Dicyclohexylcarbodiimid, oder durch Überführen der Carboxylgruppe in eine Halogencarbonyl-, z. B. Chlorcarbonylgruppe, und dann Umsetzen mit Ammoniak oder mit einem primären oder sekundären Amin.

In Verbindungen, die eine veresterte Carboxylgruppe enthalten, kann diese in üblicher Weise, z. B. durch Hydrolyse, vorzugsweise in Gegenwart von starken Basen, wie einem Alkalimetallhydroxid, z. B. Natrium- oder Kaliumhydroxyd, oder starken Säuren, z. B. einer starken Mineralsäure, wie einer Halogenwasserstoffsäure, z. B. Chlorwasserstoffsäure oder Schwefelsäure, in eine freie Carboxylgruppe übergeführt werden.

In Verbindungen mit einer veresterten Carboxylgruppe als Substituent kann diese in üblicher Weise, z. B. durch Ammonolyse oder Aminolyse mit Ammoniak oder einem primären oder sekundären Amin in die entsprechende Carbamoylgruppe übergeführt werden.

Verbindungen mit einer Carbamoylgruppe und vorzugsweise von Wasserstoff verschiedenen Resten $R_1$ und $R_2$ können in üblicher Weise, z. B. durch Einwirkung wasserentziehender Mittel, wie

Phosphorpentoxid oder Phorphoroxychlorid, vorzugsweise bei höheren Temperaturen zu den entsprechenden Cyanverbindungen dehydratisiert werden.

Verbindungen, die einen Cyansubstituenten enthalten, können in üblicher Weise, z. B. in Gegenwart konzentrierter wäßriger Mineralsäuren oder Alkalimetallhydroxiden, zu den entsprechenden Carbamoyl- oder direkt zu den Carboxylverbindungen verseift werden.

Verbindungen mit einer Cyangruppe als Substituenten können in üblicher Weise, z. B. durch Addition von Alkoholen in Gegenwart einer wasserfreien Säure, wie Chlorwasserstoff, und nachträglicher Hydrolyse des entstandenen Imidoesters zu den entsprechenden Verbindungen mit veresterten Carboxylgruppen alkoholysiert werden.

Wie bei den Herstellungsverfahren muß auch bei der Durchführung der Zusatzschritte darauf geachtet werden, daß unerwünschte Nebenreaktionen, welche die Umwandlung zusätzlicher Gruppierungen zur Folge haben können, nicht eintreten.

Die oben beschriebenen Reaktionen können gegebenenfalls gleichzeitig oder nacheinander, ferner in beliebiger Reihenfolge durchgeführt werden. Falls notwendig, erfolgen sie in Anwesenheit von Verdünnungsmitteln, Kondensationsmitteln und/oder katalytisch wirkenden Mitteln, bei erniedrigter oder erhöhter Temperatur, im geschlossenen Gefäß unter Druck und/oder in einer Inertgasatmosphäre.

Je nach den Verfahrensbedingungen und Ausgangsstoffen erhält man die neuen Verbindungen in freier Form oder in der ebenfalls von der Erfindung umfaßten Form ihrer Salze, wobei die neuen Verbindungen oder Salze davon auch als Hemi-, Mono-, Sesqui- oder Polyhydrate davon vorliegen können. Säureadditionssalze der neuen Verbindungen können in an sich bekannter Weise, z. B. durch Behandeln mit basischen Mitteln, wie Alkalimetallhydroxyden, -carbonaten oder -hydrogencarbonaten oder Ionenaustauschern, in die freien Verbindungen übergeführt werden. Andererseits können erhaltene freie Basen mit organischen oder anorganischen Säuren, z. B. mit den genannten Säuren, Säureadditionssalze bilden, wobei zu deren Herstellung insbesondere solche Säuren verwendet werden, die sich zur Bildung von pharmazeutisch annehmbaren Salzen eignen.

Diese oder andere Salze, insbesondere Säureadditionssalze der neuen Verbindungen, wie z. B. Oxalate oder Perchlorate, können auch zur Reinigung der erhaltenen freien Basen dienen, indem man die freien Basen in Salze überführt, diese abtrennt und reinigt, und aus den Salzen wiederum die Basen freisetzt.

Die neuen Verbindungen können je nach der Wahl der Ausgangsstoffe und Arbeitsweisen, als optische Antipoden oder Racemate, oder sofern sie mindestens zwei asymmetrische Kohlenstoffatome enthalten, auch als Racematgemische vorliegen. Die Ausgangsstoffe können auch als bestimmte optische Antipoden eingesetzt werden.

Erhaltene Racematgemische können auf Grund der physikalisch-chemischen Unterschiede der Diastereoisomeren in bekannter Weise, z. B. durch Chromatographie und/oder fraktionierte Kristallisation, in die beiden stereoisomeren (diastereomeren) Racemate aufgetrennt werden.

Erhaltene Racemate lassen sich nach an sich bekannten Methoden in die Antipoden zerlegen, z. B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Substanz, insbesondere Säuren, und Trennen des auf diese Weise erhaltenen Salzgemisches, z. B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z. B. die D- und L-Formen von Weinsäure, Di—O,O'-(p-Toluoyl)-weinsäure, Äpfelsäure, Mandelsäure, Camphersulfonsäure, Glutaminsäure, Asparaginsäure oder Chinasäure. Vorteilhaft isoliert man den wirksameren der beiden Antipoden.

Die Erfindung betrifft auch diejenigen Ausführungsformen des Verfahrens, bei denen eine Reaktionskomponente gegebenenfalls in Form ihrer Salze vorliegt.

Zweckmäßig verwendet man für die Durchführung der erfindungsgemäßen Reaktionen solche Ausgangsstoffe, die zu den eingangs besonders erwähnten Gruppen von Endstoffen und besonders zu den speziell beschriebenen oder hervorgehobenen Endstoffen führen.

Die Ausgangsstoffe sind bekannt oder können, falls sie neu sind, nach an sich bekannten Methoden, wie oben, z. B. analog wie in den Beispielen beschrieben, erhalten werden.

Die neuen Verbindungen können z. B. in Form pharmazeutischer Präparate Verwendung finden, welche eine pharmakologisch wirksame Menge der Aktivsubstanz, gegebenenfalls zusammen mit pharmazeutisch verwendbaren Trägerstoffen enthalten, die sich zur enteralen, z. B. oralen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z. B. Laktose, Dextrose, Sukrose, Mannitol, Sorbitol, Cellulose und/oder Glycerin und/oder Schmiermitteln, z. B. Kieselerde, Talk, Stearinsäure oder Salze davon, wie Magnesium- oder Calciumstearat, und/oder Polyäthylenglykol, aufweisen. Tabletten können ebenfalls Bindemittel, z. B. Magnesiumaluminiumsilikat, Stärken, wie Mais-, Weizen-, Re- oder Pfeilwurzstärke, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, und/oder Brausemischungen, oder Adsorptionsmittel, Farbstoffe, Geschmackstoffe und Süßmittel enthalten.

Ferner kann man die neuen pharmakologisch wirksamen Verbindungen in Form von parenteral verabreichbaren Präparaten oder von Infusionslösungen verwenden. Solche Lösungen sind vorzugsweise isotonische wäßrige Lösungen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/ oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z. B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes.

Die Dosierung kann von verschiedenen Faktoren, wie Applikationsweise, Spezies, Alter und/oder individuellen Zustand abhängen. So liegen die täglich in einer oder mehreren, vorzugsweise höchstens 4 Einzeldosen zu verabreichenden Dosen bei oraler Applikation an Warmblüter für $\beta$-Rezeptoren-Blokker der Formel I zwischen 0,03 und 3 mg/kg und für Warmblüter von etwa 70 kg vorzugsweise zwischen etwa 0,004 und etwa 0,08 g, und für $\beta$-Rezeptoren-Stimulatoren der Formel I zwischen 0,01 und 1 mg/kg bzw. zwischen etwa 0,002 und etwa 0,04 g.

Die folgenden Beispiele dienen zur Illustration der Erfindung; Temperaturen werden in Celsiusgraden angegeben.

## Beispiel 1

21 g rohes 1-[N-[2-(3-Carbamoyl-4-hydroxyphenoxy)-1-methyläthyl]-benzylamino]-3-[4-(2-methoxy-äthoxy)-phenoxy]-2-propanol, gelöst in 170 ml Methanol, werden unter Zusatz von 2 g Pd/C-Katalysator (5%) bei Normalbedingungen bis zum Stillstand der Wasserstoff-Aufnahme hydriert. Durch Filtration und Eindampfen der Lösung erhält man ein Öl, das beim Verreiben mit Toluol kristallisiert. Nach Umkristallisation des kristallinen Rückstandes aus Äthylacetat schmilzt das erhaltene 1-[2-(3-Carbamoyl-4-hydroxy-phenoxy)-1-methyl-äthylamino]-3-[4-(2-methoxyäthoxy)-phenoxy]-2-propanol bei 117—125°, (Gemisch der Diastereomeren).

Der Ausgangsstoff wird auf folgende Weise hergestellt:

1a) Nach der von Irvine et al., Synthesis 1972, 568 beschriebenen Methode wird 2,5-Dihydroxy-benzamid unter Verwendung eines Überschusses von Aceton in 2,3-Dihydro-2,2-dimethyl-6-hydroxy-4H-1,3-benzoxazin-4-on vom Smp. 215—216° übergeführt.

1b) 70 g 2,3-Dihydro-2,2-dimethyl-6-hydroxy-4H-1,3-benzoxazin-4-on werden in 400 ml Acetonitril mit 100 g Kaliumcarbonat und 32 ml Chloraceton 30 Stunden unter Rückfluß gerührt. Nach Zusatz von weiteren 3,2 ml Chloraceton wird das Reaktionsgemisch während weiteren 15—20 Stunden erhitzt. Das noch warme Reaktionsgemisch wird filtriert, der Rückstand mit Aceton gründlich gewaschen und das vereinigte Filtrat eingedampft. Der kristalline Rückstand wird aus Toluol umkristallisiert und ergibt das 2,3-Dihydro-2,2-dimethyl-6-(2-oxopropoxy)-4H-1,3-benzoxazin-4-on vom Smp. 125—126°.

1c) 74 g rohes, gemäß Beispiel 1b) erhaltenes 2,3-Dihydro-2,2-dimethyl-6-(2-oxo-propoxy)-4H-1,3-benzoxazin-4-on werden in einem Gemisch von 150 ml Dioxan und 450 ml 2-n. Salzsäure 45 Minuten auf dem Wasserbad erhitzt. Das Lösungsmittel wird abgedampft und der kristalline Rückstand mit Wasser verrieben und dann abgesaugt. Durch Umkristallisation aus Isopropanol erhält man das 5-(2-Oxo-propoxy)-Salicylamid vom Smp. 152—154°.

1d) Eine Lösung von 104,5 g 5-(2-Oxo-propoxy)-salicylamid in 1000 ml Methanol wird mit 55 g Benzylamin und 1,25 g conc. Schwefelsäure versetzt und in Anwesenheit von 3,0 g Pt/C-Katalysator bei Raumtemperatur und Atmosphärendruck bis zur Aufnahme von 1 Äquivalent Wasserstoff hydriert. Der Katalysator wird abfiltriert, die Lösung mit ca. 10 g pulverisiertem Calciumcarbonat verrührt, nochmals filtriert und eingedampft. Das verbleibende Öl kristallisiert aus Isopropanol. Nochmalige Umkristallisation aus Isopropanol ergibt 5-[2-Benzylamino)-propoxy]-salicylamid vom Smp. 102—104°.

1e) Eine Lösung von 10,2 g 1-(2,3-Epoxy-propoxy)-4-(2-methoxyäthoxy)-benzol und 11,0 g 5-[2-(Benzylamino)-propoxy]-salicylamid in 200 ml Isopropanol wird 24 Stunden am Rückfluß gekocht. Durch Eindampfen der Lösung erhält man rohes 1-[N-[2-(3-Carbamoyl-4-hydroxy-phenoxy)-1-methyl-äthyl]-benzylamino]-3-[4-(2-methoxy-äthoxy)-phenoxy]-2-propanol als Öl, welches roh zur Debenzylierung eingesetzt wird.

## Beispiel 2

6,1 g rohes 1-[N-[2-(4-Carbamoyl-3-hydroxy-phenoxy)-1-methyl-äthyl]benzylamino]-3-[4-(2-methoxy-äthoxy)-phenoxy]-2-propanol werden analog Beispiel 1 hydriert und aufgearbeitet. Man erhält so nach Umkristallisation aus Isopropanol das 1-[2-(4-Carbamoyl-3-hydroxy-phenoxy)-1-methyl-äthylamino]-3-[4-(2-methoxy-äthoxy)-phenoxy]-2-propanol als Diastereomerengemisch vom Smp. 120—121°.

Der Ausgangsstoff wird auf folgende Weise hergestellt:

2a) Aus 2,4-Dihydroxy-benzamid erhält man analog Beispiel 1a) das 2,3.-Dihydro-2,2-dimethyl-7-hydroxy-4H-1,3-benzoxazin-4-on vom Smp. 249—251°.

2b) Analog Beispiel 1b) erhält man aus 168 g 2,3-Dihydro-2,2-dimethyl-7-hydroxy-4H-1,3-benzoxazin-4-on, 305 g Kaliumcarbonat und 88 ml Chloraceton in 1,2 Liter Acetonitril durch Kochen während 28 Stunden und nachfolgendem Aufarbeiten das 2,3-Dihydro-2,2-dimethyl-7-(2-oxo-propoxy)-4H-1,3-benzoxazin-4-on vom Smp. 160—162° (aus Isopropanol).

2c) 75 g rohes 2,3-Dihydro-2,2-dimethyl-7-(2-oxo-propoxy)-4H-1,3-benzoxazin-4-on und 32 g Benzylamin, gelöst in 1000 ml Methanol werden unter Zusatz von 0,75 g konz. Schwefelsäure und 1,6 g Pt/C-Katalysator (5%) bis zum Stillstand der Wasserstoffaufnahme unter Normalbedingungen hydriert. Nach Abfiltrieren des Katalysators und Abdampfen des Lösungsmittels wird der ölige Rückstand zwischen 300 ml Äthylacetat und 500 ml 2-n. Salzsäure verteilt. Aus der wäßrigen Phase isoliert man durch Alkalischstellen mit conc. Ammoniak (Eiskühlung) und Extraktion mit Äthylacetat rohes 2,3-Dihydro-2,2-dimethyl-7-[(2-benzylamino)-propoxy]-4H-1,3-benzoxazin-4-on als Öl, welches roh weiterverwendet werden kann.

2d) Ein Gemisch von 100 g rohem 2,3-Dihydro-2,2-dimethyl-7-[(2-benzylamino)-propoxy]-4H-1,3-benzoxazin-4-on, 100 ml Isopropanol und 100 ml Isopropylamin wird 1 Stunde am Rückfluß gekocht und dann eingedampft. Das verbleibende Öl kristallisiert beim Verreiben mit Äther. Die Kristalle werden abgenutscht und mit wenig Isopropanol gewaschen. Man erhält so 4-[2-(Benzylamino)-propoxy]-salicylamid vom Smp. 121—123°.

2e) Analog Beispiel 1e) erhält man unter Verwendung von 4-[2-(Benzylamino)-propoxy]-salicylamid anstelle des 5-Derivats das 1-{N-[2-(4-Carbamoyl-3-hydroxy-phenoxy)-1-methyl-äthyl]-benzylamino}-3-[4-(2-methoxy-äthoxy)-phenoxy]-2-propanol als Öl, welches roh zur Debenzylierung eingesetzt wird.

Beispiel 3

Analog Beispiel 2 erhält man durch Debenzylieren von 22 g rohem 1-{N-[2-(Carbamoyl-4-hydroxy-phenoxy)-äthyl]-benzylamino}-3-{2-[N-(2-hydroxyäthyl)-carbamoylmethoxy]-phenoxy}2-propanol das 1-[2-(3-Carbamoyl-4-hydroxy-phenoxy)-äthylamino]-3-{2-[N-(2-hydroxyäthyl)-carbamoylmethoxy]-phenoxy}-2-propanol vom Smp. 157—159°. Es bildet ein Hydrochlorid vom Smp. 126—127° (aus Isopropanol-Wasser 1 : 1).

Der Ausgangsstoff wird auf folgende Weise erhalten:

3a) Ein Gemisch von 48,2 g 2,3-Dihydro-2,2-dimethyl-6-hydroxy-4H-1,3-benzoxazin-4-on, 70 g Kaliumcarbonat und 250 ml 1,2-Dibromäthan wird unter Rühren und Rückfluß während 4 Stunden gekocht. Das breiartige Reaktionsgemisch wird 3—4mal mit je 1 Liter Methanol heiß extrahiert, die vereinigten Methanol-Extrakte eingedampft und der Rückstand aus Methanol umkristallisiert. Man erhält das 6-(2-Bromäthoxy)-2,3-dihydro-2,2-dimethyl-4H-1,3-benzoxazin-4-on vom Smp. 190—195°.

3b) Ein Gemisch von 60 g 6-(2-Bromäthoxy)-2,3-dihydro-2,2-dimethyl-4H-1,3-benzoxazin-4-on und 110 ml Benzylamin wird in einem Bad von 80° während 30 Minuten gerührt. Das Reaktionsgemisch wird hierauf unter Eiskühlung mit conc. Salzsäure auf pH 3—4 gebracht und kristallisieren gelassen. Nach 2—4 Stunden werden die Kristalle abgesaugt, mit je 50 ml Wasser und Äthylacetat gewaschen und getrocknet. Das so erhaltene 5-[(2-Benzylamino)-äthoxy]-saliclyamid-hydrochlorid schmilzt bei 214—216°. Die daraus freigesetzte Base schmilzt bei 107—108° (aus Äthylacetat-Äther).

3c) 12 g [2-(2,3-Epoxy-propoxy)-phenoxy]-N-(2-hydroxy-äthyl)-acetamid und 11,5 g 5-[2-Benzylamino)-äthoxy]-salicylamid werden in 70 ml Isopropanol 18—24 Stunden unter Rückfluß gekocht. Der ölige Eindampfrückstand wird roh zur Debenzylierung eingesetzt.

Beispiel 4

21 g rohes 1-{N-[2-(3-Carbamoyl-4-hydroxy-phenoxy)-äthyl]-benzylamino}-3-[4-(2-methoxy-äthoxy)-phenoxy]-2-propanol werden analog Beispiel 1 hydriert. Nach Beendigung der Wasserstoffaufnahme wird das Produkt in heißem Methanol gelöst und der Katalysator abfiltriert. Durch Eindampfen der methanolischen Lösung erhält man das 1-[2-(3-Carbamoyl-4-hydroxy-phenoxy)-äthylamino]-3-[4-(2-methoxy-äthoxy)-phenoxy]-2-propanol als Kristalle vom Smp. 157—158°. Es bildet neutrales Fumarat vom 150—151° (aus Methanol-Aceton).

Der Ausgangsstoff kann auf folgende Weise hergestellt werden:

4a) Eine Lösung von 9 g 1-(2,3-Epoxy-propoxy)-4-(2-methoxy-äthoxy)-benzol und 8,6 g 5-[(2-Benzylamino)-äthoxy]-salicylamid in 60 ml Isopropanol wird 24 Stunden unter Rückfluß erhitzt. Das durch Eindampfen erhaltene Rohprodukt wird zwischen 50 ml 2-n. Salzsäure und 100 ml Äther verteilt. Die Wasserphase wird abgetrennt und unter Eiskühlung mit conc. Ammoniaklösung alkalisch gestellt. Durch Extraktion mit ca. 300 ml Äthylacetat, Trocknen ($MgSO_4$) und Eindampfen wird das rohe 1-{N-[2-(3-Carbamoyl-4-hydroxy-phenoxy)-äthyl]-benzylamino}-3-[4-(2-methoxy-äthoxy)-phenoxy]-2-propanol als Öl isoliert und ohne weitere Reinigung zur Debenzylierung eingesetzt.

Beispiel 5

Analog Beispiel 4 und 4a werden hergestellt:

das 1-[2-(3-Carbamoyl-4-hydroxy-phenoxy)-äthylamino]-3-phenoxy-2-propanol vom Smp. 154—156° (aus Methanol),
das 1-[2-(3-Carbamoyl-4-hydroxy-phenoxy)-äthylamino]-3-{4-[2-(methoxycarbonylamino)-äthoxy]-phenoxy}-2-propanol vom Smp. 150—151° (aus Methanol),
das 1-(4-Acetamido-phenoxy)-3-[2-(3-carbamoyl-4-hydroxy-phenoxy)-äthylamino]-2-propanol vom Smp. 185° (aus Methanol),
das 1-[2-(3-Carbamoyl-4-hydroxy-phenoxy)-äthylamino]-3-(2-methyl-phenoxy)-2-propanol vom Smp. 129—130° (aus Isopropanol),
das 1-[2-(3-Carbamoyl-4-hydroxy-phenoxy)-äthylamino]-3-(3-methyl-phenoxy)-2-propanol vom Smp. 154—155° (aus Methanol),
das 1-[2-(3-Carbamoyl-4-hydroxy-phenoxy)-äthylamino]-3-(2-methyl-indol-4-yloxy)-2-propanol vom Smp. 180—194° (aus Äthylacetat),
das 5-{3-[2-(Carbamoyl-4-hydroxy-phenoxy)-äthylamino]-2-hydroxy-propoxy}-1,2,3,4-tetrahydro-2,3-cis-naphthalindiol als Diastereomerengemisch vom Smp. 108—118° (aus Methanol),
das 4-{2-Hydroxy-3-[(3-carbamoyl-4-hydroxy-phenoxy)-äthylamino]-propoxy}-phenylacetamid vom Smp. 149—151° (aus Methanol); eine andere Kristallmodifikation hat den Smp. 181—182° (aus Dimethylformamid-Wasser),
das 4-{2-Hydroxy-3-[(3-carbamoyl-4-hydroxy-phenoxy)-äthylamino]-propoxy}-phenoxyacetamid vom Smp. 168—170° (aus DMF-Wasser),
der N-{4-[2-Hydroxy-3-[(3-carbamoyl-4-hydroxy-phenoxy)-äthylamino]-propoxy]-phenyl}-N',N'-dimethylharnstoff vom Smp. 140—142° (Zers.) (aus Methanol),
das 1-(4-Butyroylamino-2-acetyl-phenoxy)-3-[2-(3-carbamoyl-4-hydroxy-phenoxy)-äthylamino]-2-propanol,
das 1-[2-(3-Carbamoyl-4-hydroxy-phenoxy)-äthylamino]-3-(2-methoxy-phenoxy)-2-propanol vom Smp. 125—126° (aus Methanol),
das 1-[2-(3-Carbamoyl-4-hydroxy-phenoxy)-äthylamino]-3-(2,3-dimethyl-phenoxy)-2-propanol vom Smp. 129—131° (aus Methanol),
das 1-[2-(3-Carbamoyl-4-hydroxy-phenoxy)-äthylamino]-3-[2-(2-methoxy-äthoxy)-phenoxy]-2-propanol als Hydrochlorid vom Smp. 157—160° (aus Methanol-Aceton),
das 1-[2-(3-Carbamoyl-4-hydroxy-phenoxy)-äthylamino]-3-[3-(2-methoxy-äthoxy)-phenoxy]-2-propanol,
das 1-(2-Carbamoylphenoxy)-3-[2-(3-carbamoyl-4-hydroxy-phenoxy)-äthylamino]-2-propanol als Hydrochlorid vom Smp. 149—152° (aus Methanol),
das 1-[2-(3-Carbamoyl-4-hydroxy-phenoxy)-äthylamino]-3-[2-(pyrrol-1-yl)-phenoxy]-2-propanol vom Smp. 138—140° (sintert ab 135°),
das 1-[2-(3-Carbamoyl-4-hydroxy-phenoxy)-äthylamino]-3-(3-trifluormethyl-phenoxy)-2-propanol vom Smp. 195—196° (aus Methanol),
das 1-(2-Acetyl-phenoxy)-3-[2-(3-carbamoyl-4-hydroxy-phenoxy)-äthylamino]-2-propanol vom Smp. 122—124° (aus Isopropanol),
das 1-{4-[2-(Acetamido)-äthoxy]-phenoxy}-3-[2-(3-carbamoyl-4-hydroxy-phenoxy)-äthylamino]-2-propanol als Hydrochlorid vom Smp. 191—192° (aus Methanol),
das 1-[2-(3-Carbamoyl-4-hydroxy-phenoxy)-äthylamino]-3-(3-methyl-pyridin-2-yloxy)-2-propanol vom Smp. 147—148° (aus Methanol),
das 1-[2-(3-Carbamoyl-4-hydroxy-phenoxy)-äthylamino]-3-(3-methyl-4-methylsulfonylphenoxy)-2-propanol vom Smp. 128—131° (aus Acetonitril),
das 1-[2-(3-Carbamoyl-4-hydroxy-phenoxy)-äthylamino]-3-(1-naphthyloxy)-2-propanol vom Smp. 131—134° (aus Isopropanol).

Beispiel 6

Analog Beispiel 2 und 2e erhält man aus 10,5 g 4-[(2-Benzylamino)-propoxy]-salicylamid und 8,5 g 3,4-Dihydro-5-(2,3-epoxy-propoxy)-2-(1H)-chinolinon und Debenzylierung des Reaktionsproduktes das 5-{3-[2-(4-Carbamoyl-3-hydroxy-phenoxy)-1-methyl-äthylamino]-2-hydroxy-propoxy}-3,4-dihydro-2-(1H)-chinolinon als Diastereomeren-Gemisch, das ein Hydrochlorid vom Smp. 239—245° bildet (aus Methanol).

## Beispiel 7

Eine Lösung von 16 g rohem 1-{N-[3-(3-Carbamoyl-4-hydroxy-phenyl)-propyl]-benzylamino}-3-[4-(2-methoxy-äthoxy)-phenoxy]-2-propanol wird analog Beispiel 1 hydriert. Die Hydrierlösung wird mit einer Lösung von Salzsäuregas in Methanol neutralisiert, eingedampft und aus Aceton kristallisiert. Man erhält so das 1-[3-(3-Carbamoyl-4-hydroxy-phenyl)-propylamino]-3-[4-(2-methoxy-äthoxy)-phenoxy]-2-propanol als Hydrochlorid vom Smp. 194—200°.

Das Ausgangsmaterial kann auf folgende Weise hergestellt werden:

7a) 3-(4-Hydroxyphenyl)-propionsäure wird über das gemischte Anhydrid mit Benzylamin in das 3-(4-Hydroxyphenyl)-propionsäure-N-benzylamid (Smp. 115—116°) überführt.

7b) Kolbe-Synthese ($CO_2$, 180°, 4 Stunden, 55 bar) mit dem Natrium-Salz der nach 7a) hergestellten Verbindung führt zum 3-(3-Carboxy-4-hydroxy-phenyl)-propionsäure-N-benzylamid vom Smp. 180—181°.

7c) Veresterung mit Methanol-Schwefelsäure am Rückfluß während 48 Stunden ergibt das 3-(3-Methoxycarbonyl-4-hydroxy-phenyl)-propionsäure-N-benzylamid vom Smp. 139—140° (aus Äthylacetat).

7d) Umsetzung mit Benzylbromid-Kaliumcarbonat in Aceton (15 Stunden Rückfluß) führt zum 3-(4-Benzyloxy-3-methoxy-carbonyl-phenyl)-propionsäure-N-benzylamid als gelbliches Öl.

7e) Selektive Reduktion der Amid-Gruppe mit Diboran in Tetrahydrofuran (48 Stunden, 20—25°) und milde katalytische Debenzylierung des Produktes (Pd/C-Katalysator 5%, 15—20°, in Methanol) ergeben N-[3-(4-Hydroxy-3-methoxy-carbonyl-phenyl)-propyl]-benzylamin vom Smp. 75—77° (aus Isopropanol).

7f) Eine Lösung von 27 g N-[2-(4-Hydroxy-3-methoxy-carbonyl-phenyl)-propyl]-benzylamin in 100 ml Dioxan wird mit 200 ml konz. Ammoniak versetzt und 3—4 Tage bei 20—30° stehengelassen. Das Reaktionsgemisch wird eingedampft, zwischen Wasser und Äthylacetat verteilt und die organische Phase abgetrennt. Übliche Aufarbeitung ergibt rohes N-[3-(3-Carbamoyl-4-hydroxy-phenyl)-propyl]-benzylamin als Öl, welches ohne weitere Reinigung weiterverarbeitet wird.

7g) Eine Lösung von 6,7 g 1-(2,3-Epoxy-propoxy)-4-(2-methoxyäthoxy)-benzol und 8,5 g N-[3-(3-Carbamoyl-4-hydroxy-phenyl)-propyl]-benzylamin in 70 ml Isopropanol wird 18 Stunden am Rückfluß gekocht und hierauf eingedampft. Das so erhaltene rohe 1-{N-[3-(3-Carbamoyl-4-hydroxy-phenyl)-propyl]-benzylamino}-3-[4-(2-methoxyäthoxy)-phenoxy]-2-propanol wird roh zur Debenzylierung eingesetzt.

## Beispiel 8

Eine Lösung von 5 g rohem 1-{N-[2-(3-Carbamoyl-4-hydroxy-phenoxy)-1-methyl-äthyl]-benzylamino}-3-(4-benzyloxy-phenoxy)-2-propanol (angereichert an Diastereomeren-Paar A) in 50 ml Methanol wird unter Normalbedingungen in Anwesenheit von 0,5 g Pd/C-Katalysator (5%) bis zur Aufnahme von 2 Äquivalenten Wasserstoff hydriert, worauf die Hydrierung zum Stillstand kommt. Das Reaktionsgemisch wird filtriert, im Filtrat werden 0,52 g Fumarsäure aufgelöst und die Lösung auf ca. 10 ml eingeengt. Nach längerem Stehen bilden sich Kristalle des neutralen Fumarats von 1-[2-(3-Carbamoyl-4-hydroxy-phenoxy)-1-methyl-äthylamino]-3-(4-hydroxy-phenoxy)-2-propanol vom Smp. 195—198° (reines Enantiomerenpaar A).

Auf analoge Weise läßt sich durch Debenzylierung von angereichertem Enantiomerenpaar B das Fumarat des reinen Enantionmmerenpaares B vom Smp. 181—185° herstellen.

Die Ausgangsstoffe können auf folgende Weise erhalten werden:

8a) Eine Lösung von 49,8 g 2,3-Dihydro-2,2-dimethyl-6-(2-oxo-propoxy)-4H-1,3-benzoxazin-4-on und 21,4 g Benzylamin in 700 ml Methanol wird unter Zusatz von 0,5 g konz. Schwefelsäure und 3 g Pt/C-Katalysator (5%) bis zur Aufnahme der äuqivalenten Menge Wasserstoff hydriert. Aufarbeitung analog Beispiel 1d) ergibt das 6-(2-Benzylamino-propoxy)-2,3-dihydro-2,2-dimethyl-4H-1,3-benzoxazin-4-on vom Smp. 127—129° (aus Isopropanol).

8b) Eine Lösung von 15,4 g Benzyl-[4-(2,3-epoxy-propoxy)-phenyl]-äther und 17,0 g 6-(2-Benzylaminopropoxy)-2,3-dihydro-2,2-dimethyl-4H-1,3-benzoxazin-4-on in 100 ml Isopranol wird 24 Stunden am Rückfluß gekocht, filtriert und eingedampft. Das Verreiben des Eindampfrückstandes mit ca. 200 ml Äther führt zur Kristallisation des 1-{N-[2-(2,3-Dihydro-2,2-dimethyl-4-oxo-4H-1,3-benzoxazin-6-yloxy)-1-methyl-äthyl]-benzylamino}-3-(4-benzyloxy-phenoxy)-2-propanols vom Smp. 149—160° (in dem das Enantiomerenpaar A angereichert ist).

Durch das Eindampfen der Ätherlösung und Umkristallisation aus wenig Isopropanol erhält man nach mehrtägigem Stehen Kristalle vom Smp. 59—62° (Reste bis 140°). Die nicht mehr kristallisierende Mutterlauge wird abgetrennt. Sie enthält das Enantiomerenpaar B in angereicherter Form.

8c) 5,2 g der unter 8b) erwähnten Kristalle vom Smp. 149—160° werden in einer Mischung von 20 ml Isopropylamin und 40 ml Isopropanol 1 Stunde unter Rückfluß gekocht und anschließend eingedampft. Das so als Öl erhaltene rohe 1-{N-[2-(3-Carbamoyl-4-hydroxy-phenoxy)-1-methyl-äthyl]-benzylamino}-3-(4-benzyloxy-phenoxy)-2-propanol (5 g) enthält das Enantiomerenpaar A angereichert und wird ohne weitere Reinigung zur Debenzylierung verwendet.

Auf analoge Weise wird mit dem das Enantiomerenpaar B angereichert enthaltenden Öl aus Beispiel 8b) verfahren.

Beispiel 9

Eine Lösung von 18,0 g rohem 1-[2-(2,3-Dihydro-2,2-dimethyl-4-oxo-4H-1,3-benzoxazin-6-yloxy)-1-methyl-äthylamino]-3-(4-methylcarbamoyl-phenoxy)-2-propanol in 300 ml Methanol wird mit 80 ml Isopropylamin versetzt und 1 Stunde am Rückfluß gekocht. Das Reaktionsgemisch wird eingedampft und das verbleibende Öl aus 80 ml Isopropanol kristallisiert. Man erhält das 1-[2-(3-Carbamoyl-4-hydroxy-phenoxy)-1-methyl-äthylamino]-3-(4-methylcarbamoyl-phenoxy)-2-propanol vom Smp. 172—175° (Diastereomeren-Gemisch).

Die Ausgangsstoffe können auf folgende Weise erhalten werden:

9a) Eine Lösung von 13,6 g 6-(2-Benzylamino-propoxy)-2,3-dihydro-2,2-dimethyl-4H-1,3-benzoxazin-4-on und 10,4 g 4-(2,3-Epoxy-propoxy)-N-methyl-benzamid in 80 ml Isopropanol werden 30 Stunden am Rückfluß gekocht. Der nach dem Abdampfen des Lösungsmittels verbleibende Rückstand wird zwischen Äther und 2-n. Salzsäure verteilt. Die saure, wäßrige Phase wird abgetrennt und unter Eiskühlung mit ca. 10% wäßriger Ammoniak-Lösung alkalisch gestellt und mit Äthylacetat extrahiert. Durch Abtrennen, Trocknen ($MgSO_4$) und Eindampfen des Äthylacetat-Extraktes erhält man das rohe 1-{N-[2-(2,3-Dihydro-2,2-dimethyl-4-oxo-4H-1,3-benzoxazin-6-yloxy)-1-methyl-äthyl]-benzylamino}-3-(4-methylcarbamoyl-phenoxy)-2-propanol als Öl, das ohne weitere Reinigung zur katalytischen Debenzylierung verwendet werden kann.

9b) Das gemäß 9a) erhaltene Produkt wird in 300 ml Methanol gelöst und unter Zusatz von 2,8 g Pd/C-Katalysator (5%) und einem weiteren Zusatz von 1,4 g Katalysator bis zur Beendigung der Wasserstoff-Aufnahme hydriert. Die nach Abfiltrieren des Katalysators erhaltene methanolische Lösung von 1-[2-(2,3-Dihydro-2,2-dimethyl-4-oxo-4H-1,3-benzoxazin-6-yloxy)-1-methyl-äthylamino]-3-(4-methylcarbamoyl-phenoxy)-2-propanol wird direkt weiter verarbeitet.

Beispiel 10

Genau analog der in Beispiel 9 beschriebenen Methode erhält man unter Verwendung von 4-(2,3-Epoxy-propoxy)-(2-methoxyäthyl)-benzol das 1-[2-(3-Carbamoyl-4-hydroxy-phenoxy)-1-methyl-äthylamino]-3-[4-(2-methoxyäthyl)-phenoxy]-2-propanol als Diastereomerengemisch vom Smp. 139—142° (aus Äthylacetat).

Beispiel 11

Ein Gemisch von 9,0 g 6-(2-Bromäthoxy)-2,3-dihydro-2,2-dimethyl-4H-1,3-benzoxazin-4-on und 14,5 g 1-(2-Allyloxy-phenoxy)-3-amino-2-propanol wird während 1 Stunde in einem Bad von 110—120° gerührt. Die Schmelze wird hierauf mit 100 ml Isopropanol ausgekocht, die Lösung filtriert und eingedampft. Der Eindampfrückstand wird zwischen 400 ml Äthylacetat und 50 ml 2-n. Kaliumbicarbonat-Lösung verteilt. Aus der Äthylacetat-Lösung wird durch Eindampfen und fraktionierte Kristallisation das 1-(2-Allyloxy-phenoxy)-3-[2-(3-carbamoyl-4-hydroxy-phenoxy)-äthylamino]-2-propanol vom Smp. 147—148° (aus Isopropanol) erhalten. Das neutrale Fumarat der Verbindung schmilzt bei 136—137° (aus Methanol).

Beispiel 12

Ein Gemisch von 11,2 g 1-(2-Allyloxy-phenoxy)-3-amino-2-propanol und 10,5 g 5-(2-Oxo-propoxy)-salicylamid wird in 200 ml Toluol unter Zusatz von einigen Tropfen Essigsäure am Wasserabscheider gekocht. Nach Aufhören der Wasserabspaltung (~2—3 Stunden) wird die Lösung eingedampft, der dunkelrote Rückstand in 300 ml Äthanol gelöst und portionenweise unter Rühren mit insgesamt 5,7 g Natriumborhydrid versetzt. Die Temperatur steigt hierbei bis 36°. Das Reaktionsgemisch wird noch 2 Stunden bei 20—30° weitergerührt und über Nacht stehengelassen. Unter Eiskühlung wird es dann mit ca. 6-n. Salzsäure auf pH 3—4 gebracht, filtiert und eingedampft. Der Eindampfrückstand wird zwischen je 100 ml Wasser und Äthylacetat verteilt, die Wasserphase wird abgetrennt, mit konz. Ammoniak alkalisch gestellt und mit 200 ml Äthylacetat extrahiert. Aufarbeiten der organischen Phase führt zum rohen, öligen 1-(2-Allyloxy-phenoxy)-3-[2-(3-carbamoyl-4-hydroxy-phenoxy)-1-methyl-äthylamino]-2-propanol als Enantiomeren-Gemisch. Durch langsames Kristallisieren aus Isopropanol erhält man die beiden reinen Enantiomeren-Paare vom Smp. 123—125° bzw. 98—102°.

Beispiel 13

Ein Gemisch von 6,5 g 5-(2-Bromäthoxy)-salicylamid und 8,9 g 1-(2-Allyloxy-phenoxy)-3-amino-2-propanol wird in einem Ölbad von 100° geschmolzen und magnetisch während 1 Stunde gerührt. Die Aufarbeitung analog Beispiel 11 ergibt 1-(2-Allyloxy-phenoxy)-3-[2-(carbamoyl-4-hydroxy-phenoxy)-äthylamino]-2-propanol vom Smp. 147—148° (aus Isopropanol).

Die als Ausgangsmaterial verwendete Salicylamid-Verbindung kann auf folgende Weise erhalten werden:

13a) 30,0 g 6-(2-Bromäthoxy)-2,3-dihydro-2,2-dimethyl-4H-1,3-benzoxazin-4-on werden in einem Gemisch von 100 ml Dioxan und 100 ml 6-n. Salzsäure unter Rühren 1,5 Stunden am Rückfluß gekocht. Die nach dem Eindampfen des Reaktionsgemisches erhaltenen Kristalle werden mit 50 ml Wasser gewaschen und im Vakuum getrocknet. Das so erhaltene 5-(2-Bromäthoxy)-salicylamid schmilzt bei 141—143°.

Beispiel 14

Durch Verwendung der entsprechenden 1-Aryloxy-3-amino-2-propanole werden analog Beispiel 13 die folgenden Verbindungen erhalten:

Das 1-[2-(3-Carbamoyl-4-hydroxy-phenoxy)-äthylamino]-3-(2-cyano-phenoxy)-2-propanol vom Smp. 121—124° (aus Äthanol),
das 1-[2-(3-Carbamoyl-4-hydroxy-phenoxy)-äthyl-amino]-3-[4-(2-methoxy-äthoxy)-phenoxy]-2-propanol vom Smp. 157—158° (aus Isopropanol),
das 1-[2-(3-Carbamoyl-4-hydroxy-phenoxy)-äthylamino]-3-[2-(2-propinyloxy)-phenoxy]-2-propanol vom Smp. 140—141° (aus Äthanol),
das 1-[2[3-Carbamoyl-4-hydroxy-phenoxy)-äthylamino]-3-[4-(2-methylthio-äthoxy)-phenoxy]-2-propanol, das ein Hydrochlorid vom Smp. 202—204° (aus Methanol) bildet,
das 1-[2-(3-Carbamoyl-4-hydroxy-phenoxy)-äthylamino]-3-(2-allyl-phenoxy)-2-propanol, das ein neutrales Fumarat vom Smp. 165—166° (aus Äthanol) bildet.

Beispiel 15

Analog Beispiel 1 erhält man durch Debenzylierung von 16 g rohem 1-{N-[2-(Carbamoyl-4-hydroxy-phenoxy)-1-methyl-äthyl]-benzylamino}-3-[4-(carbamoylmethoxy)-phenoxy]-2-propanol nach Kristallisation aus Dioxan das reine 1-[2-(3-Carbamoyl-4-hydroxy-phenoxy)-1-methyläthylamino]-3-[4-(carbamoylmethoxy)-phenoxy]-2-propanol vom Smp. 145—149° (sintert bei 140°) (Gemisch der Diastereomeren).

Beispiel 16

Analog Beispiel 3 erhält man durch Debenzylierung von 18 g rohem 1-{N-[2-(3-Carbamoyl-4-hydroxy-phenoxy)-äthyl]-benzylamino}-3-{2-[N'-(2-hydroxyäthyl-ureidomethyl]-phenoxy}-2-propanol nach Kristallisation aus Dimethylformamid-Äther das 1-[2-(3-Carbamoyl-4-hydroxy-phenoxy)-äthylamino]-3-{2-[N'-(2-hydroxyäthyl)-ureidomethyl]-phenoxy}-2-propanol vom Smp. 164—166°.

Das Ausgangsmaterial kann wie folgt hergestellt werden:

2-Benzyloxy-benzylamin

In einer Soxhlet-Apparatur werden 18,4 g Lithiumaluminiumhydrid in 1800 ml trockenem Äther unter Stickstoff-Atmosphäre bei einer Badtemperatur von 70° gekocht, wobei in der Soxhlet-Hülse 5,3 g 2-Benzyloxy-benzamid vorgelegt wird. Nach 21 Stunden wird das Reaktionsgemisch in ein Eisbad getaucht, und es werden nacheinander unter Rühren 18,4 ml Wasser, 18,4 ml 15%ige Natronlauge und 55 ml Wasser zugetropft. Die Temperatur darf maximal bis +10° steigen. Darauf wird das Gemisch bei 20° nachgerührt und der entstandene Niederschlag abgenutscht und mit Äther nachgewaschen. Das Filtrat wird am Vakuum eingedampft und das verbleibende Öl bei Eiskühlung mit 500 ml 10%iger Salzsäure und 400 ml Äther 2 Stunden gerührt. Das ausgefallene 2-Benzyloxy-benzylamin Hydrochlorid wird abgesaugt, mit Wasser und Äther gewaschen und unter Vakuum getrocknet. Smp. 190—191°.

2-Benzyloxy-benzylisocyanat

38,5 g 2-Benzyloxy-benzylamin Hydrochlorid werden in 400 ml destilliertem Toluol suspendiert und bei einer Badtemperatur von 140° erhitzt. Unter Rühren wird Phosgen eingeleitet, wobei nach ca. 50 Minuten eine klare Lösung entsteht. Nach weiteren 10 Minuten wird die Phosgenzugabe unterbrochen und für 1 Stunde weitergekocht. Darauf läßt man etwas abkühlen und destilliert das Toluol unter Vakuum ab. Eine Probe des verbleibenden Öls wurde im Kugelrohr destilliert: Sdp. Badtemp. 120°/0,06 Torr.

N-(2-Hydroxyäthyl)-N'-(2-benzyloxy-benzyl)-harnstoff

Zu einer Lösung von 36,8 ml Äthanolamin in 370 ml Methylenchlorid wird innert 50 Minuten eine Lösung von 73,6 g rohem 2-Benzyloxy-benzylisocyanat in 120 ml Methylenchlorid zugetropft. Die Reaktion ist schwach exotherm. Nach 2 Stunden wird die Reaktionslösung 3mal mit je 200 ml Wasser gewaschen und mit Natriumsulfat getrocknet. Methylenchlorid wird abdestilliert und der Rückstand aus Isopropanol umkristallisiert. Das erhaltene Produkt schmilzt bei 92—94°.

N-(2-Hydroxyäthyl)-N'-(2-hydroxy-benzyl)-harnstoff

59,6 g N-(2-Hydroxyäthyl)-N'-(2-benzyloxy-benzyl)-harnstoff werden in 600 ml Methanol gelöst und in Gegenwart von 6 g Pd/C-Katalysator (5%) hydriert. Nach 2 Stunden kommt die Hydrierung zum Stillstand. Der Katalysator wird abgenutscht und das Filtrat unter Vakuum eingedampft. Der Rückstand wird aus 350 ml Essigester umkristallisiert; das reine Produkt schmilzt bei 100—101°.

N-[2-(2,3-Epoxypropoxy)-benzyl]-N'-(2-hydroxyäthyl)-harnstoff

Ein Gemisch von 29,2 g N-(2-Hydroxyäthyl)-N'-(2-hydroxy-benzyl)-harnstoff, 440 ml Epichlorhydrin und 38,9 g Kaliumcarbonat wird während 6 Stunden bei 90° gerührt. Darauf werden die Feststoffe heiß abgenutscht, mit Acetonitril nachgewaschen und das Filtrat unter Vakuum eingedampft. Das verbleibende Öl kristallisiert beim Stehen und wird aus 320 ml Essigester, mit Kohlebehandlung, umkristallisiert. Das erhaltene Epoxid schmilzt bei 96—99°.

1-{N-[2-(3-Carbamoyl-4-hydroxy-phenoxy)-äthyl]-benzylamino}-
3-{2-[N'-(2-hydroxyäthyl)-ureidomethyl]-phenoxy}-2-propanol

Eine Lösung von 9,75 g des obigen Epoxids und 9,4 g N[(3-Carbamoyl-4-hydroxy-phenoxy)-äthyl]-benzylamin in 100 ml Isopropanol wird während 7 Stunden bei einer Badtemperatur von 95° gerührt. Die Lösung wird unter Vakuum eingedampft. Das erhaltene rohe Produkt kann direkt der Hydrogenolyse unterworfen werden.

Beispiel 17

Eine Mischung von 10,2 g [2-(2,3-Epoxy-propoxy)-phenyl]-2-propinyl-äther, 7,8 g 5-(2-Aminoäthoxy)-salicylamid und 25 ml Isopropanol wird unter Rühren 1 Stunde am Rückfluß gekocht. Der nach dem Eindampfen des Reaktionsgemisches verbleibende Rückstand wird in 30 ml Äthylacetat gelöst. Das so auskristallisierende 1-[2-(3-Carbamoyl-4-hydroxy-phenoxy)-äthylamino]-3-[2-propinyloxy)-phenoxy]-2-propanol schmilzt nach Umkristallisation aus Äthanol bei 140—141°.

17a) Das als Ausgangsmaterial benötigte 5-(2-Aminoäthoxy)-salicylamid kann durch Debenzylierung mittels Wasserstoff in Gegenwart eines Pd/C-Katalysators (5%ig) der entsprechenden N-Benzyl-Verbindung (analog Beispiel 3b) in Methanol hergestellt werden, es schmilzt bei 140°.

Beispiel 18

Analog Beispiel 17 erhält man unter Verwendung der entsprechend substituierten Epoxide

das 1-[2-(3-Carbamoyl-4-hydroxy-phenoxy)-äthylamino]-3-(2-cyano-phenoxy)-2-propanol vom Smp. 121—124° (aus Äthanol),
das 1-[2-(3-Carbamoyl-4-hydroxy-phenoxy)-äthylamino]-3-(2-chlor-phenoxy)-2-propanol vom Smp. 140—141° (aus Äthanol),

das 1-[2-(3-Carbamoyl-4-hydroxy-phenoxy)-äthylamino]-3-[4-(2-methylthio-äthoxy)-phenoxy]-2-propanol, das ein Hydrochlorid vom Smp. 202—204° bildet (aus Methanol),
das 1-(2-Allyl-phenoxy)-3-[2-(3-carbamoyl-4-hyroxy-phenoxy)-äthylamino]-2-propanol, dessen neutrales Fumarat bei 165—166° schmilzt (aus Äthanol),
das 1-[2-(3-Carbamoyl-4-hydroxy-phenoxy)-äthylamino]-3-[4-(2-methoxy-äthoxy)-phenoxy]-2-propanol vom Smp. 157—158° (aus Methanol),
das 1-(2-Allyloxy-phenoxy)-3-[2-(3-carbamoyl-4-hydroxy-phenoxy)-äthylamino]-2-propanol vom Smp. 147—148° (aus Methanol),
das 1-[2-(3-Carbamoyl-4-hydroxy-phenoxy)-äthylamino]-3-(3-methyl-4-methylthio-phenoxy)-2-propanol vom Smp. 139—141° (aus Acetonitril),
das 1-[2-(3-Carbamoyl-4-hydroxy-phenoxy)-äthylamino]-3-(3-methyl-4-methylsulfinyl-phenoxy)-2-propanol mit Doppel-Smp. 92° und 140°,
das 1-[2-(3-Carbamoyl-4-hydroxy-phenoxy)-äthylamino]-3-(3-methyl-1,2,4-thiadiazol-5-yloxy)-2-propanol nach Chromatographie an Kieselgel als amorphes Pulver vom Smp. 132° unter vorherigem Sintern.

Beispiel 19

3,09 g 4-(2,3-Epoxypropoxy)-benzimidazol-2-on und 4,29 g 5-[2-(Benzylamino)-äthoxy]-salicylamid werden in 80 ml Isopropanol 3 Stunden am Rückfluß gekocht. Dann wird das Lösungsmittel unter vermindertem Druck entfernt. Das zurückbleibende rohe N-[2-(Hydroxy-3-carbamoylphenoxy)-äthyl]-N-[3-(2-Oxo-benzimidazol-4-yloxy)-2-hydroxypropyl]-N-benzylamin wird in 80 ml Methanol gelöst, mit 3 ml einer 5-n. methanolischen Chlorwasserstofflösung versetzt und anschließend unter Zugabe von 0,8 g Pd/C-Katalysator (5%) in einer Hydrierapparatur unter Wasserstoff-Atmosphäre geschüttelt. Nach Beendigung der Wasserstoffaufnahme, die der berechneten Menge entspricht, wird vom Katalysator abfiltriert und das Filtrat unter vermindertem Druck konzentriert. Beim Abkühlen des Konzentrats kristallisiert das 4-{3-[2-(3-Carbamoyl-4-hydroxy-phenoxy)-äthylamino]-2-hydroxypropoxy}-benzimidazol-2-on-hydrochlorid, Smp. 148—152° (nach Umkristallisieren aus Methanol).

Beispiel 20

Durch katalytische Debenzylierung von 1-{N-[2-(4-Carbamoyl-3-hydroxy-phenoxy)-äthyl-benzylamino}-3-[4-(2-methoxy-äthoxy)-phenoxy]-2-propanol erhält man analog Beispiel 1 das 1-[2-(4-Carbamoyl-3-carbamoyl-3-hydroxy-3-hydroxy-phenoxy)-äthylamino]-3-[4-(2-methoxy-äthoxy)-phenoxy]-2-propanol vom Smp. 151—152° (aus Methanol).

Das Ausgangsmaterial wird auf folgende Weise hergestellt:

20a) 16,2 g 2,3-Dihydro-2,2-dimethyl-7-hydroxy-4H-1,3-benzoxazin-4-on werden analog Beispiel 3a) mit 84 ml 1,2-Dibromäthan umgesetzt und ergeben das 2,3-Dihydro-2,2-dimethyl-7-(2-bromäthoxy)-4H-1,3-benzoxazin-4-on vom Smp. 156—158° C (aus Isopropanol).

20b) 53 g 2,3-Dihydro-2,2-dimethyl-7-(2-bromäthoxy)-4H-1,3-benzoxazin-4-on und 94 g Benzylamin werden unter Rühren 3 Stunden gekocht. Das Reaktionsgemisch wird mit konz. Ammoniak alkalisch gestellt, und die organische Phase bei max. 50° eingedampft.

Das so erhaltene 4-[2-(Benzylamino)äthoxy]-salicylamid bildet ein Öl, dessen Hydrochlorid bei 252—254° C schmilzt (aus Methanol).

20c) Analog Beispiel 4a erhält man unter Verwendung des 4-[2-(Benzylamino)-äthoxy]-salicylamid das 1-{N-[2-(4-Carbamoyl-3-hydroxy-phenoxy)-äthyl]-benzylamino}-3-[4-(2-methoxy-äthoxy)-phenoxy]-2-propanol als Öl, das roh debenzyliert wird.

Beispiel 21

Analog Beispiel 8 erhält man durch Debenzylierung von rohem 1-[N-[2-(3-Carbamoyl-4-hydroxy-phenoxy)-äthyl]-benzylamino]-3-(4-benzyloxy-phenoxy)-2-propanol das 1-[2-(3-Carbamoyl-4-hydroxy-phenoxy)-äthylamino]-3-(4-hydroxy-phenoxy)-2-propanol vom Smp. 130—131° (aus Isopropanol); und aus dem 4-Carbamoyl-3-hydroxy-Isomeren das 1-[2-(4-Carbamoyl-3-hydroxy-phenoxy)-äthylamino]-3-(4-hydroxy-phenoxy)-2-propanol vom Smp. 148—151° (aus Methanol).

21a) Die Ausgangsstoffe kann man durch Umsetzen von Benzyl[4-(2,3-epoxy-propoxy)-phenyl]-äther mit 5- bzw. 4-[2-(Benzylamino-äthoxy]-salicylamid analog Beispiel 8b erhalten.

Beispiel 22

Analog Beispiel 13 erhält man unter Verwendung von 6-(2-Bromäthoxy)-salicylamid das 1-[2-(2-Carbamoyl-3-hydroxy-phenoxy)-äthylamino]-3-[4-(2-methoxy-äthoxy)-phenoxy]-2-propanol; Smp. 176—179° (aus Methanol).

22a) Das Ausgangsmaterial kann wie folgt hergestellt werden:

Ein Gemisch von 23,0 g 2,6-Dihydroxybenzamid, 20,7 g Kaliumcarbonat und 28,2 g 1,2-Dibromäthan wird in 300 ml Acetonitril 2—3 Stunden unter Rückfluß gerührt. Das Reaktionsgemisch wird noch warm filtriert, das Filtrat eingedampft und der Rückstand aus wenig Methanol umkristallisiert. Man erhält das 6-(2-Bromäthoxy)-salicylamid vom Smp. 120—121°.

Beispiel 23

Die Lösung von 2,24 g 5-(2-Amino-2-methylpropoxy)-salicylamid in 30 ml Dioxan wird nach Zugabe von 2,3 g 2-(2,3-Epoxy-propoxy)-benzonitril 7 Stunden unter Rückfluß gekocht und dann eingedampft. Der Eindampfrückstand wird zwischen 10 ml 2-n.Salzsäure und 100 ml Äthylacetat verteilt. Die saure, wäßrige Phase wird mit konz. Ammoniak-Lösung alkalisch gestellt, die Base mit Äthylacetat extrahiert und das Lösungsmittel abgedampft, wonach man ein Öl erhält, aus dem durch Kristallisation aus Isopropanol und Umkristallisation aus Äthylacetat das 1-[2-(3-Carbamoyl-4-hydroxy-phenoxy)-1,1-dimethyläthylamino]-3-(2-cyanophenoxy)-2-propanol vom Smp. 125—126° erhalten wird.

Das Ausgangsmaterial kann man wie folgt erhalten:

23a) Ein Gemisch von 84,3 g 2,3-Dihydroxy-2,2-dimethyl-6-hydroxy-4H-1,3-benzoxazin-4-on, 144,2 g Methansulfonsäure-(2-methyl-2-nitropropyl)-ester und 121 g trockenes Kaliumcarbonat in 440 ml Diäthylenglykol-dimethyläther wird 9 Stunden in einem Bad von ca. 150° gerührt. Das Reaktionsgemisch wird abgekühlt, auf 4000 ml Wasser gegossen und mit 3000 ml Äthylacetat extrahiert. Das durch Eindampfen der organischen Phase erhaltene Öl wird in 250 ml Dioxan gelöst und mit ca. 750 ml 2-n. Salzsäure bis zur sauren Reaktion versetzt. Die Lösung wird 1$^1/_2$ Stunden bei 80—100° gehalten, hierauf unter vermindertem Druck auf das halbe Volumen eingeengt und mit je 500 ml Äthylacetat 3mal extrahiert. Die vereinigten organischen Phasen werden mit 200 ml Wasser, dann mit gesättigter Natriumcarbonat-Lösung und schließlich mit gesättigter Kochsalzlösung gewaschen, über Magnesiumsulfat getrocknet und eingedampft. Das so erhaltene dunkelbraune Öl wird an 500 g Kieselgel chromatographiert. Durch Elution mit Äther erhält man kristallines 5-(2-Methyl-2-nitro-propoxy)-salicylamid vom Smp. 145—148°.

23b) 11,5 g 5-(2-Methyl-2-nitro-propoxy)-salicylamid werden in 150 ml Methanol bei 40—50° und 80 bar über 5 g Raney-Nickel bis zum Stillstand der Wasserstoff-Aufnahme hydriert. Durch Filtration und Eindampfen des Filtrats erhält man rohes 5-(2-Amino-2-methyl-propoxy)-salicylamid, das nach längerem Stehen aus Isopropanol kristallisiert und bei 115—117° schmilzt.

Beispiel 24

21,5 g 1-[2-(3-Carbamoyl-4-hydroxy-phenoxy)-1-methyl-äthylamino]-3-(3-hydroxy-4-methoxycarbonyl-phenoxy)-2-propanol wird mit einem Gemisch von 50 ml Dioxan und 500 ml konz. Ammoniak-Lösung versetzt. Das Reaktionsgemisch wird 1—2 Stunden gerührt und, sobald es homogen geworden ist, noch 3 Tage bei 20—30° stehengelassen. Durch Eindampfen erhält man 20 g rohes, kristallines 1-(4-Carbamoyl-3-hydroxy-phenoxy)-3-[2-(3-carbamoyl-4-hydroxy-phenoxy)-1-methyl-äthylamino]-2-propanol als Diastereomerengemisch vom Smp. 180—190°. Es bildet ein Hydrochlorid, das bei 238—243° schmilzt (aus Äthanol-Methanol).

Das Ausgangsmaterial kann auf folgende Weise hergestellt werden:

24a) Durch Kochen von 34 g 2,4-Dihydroxybenzoesäuremethyl-ester mit 185 g Epichlorhydrin und 35 g Kaliumcarbonat während 2—3 Stunden am Rückfluß und Chromatographie des Rohproduktes an 100 g Kieselgel (Elution mit Toluol) erhält man den 4-(2,3-Epoxy-propoxy)-salicylsäuremethylester vom Smp. 53—55°.

24b) 22,4 g 4-(2,3-Epoxy-propoxy)-salicylsäure-methylester und 30 g 5-(2-Benzylamino-propoxy)-salicylamid in 200 ml Isopropanol ergeben nach 40 Stunden Kochen und Aufarbeiten analog Beispiel 4a) das rohe 1-{N-[2-(3-Carbamoyl-4-hydroxy-phenoxy)-1-methyl-äthyl]-benzylamino}-3-(3-hydroxy-4-methoxycarbonyl-phenoxy)-2-propanol als hellen Schaum, das als Rohprodukt weiterverarbeitet wird.

24c) Eine Lösung von 46 g 1-{N-[2-(3-Carbamoyl-4-hydroxyphenoxy)-1-methyl-äthyl]-benzylamino}-3-(3-hydroxy-4-methoxycarbonyl-phenoxy)-2-propanol in 500 ml Methanol wird unter Zusatz von 5 g Pd/C Katalysator (5%) bei Normalbedingungen hydriert bis zur Aufnahme von 1 Äquivalent Wasserstoff. Das zum Teil auskristallisierte Produkt wird mit ca. 2000 ml heißem Methanol in Lösung gebracht und der Katalysator abfiltriert. Durch Einengen des Filtrates erhält man das 1-[2-(3-Carbamoyl-4-hydroxy-phenoxy)-1-methyläthylamino]-3-(3-hydroxy-4-methoxycarbonyl-phenoxy)-2-propanol vom Smp. 168—172° als Diastereomerengemisch.

Beispiel 25

5,2 g rohes 1-[N-{2-(3-Carbamoyl-2-hydroxyphenoxy)-äthyl}-benzylamino]-3-[4-(2-methoxyäthoxy)-phenoxy[-2-propanol werden analog Beispiel 4 hydriert und aufgearbeitet. Man erhält so nach Umkristallisation aus Isopropanol das 1-[2-(3-Carbamoyl-2-hydroxy-phenoxy)-äthylamino]-3-[4-(2-methoxy-äthoxy)-phenoxy]-2-propanol vom Smp. 125—129°.

Das Ausgangsmaterial wird auf folgende Weise hergestellt:

25a) 2,3-Dihydroxybenzoesäure-methylester wird in Anwesenheit von Kaliumcarbonat in Acetonitril mit 1,1 Äquivalenten 1-Dibenzylamino-2-chloräthan während 18 Stunden bei 82° umgesetzt. Der nach dem Aufarbeiten erhaltene rohe 3-(2-Dibenzylamino-äthoxy)-salicylsäuremethylester wird ohne weitere Reinigung weiterverwendet.

25b) Die nach Beispiel 25a) erhaltene Verbindung wird in Methanol gelöst und nach Zugabe von Palladium-auf-Kohle-Katalysator bis zur Aufnahme von 1,1-Äquivalenten Wasserstoff hydriert. Der Katalysator wird abfiltriert, das Lösungsmittel abgedampft, der Rückstand in Äthylacetat aufgenommen, die organische Phase mit Wasser gewaschen und eingedampft, wonach man den rohen 3-(2-Benzylaminoäthoxy)-salicylsäure-methylester als honigfarbenes Öl erhält.

25c) Das nach Beispiel 25b) erhaltene Produkt wird mit der 10fachen Gewichtsmenge konz. Ammoniak gerührt und nach erfolgter Lösung 4—5 Tage bei Zimmertemperatur stehengelassen. Die Lösung wird hierauf eingedampft, der Rückstand zwischen Wasser und Äthylacetat verteilt, die organische Phase über Magnesiumsulfat getrocknet und eingedampft, wonach man das 3-(2-Benzylamino-äthoxy)-salicylamid als gelbliches Öl erhält.

25d) Durch Umsetzen von 2,5 g 1-(2,3-Epoxy-propoxy)-4-(2-methoxy-äthoxy)-benzol mit 2,9 g rohem, nach Beispiel 25c) erhaltenen 3-(2-Benzylamino-äthoxy)-salicylamid analog Beispiel 4a) erhält man das 1-{N-[2-(3-Carbamoyl-2-hydroxyphenoxy)-äthyl]-benzylamino}-3-[4-methoxy-äthoxy)-phenoxy]-2-propanol als Öl, welches als solches weiterverarbeitet wird.

Beispiel 26

Eine Lösung von 2,5 g 1-Amino-3-[4-(2-methoxyäthoxy)-phenoxy]-2-propanol und 2,23 g (2,3-Dihydroxy-2,2-dimethyl-4H-1,3-benzoxazin-4-on-6-yloxy)-acetaldehyd in 20 ml Äthanol wird 3 Stunden unter Rückfluß gekocht. Nach dem Abkühlen setzt man 0,8 g Natriumborhydrid portionenweise unter Rühren zu und rührt noch weitere 3—4 Stunden beim Raumtemperatur nach.

Durch Zugabe von 2-n Salzsäure wird das überschüssige Natriumhydrid zersetzt, die Lösung hierauf eingedampft, der Rückstand mit Ammoniaklösung alkalisch gestellt und mit je 300 ml Äthylacetat 3mal extrahiert. Durch Eindampfen der über Magnesiumsulfat getrockneten, vereinigten Äthylacetat-Lösungen erhält man einen braunen Rückstand, aus dem durch wiederholte Umkristallisation aus Isopropanol das 1-[2-(-Carbamoyl-4-hydroxy-phenoxy)-äthylamino]-3-[4-(2-methoxy-äthoxy)-phenoxy]-2-propanol vom Smp. 157—158° erhalten wird.

Das Ausgangsmaterial wird auf folgende Weise hergestellt:

26a) Eine Lösung von 9,65 g 2,3-Dihydroxy-2,2-dimethyl-6-hydroxy-4H-1,3-benzoxazin-4-on und 9,1 g Allylbromid in 150 ml Acetonitril wird unter Zugabe von 10,3 g trockenem Kaliumcarbonat 5 Stunden unter Rückfluß gerührt. Das Reaktionsgemisch wird warm filtriert, das Filtrat eingedampft und die verbleibende Kristalle nach Verreiben mit Äther abgesaugt. Das so erhaltene rohe 2,3-Dihydro-2,2-dimethyl-6-(l-propen-3-yloxy)-4H-1,3-benzoxazin-4-on schmilzt bei 137—138°.

26b) Eine Lösung von 4,7 g 2,3-Dihydro-2,2-dimethyl-6-(1-propen-3-yloxy)-4H-1,3-benzoxazin-4-on in einem Gemisch von 50 ml Dioxan und 15 ml Wasser wird unter Rühren mit ca. 20 mg Osmiumtetroxid versetzt. Nach 15 Minuten wird portionsweise 8,6 g Natriummetaperiodat zugegeben, wobei die Temperatur bis 45° ansteigt. Nach 2 Stunden wird das Reaktionsgemisch filtriert, das Filtrat eingedampft und der Rückstand zwischen 20 ml Wasser und 200 ml Äthylacetat verteilt. Die organische Phase wird abgetrennt, über Natriumsulfat getrocknet und eingedampft, und das erhaltene Öl an 100 g Kieselgel chromatographiert. Durch Eluieren mit Äthylacetat und Eindampfen wird (2,3-Dihydro-2,2-dimethyl-4H-1,3-benzoxazin-4-on-6-yloxy)-acetaldehyd vom Smp. 153—163° erhalten.

Beispiel 27

Eine Lösung von 4,1 g 1-(2-Allyloxy-phenoxy)-3-[2-(3-carbamoyl-4-hydroxy-phenoxy)-äthylamino]-2-propanol in 100 ml Methanol wird nach Zusatz von 0,2 g Palladium-auf-Kohle-Katalysator bis zur Aufnahme von 1 Äquivalent Wasserstoff unter Normalbedingungen hydriert. Durch Filtration und Eindampfen der Lösung erhält man farblose Kristalle, die nach Umkristallisation aus Methanol bei 142—143° schmelzen und das 1-[2-(3-Carbamoyl-4-hydroxy-phenoxy)-äthylamino]-3-(2-propyloxy-phenoxy)-2-propanol darstellen.

## Beispiel 28

Analog Beispiel 8 erhält man unter Verwendung von 1-{N-[2-(3-Carbamoyl-4-hydroxy-phenoxy)-äthyl]-benzylamino}-3-(2-benzyloxy-phenoxy)-2-propanol als Ausgangsmaterial das 1-[2-(3-Carbamoyl-4-hydroxy-phenoxy)-äthylamino]-3-(2-hydroxy-phenoxy)-2-propanol, welches ein neutrales Fumarat vom Smp. 178—180° (aus Äthanol) bildet.

Der Ausgangsstoff kann analog Beispiel 8b) aus Benzyl-[2-(2,3-epoxy-propoxy)-phenyl]-äther und 5-[2-(Benzylamino)-äthoxy]-salicylamid erhalten werden.

## Beispiel 29

Analog Beispiel 4 erhält man aus

a) 1-{N-[2-(3-Carbamoyl-4-hydroxy-phenoxy)-äthyl]-benzylamino}-3-(3-Carbamoyl-4-hydroxy-phenoxy)-2-propanol das 1-[2-(3-Carbamoyl-4-hydroxy-phenoxy)-äthylamino]-3-(3-Carbamoyl-4-hydroxy-phenoxy)-2-propanol vom Smp. 212—215°, (aus Methanol);

b) 1-{N-[2-(3-Carbamoyl-4-hydroxy-phenoxy)-äthyl]-benzylamino}-3-(2-carbamoyl-phenoxy)-2-propanol das 1-[2-(3-Carbamoyl-4-hydroxy-phenoxy)-äthylamino]-3-(2-carbamoyl-phenoxy)-2-propanol;

c) 1-N{N-[2-(3-Carbamoyl-4-hydroxy-phenoxy)-äthyl]-benzylamino}-3-[4-(2-oxo-propoxy)-phenoxy]-2-propanol das 1-[2-(3-Carbamoyl-4-hydroxy-phenoxy)-äthylamino]-3-[4-(2-oxo-propoxy)-phenoxy)]-2-propanol vom Smp. 118—120° (aus Acetonitril).

## Beispiel 30

Analog Beispiel 17 erhält man unter Verwendung der entsprechend substituierten Epoxide:

das 1-[2-(3-Carbamoyl-4-hydroxy-phenoxy)-äthylamino]-3-[4-(2-acetamido-äthyl)-phenoxy]-2-propanol; Smp. des Hydrochlorids 223—224° (aus Methanol).

das 1-[2-(3-Carbamoyl-4-hydroxy-phenoxy)-äthylamino]-3-(4-acetamidomethyl-phenoxy)-2-propanol, Smp. 173—176° aus Methylcellosolve).

das 1-[2-(3-Carbamoyl-4-hydroxy-phenoxy)-äthylamino]-3-(4-carbamoylmethyl-phenoxy)-2-propanol, Smp. 181—182°, aus Dimethylformamid-Wasser).

## Beispiel 31

Eine Lösung von 25 g rohem 1-[N-4(3-Carbamoyl-4-hydroxy-phenoxy)-butyl]-benzylamino{-3-[4-(2-methoxy-äthoxy)-phenoxy]-2-propanol in 250 ml Methanol wird analog Beispiel 4 hydriert und aufgearbeitet. Das erhaltene kristalline Rohprodukt wird aus Isopropanol umkristallisiert und ergibt das 1-[4-(3-Carbamoyl-4-hydroxy-phenoxy)-butylamino]-3-[4-(2-methoxy-äthoxy)-phenoxy]-2-propanol vom Smp. 122—124°.

Der Ausgangsstoff wird auf folgende Weise herhestellt:

31a) Eine Suspension von 96,5 g 2,3-Dihydro-2,2-dimethyl-6-hydroxy-4H-1,3-benzoxazin-4-on und 76 g Kaliumcarbonat in 300 ml 1,4-Dibrombutan wird 5 Stunden in einem Bad von 120—130° gerührt. Das Reaktionsgemisch wird filtriert und das überschüssige 1,4-Dibrombutan bei ca. 1 Torr abdestilliert. Der kristalline Rückstand wird mit Äther verrieben und abgesaugt. Man erhält so rohes 2,3-Dihydro-2,2-dimethyl-6-(4-brombutoxy)-4H-1,3-benzoxazin-4-on vom Smp. 139—142°, welches für die weitere Umsetzung genügend rein ist.

31b) Eine Mischung von 65,6 g 2,3-Dihydro-2,2-dimethyl-6-(4-brombutoxy)-4H-1,3-benzoxazin-4-on, 85 g Benzylamin und 100 ml Wasser wird während 1 Stunde unter Rühren auf 110—120° erwärmt. Unter Eiskühlung wird das Reaktionsgemisch hierauf mit konz. Salzsäure sauer gestellt, wobei nach einigen Stunden ein Salzgemisch des 5-(4-Benzylamino-äthoxy)-salicylamid auskristallisiert. Die daraus mittels 20%igem Ammoniak freigesetzte Base wird mit Äthylacetat extrahiert und die organische Phase abgedampft. Der Rückstand bildet ein Öl, welches allmählich kristallisiert, Smp. 103—103°, sintert ab 86°).

31c) Eine Lösung von 15,7 g der gemäß Beispiel 21b erhaltenen Verbindung und 13,4 g 1-(2,3-Epoxypropoxy)-4-(2-methoxy-äthoxy)-benzol wird analog Beispiel 4a zum 1-{N-[4-(3-Carbamoyl-4-hydroxy-phenoxy)-butyl]-benzylamino}-3-[4-(2-methoxy-äthoxy)-phenoxy]-2-propanol umgesetzt und als solches weiterverarbeitet.

Beispiel 32

Ein Gemisch von 8,4 g 1-[2-(3-Carbamoyl-4-hydroxy-phenoxy)-äthylamino]-3-[4-(2-methoxy-äthoxy)-phenoxy]-2-propanol und 30 ml n-Butylamin wird in einem geschlossenen Gefäß unter Rotieren während 17 Stunden auf 160—170° erhitzt. Nach dem Abdampfen des Butylamins verbleibt ein kristalliner Rückstand, welcher aus Methanol umkristallisiert wird und 1-[2-(3-N-n-Butylcarbamoyl-4-hydroxy-phenoxy)-äthylamino]-3-[4-(2-methoxy-äthoxy)-phenoxy]-2-propanol vom Smp. 118—119° darstellt.

Beispiel 33

Ein Gemisch von 8,1 g 1-[2-(3-Carbamoyl-4-hydroxy-phenoxy)-äthylamino]-3-(2-propyloxy-phenoxy)-2-propanol und 50 ml einer 33%igen Lösung von Methylamin in Äthanol wird analog Beispiel 32 im geschlossenen Gefäß umgesetzt. Durch Eindampfen der erhaltenen Lösung erhält man ein Öl, welches durch Zugabe einer 5-n Lösung von Salzsäure in Methanol neutralisiert wird. Nach Zugabe von Äther bis zur beginnenden Trübung kristallisiert allmählich das 1-{2-[3-(N-Methylcarbamoyl)-4-hydroxy-phenoxy]-äthylamino}-3-(2-propyloxy-phenoxy)-2-propanol als Hydrochlorid vom Smp. 114—116° aus.

Auf analoger Weise erhält man unter Verwendung von Piperidin das 1-{2-[3-(N-Piperidino-Carbonyl)-4-hydroxy-phenoxy}-3-(2-propyloxy-phenoxy)-2-propanol als dickflüssiges Öl.

Beispiel 34

Eine Lösung von 6,7 g 5-(4-Aminobutoxy)-salicylamid in 60 ml Dimethylsulfoxid wird mit 7,3 g 2-(2,3-Epoxypropoxy)-benzonitril versetzt und das Gemisch 1 Stunde in einem Bad von 90° gerührt. Das Reaktionsgemisch wird auf 300 ml Wasser gegossen und mit je 200 ml Äthylacetat zweimal extrahiert. Aufarbeitung analog Beispiel 23 ergibt rohes 1-[4-(3-Carbamoyl-4-hydroxyphenoxy)-butylamino]-3-(2-cyano-phenoxy)-2-propanol als dickflüssiges Öl.

Das als Ausgangsmaterial benötigte 5-(4-Amino-butoxy)-salicylamid wird durch katalytische Debenzylierung von 5-(4-Benzylamino-butoxy)-salicylamid in Methanol unter Verwendung eines Palladium-auf Kohle-Katalysators (5%) erhalten, Smp. 78—81° (aus Äthanol).

Beispiel 35

Eine Lösung von 3,5 g 1-[2-(3-Cyano-4-hydroxy-phenoxy)-äthylamino]-3-[4-(2-methoxy-äthoxy)-phenoxy]-2-propanol in einem Gemisch von 15 ml konz. Salzsäure und 20 ml Dioxan wird 15 Stunden bei 20—25° gerührt. Das Reaktionsgemisch wird hierauf eingedampft und mit 10%iger, wäßriger Ammoniaklösung alkalisch gestellt. Das nach einigen Stunden abgeschiedene rohe 1-[2-(3-Carbamoyl-4-hydroxy-phenoxy)-äthylamino]-3-[4-(2-methoxy-äthoxy)-phenoxy[-2-propanol wird abfiltriert und aus einem Gemisch von Dioxan/Methanol (1 : 1) umkristallisiert, Smp. 157—158°.

Das als Ausgangsmaterial benötigte 1-[2-(3-Cyano-4-hydroxy-phenoxy)-äthylamino]-3-[4-(2-methoxy-äthoxy)-phenoxy]-2-propanol ist analog der im Beispiel 13 beschriebenen Arbeitsweise aus 1-[2-Methoxy-äthoxy)-phenoxy]-3-amino-2-propanol und 5-(2-Bromäthoxy)-2-hydroxy-benzonitril zugänglich. Das nach dem Aufarbeiten erhaltene Rohprodukt wird als solches weiterverwendet.

Beispiel 36

Tabletten enthaltend 20 mg an aktiver Substanz werden in folgender Zusammensetzung in üblicher Weise hergestellt:

Zusammensetzung:

| | |
|---|---|
| 1-[2-(3-Carbamoyl-4-hydroxy-phenoxy)-äthylamino]-3-[4-(2-methoxyäthoxy)-phenoxy]-2-propanol | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung

1-[2-(3-Carbamoyl-4-hydroxy-phenoxy)-äthylamino]-3-[4-(2-methoxyäthoxy)-phenoxy]-2-propanol wird mit einem Teil der Weizenstärke, mit Milchzucker und kolloidaler Kieselsäure gemischt und die Mischung durch ein Sieb getrieben. Ein weiterer Teil der Weizenstärke wird mit der 5fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpreßt.

Beispiel 37

Tabletten enthaltend 1 mg an aktiver Substanz werden in folgender Zusammensetzung in üblicher Weise hergestellt:

Zusammensetzung

| | |
|---|---|
| 1-(4-Hydroxy-phenoxy)-3-[2-(3-carbamoyl-4-hydroxy-phenoxy)-1-methyläthylamino]-2-propanol | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

Herstellung

1-(4-Hydroxy-phenoxy)-3-[2-(3-carbamoyl-4-hydroxy-phenoxy)-1-methyläthylamino]-2-propanol wird mit einem Teil der Weizenstärke, mit Milchzucker und kolloidaler Kieselsäure gemischt und die Mischung durch ein Sieb getrieben. Ein weiterer Teil der Weizenstärke wird mit der 5fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpreßt.

Beispiel 38

Kapseln enthaltend 10 mg an aktiver Substanz werden wie folgt auf übliche Weise hergestellt:

Zusammensetzung:

| | |
|---|---|
| 1-(-Allyloxy-phenoxy)-3-[2-[3)carbamoyl-4-hydroxy-phenoy)-äthylamino]-2-propanol | 2500 mg |
| Talkum | 200 mg |
| Kolloidale Kieselsäure | 50 mg |

Herstellung

Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Größe abgefüllt.

Beispiel 39

Eine sterile Lösung von 5,0 g 1-[2-(3-Carbamoyl-4-hydroxy-phenoxy)-äthylamino]-3-[4-(2-methoxy-äthoxy)-phenoxy]-2-propanol-methansulfonat in 5000 ml destilliertem Wasser wird in Ampullen zu 5 ml abgefüllt, die in 5 ml Lösung 5 mg Wirkstoff enthalten.

Beispiel 40

3,62 g 1-[2-(3-Carbamoyl-4-hydroxy-phenoxy)-äthylamino]-3-(4-hydroxy-phenoxy)-2-propanol werden unter Zusatz von 100,0 ml 0,10-n. Salzsäure mit 18 000 ml destilliertem Wasser auf ein Volumen von 18 100 ml gelöst. Die sterilisierte Lösung wird in Ampullen à 5,0 ml abgefüllt, in denen 1 mg Wirkstoff enthalten sind.

Beispiel 41

Anstelle der in den Beispielen 36 bis 40 als aktive Substanz verwendeten Verbindungen können auch folgende Verbindungen der Formel I, oder deren pharmazeutisch annehmbare nicht-toxischen Säureadditionssalze als Wirkstoffe in Tabletten, Dragées, Kapseln, Ampullenlösungen usw. verwendet werden:

1-[2-(3-Carbamoyl-4-hydroxy-phenoxy)-1-methyl-äthylamino]-3-[4-(2-methoxyäthoxy)phenoxy]-2-propanol,
1-[2-(4-Carbamoyl-3-hydroxy-phenoxy)-1-methyl-äthylamino]-3-[4-(2-methoxy-äthoxy)-phen oxy]-2-propanol,
1-[2-(3-Carbamoyl-4-hydroxy-phenoxy)-äthylamino]-3-{2-(2-hydroxyäthyl)-carbamoylmethoxy]-phenoxy}-2-propanol,
1-[2-(3-Carbamoyl-4-hydroxy-phenoxy)-äthylamino]-3-phenoxy-2-propanol,
1-[2-(3-Carbamoyl-4-hydroxy-phenoxy)-äthylamino]-3-{4-[2-(methoxycarbonyl)-äthoxy]phen-oxy}-2-propanol,
1-(4-Acetamido-phenoxy)-3-[2-(3-Carbamoyl-4-hydroxy-phenoxy)-äthylamino]-2-propanol,
1-[2-(3-Carbamoyl-4-hydroxy-phenoxy)-äthylamino]-3-(2-methyl-phenoxy)-2-propanol,
1-[2-(3-Carbamoyl-4-hydroxy-phenoxy)-äthylamino]-3-(3-methyl-phenoxy)-2-propanol,
1-[2-(3-Carbamoyl-4-hydroxy-phenoxy)-äthylamino]-3-(2-methyl-indol-4-yloxy)-2-propanol,
1-[2-(3-Carbamoyl-4-hydroxy-phenoxy)-äthylamino]-3-(trifluormethyl-phenoxy)-2-propanol,
1-(2-Acetyl-phenoxy)-3-[2-(3-carbamoyl-4-hydroxy-phenoxy)-äthylamino]-2-propanol,
1-{4-[2-(Acetamido)-äthoxy]-phenoxy}-3-[2-(3-carbamoyl-4-hydroxy-phenoxy)-äthylamino]-2-propanol,
1-[2-(3-Carbamoyl-4-hydroxy-phenoxy)-äthylamino]-3-(3-methyl-pyridin-2-yloxy)-2-propanol,
5-{3-[2-(3-Carbamoyl-4-hydroxy-phenoxy)-äthylamino]-2-hydroxy-propoxy}-1,2,3,4-tetrahydro-2,3-cis-naphthalindiol,
4-{2-Hydroxy-3-[3-carbamoyl-4-hydroxy-phenoxy)-äthylamino]-propoxy}-phenylacetamid,
4-{2-Hydroxy-3-[(3-carbamoyl-4-hydroxy-phenoxy)-äthylamino]-propoxy}-phenoxyacetamid.
N-{4-[2-Hydroxy-3-[(3-carbamoyl-4-hydroxy-phenoxy)-äthylamino]-propoxy]-phenyl} N',N'-dimethylharnstoff,
1-(4-Butyroylamino-2-acetyl-phenoxy)-3-[2-(3-carbamoyl-4-hydroxy-phenoxy)-äthylamino]-2-propanol,
1-[2-(3-Carbamoyl-4-hydroxy-phenoxy)-äthylamino]-3-(2-methoxy-phenoxy)-2-propanol,
1-[2-(3-Carbamoyl-4-hydroxy-phenoxy)-äthylamino]-3-(2,3-dimethyl-phenoxy)-2-propanol,
1-[2-(3-Carbamoyl-4-hydroxy-phenoxy)-äthylamino]-3-[3-(2-methoxy-äthoxy)-phenoxy]-2-propanol,

1-[2-(3-Carbamoyl-4-hydroxy-phenoxy)-äthylamino]-3-[2-(pyrrol-1-yl)-phenoxy]-2-propanol,
1-[2-(3-Carbamoyl-4-hydroxy-phenoxy)-äthylamino]-3-(3-methyl-4-methylsulfonyl-phenoxy)-2-propanol,
1-[2-(3-Carbamoyl-4-hydroxy-phenoxy)-äthylamino]-3-(1-naphthyloxy)-2-propanol,
5-{3-[2-(4-Carbamoyl-3-hydroxy-phenoxy)-1-methyl-äthylamino]-2-hydroxy-propoxy}-3,4-dihydro-2(1H)-chinolinon,
1-[3-(3-Carbamoyl-4-hydroxy-phenyl)-propylamino]-3-[4-(2-methoxy-äthoxy)-phenoxy]-2-propanol,
1-[2-(3-Carbamoyl-4-hydroxy-phenoxy)-1-methyl-äthylamino]-3-(4-methylcarbamoyl-phenoxy)-2-propanol,
1-[2-(3-Carbamoyl-4-hydroxy-phenoxy)-1-methyl-äthylamino]-3-[4-(2-methoxyäthyl)-phenoxy]-2-propanol,
1-(2-Allyloxy-carbamoyl-4-hydroxy-phenoxy)-1-methyläthylamino]-2-propanol,
1-[2-(3-Carbamoyl-4-hydroxy-phenoxy)-äthylamino]-3-(2-cyano-phenoxy)-2-propanol,
1-[2-(3-Carbamoyl-4-hydroxyphenoxy)-äthylamino]-3-[2-(2-propinyloxy)-phenoxy]-2-propanol,
1-[2-(3-Carbamoyl-4-hydroxy-phenoxy)-äthylamino]-3-[4-(2-methylthio-äthoxy)-phenoxy]-2-propanol,
1-[2-(3-Carbamoyl-4-hydroxy-phenoxy)-äthylamino]-3-[2-allyl-phenoxy]-2-propanol,
1-[2-(3-Carbamoyl-4-hydroxy-phenoxy)-1-methyläthylamino]-3-[4-(carbamoylmethoxy)-phen oxy]-2-propanol,
1-[2-(3-Carbamoyl-4-hydroxy-phenoxy)-äthylamino]-3-[2-[N'-(hydroxyäthyl)-ureidomethyl]-phenoxy]-2-propanol,
1-[2-(3-Carbamoyl-4-hydroxy-phenoxy)-äthylamino]-3-[2-(2-propinyloxy)-phenoxy]-2-propanol,
1-[2-(3-Carbamoyl-4-hydroxy-phenoxy)-äthylamino]-3-(2-cyano-phenoxy)-2-propanol,
1-[2-(3-Carbamoyl-4-hydroxy-phenoxy)-äthylamino]-3-(2-chlor-phenoxy)-2-propanol,
1-[2-(3-Carbamoyl-4-hydroxy-phenoxy)-äthylamino]-3-[4-(2-methylthioäthoxy)-phenoxy]-2-propanol,
1-[2-(3-Carbamoyl-4-hydroxy-phenoxy)-äthylamino]-3-(methyl-4-methylthio-phenoxy)-2-propanol,
1-[2-(3-Carbamoyl-4-hydroxy-phenoxy)-äthylamino]-3-(3-methyl-4-methylsulfinyl-phenoxy)-2-propanol,
1-[2-(3-Carbamoyl-4-hydroxy-phenoxy)-äthylamino]-3-(3-methyl-1,2,4-thiadiazol-5-yloxy)-2-propanol,
4-{3-[2-(3-Carbamoyl-4-hydroxy-phenoxy)-äthylamino]-2-hydroxy-propoxy}-benzimidazol-2-on-hydrochlorid,
1-[2-(4-Carbamoyl-3-hydroxy-phenoxy)-äthylamino]-3-[4-(2-methoxy-äthoxy)-phenoxy]-2-propanol,
1-[2-(2-Carbamoyl-3-hydroxy-phenoxy)-äthylamino]-3-[4-(2-methoxy-äthoxy)-phenoxy]-2-propanol,
1-[2-(3-Carbamoyl-4-hydroxy-phenoxy)-1,1-dimethyl-äthylamino]-3-[4-(methylcarbamoyl)-phenoxy]-2-propanol,
1-(4-Carbamoyl-3-hydroxy-phenoxy)-3-[2-(3-carbamoyl-4-hydroxy-phenoxy)-1-methyl-äthylamino]-2-propanol als Diastereomerengemisch,
1-[2-(3-Carbamoyl-2-hydroxy-phenoxy)-äthylamino]-3-[4-(2-methoxy-äthoxy)-phenoxy]-2-propanol,
1-[2-(3-Carbamoyl-4-hydroxy-phenoxy)-äthylamino]-3-(2-propyloxy-phenoxy)-2-p-ropanol,
1-[2-(3-Carbamoyl-4-hydroxy-phenoxy)-äthylamino]-3-(2-hydroxy-phenoxy)-2-propanol,
1-[2-(3-Carbamoyl-4-hydroxy-phenoxy)-äthylamino]-3-(3-carbamoyl-4-hydroxy-phenoxy)-2-propanol,
1-[2-(3-Carbamoyl-4-hydroxy-phenoxy)-äthylamino]-3-(2-carbamoyl-phenoxy)-2-propanol,
1-[2-(3-Carbamoyl-4-hydroxy-phenoxy)-äthylamino]-3-[4-(2-oxo-propoxy)-phenoxy]-2-propanol,
1-[2-(3-Carbamoyl-4-hydroxy-phenoxy)-äthylamino]-3-[4-(2-acetamidoäthyl)-phenoxy]-2-propanol,
1-[2-(3-Carbamoyl-4-hydroxy-phenoxy)-äthylamino]-3-[4-(acetamidomethyl)-phenoxy]-2-propanol,
1-[2-(3-Carbamoyl-4-hydroxy-phenoxy)-äthylamino]-3-(4-carbamoylmethyl-phenoxy)-2-propanol,
1-[4-(3-Carbamoyl-4-hydroxy-phenoxy)-butylamino]-3-[4-(2-methoxy-äthoxy)-phenoxy]-2-propanol,
1-{2-[3-(N-n-Butylcarbamoyl)-4-hydroxy-phenoxy]-äthylamino}3-[4-(2-methoxy-äthoxy)-phenoxy]-2-propanol,
1-{2-[3-(N-Methylcarbamoyl)-4-hydroxy-phenoxy]-äthylamino}-3-(2-propyloxy-phenoxy)-2-propanol oder das
1-[4-(3-Carbamoyl-4-hydroxy-phenoxy)-butylamino]-3-(2-cyano-phenoxy)-2-propanol.

**Patentansprüche für die Vertragsstaaten: BE, CH, DE, FR, GB, IT, LU, NL, SE**

1. Derivate des 3-Amino-1,2-propandiols der Formel

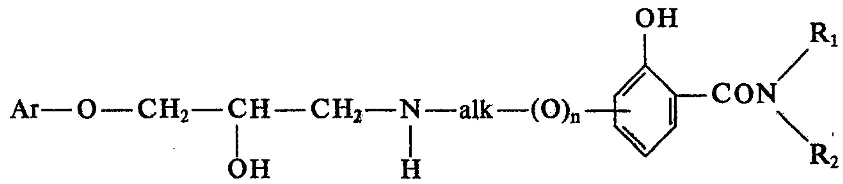

(I)

in welcher Ar gegebenenfalls substituiertes Aryl, n die Zahl 0 oder 1 und alk Alkylen mit 2—5 Kohlenstoffatomen bedeuten, wobei das Stickstoffatom und das Sauerstoffatom oder, falls n für Null steht, der Phenylrest durch mindestens zwei Kohlenstoffatome voneinander getrennt sind, und $R_1$ und $R_2$ unabhängig voneinander je Wasserstoff oder Niederalkyl mit bis zu 7 Kohlenstoffatomen, oder zusammen Niederalkylen, Oxaniederalkylen, Thianiederalkylen oder Azaniederalkylen mit jeweils bis zu 7 Kohlenstoffatomen, oder N-Niederalkyl-azaniederalkylen mit jeweils bis zu 7 Kohlenstoffatomen im Niederalkyl- bzw. Azaniederalkylenteil darstellen.

2. Verbindungen der Formel I gemäß Anspruch 1, worin Ar mono- oder bicyclisches carbocyclisches Aryl oder mono- oder bicyclisches, über ein Ringkohlenstoffatom gebundenes, höchstens zwei Stickstoffatome und/oder ein Sauerstoff- oder Schwefelatom als Ringglieder enthaltendes Heteroaryl bedeutet, welche Reste unsubstituiert oder ein- oder mehrfach substituiert sein können, wobei als Substituenten gegebenenfalls substituiertes Niederalkyl, Niederalkenyl oder Niederalkoxy mit jeweils bis zu 7 Kohlenstoffatomen, weiter Niederalkenyloxy, Niederalkinyl, Niederalkinyloxy mit jeweils bis zu 7 Kohlenstoffatomen, Cyan und/oder Nitro, und/oder direkt oder an vorgenanntes Niederalkyl, Niederalkenyl oder Niederalkoxy mit jeweils bis zu 7 Kohlenstoffatomen gebundenes Niederalkanoyl mit bis zu 7 Kohlenstoffatomen, verestertes oder amidiertes Carboxyl, Niederalkylsulfunyl, Niederalkylsulfonyl mit jeweils bis zu 7 Kohlenstoffatomen, Sulfamoyl oder Niederalkylsulfamoyl mit bis zu 7 Kohlenstoffatomen, und/oder direkt oder an vorgenanntes Niederalkyl mit bis zu 7 Kohlenstoffatomen oder in höherer als der 1-Stellung an vorgenanntes Niederalkoxy mit bis zu 7 Kohlenstoffatomen gebundenes Halogen, gegebenenfalls veräthertes oder verestertes Hydroxy, wie Hydroxy, oder als nicht direkt gebundenes Substituent, wiederum Niederalkoxy mit bis zu 7 Kohlenstoffatomen, ferner Phenylniederalkoxy mit bis zu 7 Kohlenstoffatomen im Niederalkoxyteil, oder Niederalkenyloxy mit bis zu 7 Kohlenstoffatomen, veräthertes Mercapto, gegebenenfalls substituiertes Amino, Pyrrol-1-yl, Acylamino, gegebenenfalls durch Niederalkyl oder Hydroxyniederalkyl mit jeweils bis zu 7 Kohlenstoffatomen, oder Cycloalkyl mit 5 bis 7 Ringgliedern substituiertes Ureido, oder Niederalkylsulfonylamino mit bis zu 7 Kohlenstoffatomen vorliegen können, und in bicyclischen Resten Ar der nicht direkt mit der Äthergruppe verbundene Ring auch mindestens partiell hydriert und in diesem Fall auch durch Oxo substituiert sein kann, und n für die Zahl 0 oder 1 steht, und alk einen Alkylenrest mit 2 bis 4 Kohlenstoffatomen bedeutet, wobei das Stickstoffatom und das Sauerstoffatom oder, falls n für 0 steht, der Phenylrest durch 2 bis 3 Kohlenstoffatome voneinander getrennt sind, und $R_1$ und $R_2$ die im Anspruch 1 angegebene Bedeutung haben.

3. Verbindungen der Formel I, gemäß Anspruch 1 worin Ar mono- oder bicyclisches carbocyclisches Aryl oder mono- oder bicyclisches, über ein Ringkohlenstoffatom gebundenes, höchstens zwei Stickstoffatome und/oder ein Sauerstoff- oder Schwefelatom als Ringglieder enthaltendes Heteroaryl bedeutet, welche Reste unsubstituiert oder ein- bis dreifach substituiert sein können, wobei als Substituenten gegebenenfalls in untenstehend angegebener Weise substituierte Niederalkyl, Niederalkenyl oder Niederalkoxy mit jeweils bis zu 7 Kohlenstoffatomen, weiter Niederalkenyloxy, Niederalkinyl, Niederalkinyloxy mit jeweils bis zu 7 Kohlenstoffatomen, Cyan und/oder Nitro und/oder direkt oder an vorgenanntes Niederalkyl, Niederalkenyl oder Niederalkoxy mit jeweils bis zu 7 Kohlenstoffatomen gebundenes Niederalkanoyl mit bis zu 7 Kohlenstoffatomen, Niederalkoxycarbonyl mit bis zu 7 Kohlenstoffatomen im Niederalkoxyteil, Carbamoyl, Niederalkylcarbamoyl oder (Hydroxyniederalkyl)-carbamoyl mit jeweils bis zu 7 Kohlenstoffatomen im Niederalkyl- bzw. (Hydroxyniederalkyl)-teil, Niederalkylsulfinyl oder Niederalkylsulfonyl mit jeweils bis zu 7 Kohlenstoffatomen, Sulfamoyl oder Niederalkylsulfamoyl mit bis zu 7 Kohlenstoffatomen, und/oder direkt oder an vorgenanntes Niederalkyl mit bis zu 7 Kohlenstoffatomen, oder in höherer als der 1-Stellung an vorgenanntes Niederalkoxy mit bis zu 7 Kohlenstoffatomen gebundenes Halogen, Hydroxy oder, als nicht direkt gebundener Substituent, wiederum Niederalkoxy mit bis zu 7 Kohlenstoffatomen, Phenylniederalkoxy mit bis zu 7 Kohlenstoffatomen im Niederalkoxyteil, Niederalkanoyloxy, oder Niederalkylthio mit jeweils bis zu 7 Kohlenstoffatomen, Amino, Niederalkylamino mit bis zu 7 Kohlenstoffatomen, Diniederalkylamino mit jeweils bis zu 7 Kohlenstoffatomen in den Niederalkylteilen, Alkylenamino oder Oxaalkylenamino, Pyrrol-1-yl, Niederalkanoylamino mit bis zu 7 Kohlenstoffatomen, oder Niederalkoxycarbonylamino mit bis zu 7 Kohlenstoffatomen im Niederalkoxyteil, gegebenenfalls durch Niederalkyl oder Hydroxyniederalkyl mit jeweils bis zu 7 Kohlenstoffatomen, oder Cycloalkyl mit 5 bis 7 Ringgliedern substituiertes Ureido, oder Niederalkylsulfonylamino mit bis zu 7 Kohlenstoffatomen vorliegen können, und in

bicyclischen Resten Ar der nicht direkt mit der Äthergruppe verbundene Ring auch mindestens partiell hydriert und in diesem Fall auch durch Oxo substituiert sein kann, und n die Zahl 0 oder 1, und alk einen Alkylenrest mit 2 bis 4 Kohlenstoffatomen bedeutet, wobei das Stickstoffatom und das Sauerstoffatom, oder, falls n für 0 steht, der Phenylrest durch 2 bis 3 Kohlenstoffatome voneinander getrennt sind, und $R_1$ und $R_2$ die im Anspruch 1 angegebene Bedeutung haben.

4. Verbindung der Formel I gemäß Anspruch 1, worin Ar Phenyl, Naphthyl oder 1,2,3,4-Tetrahydro-5-naphthyl bedeutet, welche Reste unsubstituiert oder ein- bis dreifach substituiert sein können, wobei als Substituenten gegebenenfalls in untenstehend angegebener Weise substituiertes Niederalkyl, Niederalkenyl oder Niederalkoxy mit jeweils bis zu 7 Kohlenstoffatomen, weiter Niederalkenyloxy, Niederalkinyloxy mit jeweils bis zu 7 Kohlenstoffatomen und/oder Cyan und/oder direkt oder an vorgenanntes Niederalkyl, Niederalkenyl oder Niederalkoxy mit jeweils bis zu 7 Kohlenstoffatomen gebundenes Niederalkanoyl mit bis zu 7 Kohlenstoffatomen, Niederalkoxycarbonyl mit bis zu 7 Kohlenstoffatomen im Niederalkoxyteil, Carbamoyl, Niederalkylcarbamoyl oder (Hydroxyniederalkyl)-carbamoyl mit jeweils bis zu 7 Kohlenstoffatomen im Niederalkyl- bzw. (Hydroxyniederalkyl)-teil, Niederalkylsulfinyl oder Niederalkylsulfonyl mit jeweils bis zu 7 Kohlenstoffatomen, Sulfamoyl oder Niederalkylsulfamoyl mit bis zu 7 Kohlenstoffatomen, und/oder direkt oder an vorgenanntes Niederalkyl mit bis zu 7 Kohlenstoffatomen oder in höherer als der 1-Stellung an vorgenanntes Niederalkoxy mit bis zu 7 Kohlenstoffatomen gebundenes Halogen, Hydroxy oder, als nicht direkt gebundener Substituent, wiederum Niederalkoxy mit bis zu 7 Kohlenstoffatomen, Phenylniederalkoxy mit bis zu 7 Kohlenstoffatomen im Niederalkoxyteil, Niederalkanoyloxy oder Niederalkylthio mit jeweils bis zu 7 Kohlenstoffatomen, Amino, Niederalkylamino mit bis zu 7 Kohlenstoffatomen, Diniederalkylamino mit jeweils bis zu 7 Kohlenstoffatomen in den Niederalkylteilen, Alkylenamino oder Oxaalkylenamino, Pyrrol-1-yl, Niederalkanoylamino mit bis zu 7 Kohlenstoffatomen, Niederalkoxycarbonylamino mit bis zu 7 Kohlenstoffatomen im Niederalkoxyteil, gegebenenfalls durch Niederalkyl mit bis zu 7 Kohlenstoffatomen oder Cycloalkyl mit 5 bis 7 Ringgliedern substituiertes Ureido, oder Niederalkylsulfonylamino mit bis zu 7 Kohlenstoffatomen vorliegen können, und n die Zahl 0 oder 1 und alk einen Alkylenrest mit 2 bis 4 Kohlenstoffatomen bedeutet, wobei das Stickstoffatom und das Sauerstoffatom, oder, falls n für 0 steht, der Phenylrest durch 2 bis 3 Kohlenstoffatome voneinander getrennt sind, und $R_1$ und $R_2$ unabhängig voneinander je Wasserstoff oder Niederalkyl mit bis 7 Kohlenstoffatomen darstellen, oder zusammen mit dem Stickstoffatom der Amidgruppen Morpholino bilden.

5. Verbindungen der Formel I gemäß Anspruch 1, worin Ar Phenyl, welches unsubstituiert oder ein- bis dreifach substituiert sein kann, wobei als Substituenten gegebenenfalls in untenstehend angegebener Weise substituiertes Niederalkyl oder Niederalkoxy mit jeweils bis zu 7 Kohlenstoffatomen, weiter Niederalkenyl, Niederalkenyloxy oder Niederalkinyloxy mit jeweils bis zu 7 Kohlenstoffatomen, und/oder Cyan, und/oder direkt oder an vorgenanntes Niederalkyl oder Niederalkoxy mit jeweils bis zu 7 Kohlenstoffatomen gebundenes Niederalkanoyl mit bis zu 7 Kohlenstoffatomen, Niederalkoxycarbonyl mit bis zu 7 Kohlenstoffatomen im Niederalkoxyteil, Carbamoyl, Niederalkylcarbamoyl oder (Hydroxyniederalkyl)-carbamoyl mit jeweils bis zu 7 Kohlenstoffatomen im Niederalkyl- bzw. (Hydroxyniederalkyl)-teil, Niederalkylsulfinyl oder Niederalkylsulfonyl mit jeweils bis zu 7 Kohlenstoffatomen, und/oder direkt oder an vorgenanntes Niederalkyl mit bis zu 7 Kohlenstoffatomen oder in höherer als der 1-Stellung an vorgenanntes Niederalkoxy mit bis zu 7 Kohlenstoffatomen gebundenes Halogen, Hydroxy oder, als nicht direkt gebundenes Substituent, wiederum Niederalkoxy mit bis zu 7 Kohlenstoffatomen, ferner Phenylniederalkoxy mit bis zu 7 Kohlenstoffatomen im Niederalkoxyteil, Niederalkylthio mit bis zu 7 Kohlenstoffatomen, Alkylen- oder Oxaalkylenamino, Pyrrol-1-yl, Niederalkanoylamino mit bis zu 7 Kohlenstoffatomen, Niederalkoxycarbonylamino mit bis zu 7 Kohlenstoffatomen im Niederalkoxyteil, oder gegebenenfalls durch Niederalkyl mit bis zu 7 Kohlenstoffatomen substituiertes Ureido vorliegen können, und n die Zahl 0 oder 1, und alk einen Alkylenrest mit 2 bis 4 Kohlenstoffatomen bedeutet, wobei das Stickstoffatom und das Sauerstoffatom oder, falls n für 0 steht, der Phenylrest durch 2 bis 3 Kohlenstoffatomen voneinander getrennt sind, und $R_1$ und $R_2$ unabhängig je Wasserstoff oder Niederalkyl mit bis zu 7 Kohlenstoffatomen darstellen.

6. Verbindungen der Formel I gemäß Anspruch 1, worin Ar Phenyl, welches unsubstituiert oder ein- bis dreifach substituiert sein kann, wobei als Substituenten gegebenenfalls in untenstehend angegebener Weise substituiertes Niederalkyl oder Niederalkoxy mit jeweils bis zu 7 Kohlenstoffatomen, weiter Niederalkenyl, Niederalkenyloxy oder Niederalkinyloxy mit jeweils bis zu 7 Kohlenstoffatomen, und/oder Cyan, und/oder direkt oder an vorgenanntes Niederalkyl oder Niederalkoxy mit jeweils bis zu 7 Kohlenstoffatomen gebundenes Niederalkanoyl mit bis zu 7 Kohlenstoffatomen, Carbamoyl, Niederalkylcarbamoyl oder (Hydroxyniederalkyl)-carbamoyl mit jeweils bis zu 7 Kohlenstoffatomen im Niederalkyl- bzw. (Hydroxyniederalkyl)-teil, Niederalkylsulfinyl oder Niederalkylsulfonyl mit jeweils bis zu 7 Kohlenstoffatomen, und/oder direkt oder an vorgenanntes Niederalkyl mit bis zu 7 Kohlenstoffatomen, oder in höherer als der 1-Stellung an vorgenanntes Niederalkoxy mit bis zu 7 Kohlenstoffatomen gebundenes Fluor oder Chlor, Hydroxy, oder, als nicht direkt gebundener Substituent wiederum Niederalkoxy mit bis zu 7 Kohlenstoffatomen, ferner Phenylniederalkoxy mit bis zu 7 Kohlenstoffatomen im Niederalkoxyteil, Niederalkylthio mit bis zu 7 Kohlenstoffatomen, Pyrrol-1-yl, Niederalkanoylamino mit bis zu 7 Kohlenstoffatomen, Niederalkoxycarbonylamino mit bis zu 7 Kohlenstoffatomen im Niederalkoxyteil, oder Ureido vorliegen können, und n die Zahl 1, und alk einen Alkylenrest mit 2 bis 4

Kohlenstoffatomen bedeutet, wobei das Stickstoffatom und das Sauerstoffatom durch 2 Kohlenstoffatome voneinander getrennt sind, und $R_1$ und $R_2$ Wasserstoff darstellen.

7. Verbindungen der Formel I gemäß Anspruch 1, worin der die Amid- und die Hydroxygruppe tragende Phenylrest vorzugsweise in seiner 4-Stellung und vor allem in seiner 5-Stellung mit dem restlichen Molekül verbunden ist.

8. 1-[2-(3-Carbamoyl-4-hydroxy-phenoxy)-äthylamino]-3-[4-(2-methoxyäthoxy)-phenoxy]-2-propanol.

9. 1-(4-Hydroxy-phenoxy)-3-[2-(3-carbamoyl-4-hydroxy-phenoxy)-1-methyläthylamino]-2-propanol.

10. 1-(2-Allyloxy-phenoxy)-3-[2-(3-carbamoyl-4-hydroxy-phenoxy)-äthylamino]-2-propanol.

11. 1-[4-{2-(Acetamido)-äthoxy]-phenoxy}-3-[2-(3-carbamoyl-4-hydroxy-phenoxy)-äthylamino]-2-propanol.

12. 1-[2-(3-Carbamoyl-4-hydroxy-phenoxy)-äthylamino]-3-[4-(2-oxo-propoxy)-phenoxy]-2-propanol.

13. 1-[2-(4-Carbamoyl-3-hydroxy-phenoxy)-1-methyl-äthylamino]-3-[4-(2-methoxy-äthoxy)-phenoxy]-2-propanol.

14. 4-[2-Hydroxy-3-[(3-carbamoyl-4-hydroxy-phenoxy)-äthylamino-propoxy]-phenylacetamid.

15. 1-[2-(3-Carbamoyl-4-hydroxy-phenoxy)-äthylamino]-3-{4-[2-methoxycarbonylamino)-äthoxy]-phenoxy}-2-propanol.

16. 1-[2-(3-Carbamoyl-4-hydroxy-phenoxy)-äthylamino]-3-(3-methyl-4-methylsulfinyl-phenoxy)-2-propanol.

17. 1-[2-(3-Carbamoyl-4-hydroxy-phenoxy)-äthylamino]-3-(2-cyano-phenoxy)-2-propanol.

18. 1-[2-(3-Carbamoyl-4-hydroxy-phenoxy)-äthylamino]-3-(2-propyloxy-phenoxy)-2-propanol.

19. 1-[2-(3-Carbamoyl-4-hydroxy-phenoxy)-äthylamino]-3-[2-(pyrrol-1-yl)-phenoxy]-2-propanol.

20. 1-[2-(3-Carbamoyl-4-hydroxy-phenoxy)-äthylamino]-3-[2-(2-propinyloxy)-phenoxy]-2-propanol.

21. 1-[2-(3-Carbamoyl-4-hydroxy-phenoxy)-äthylamino]-3-(2-methoxy-phenoxy)-2-propanol.

22. 1-[2-(3-Carbamoyl-4-hydroxy-phenoxy)-äthylamino]-3-(2-methyl-indol-4-yloxy)-2-propanol.

23. 1-(2-Acetyl-phenoxy)-3-[2-(3-Carbamoyl-4-hydroxy-phenoxy)-äthylamino]-2-propanol.

24. 5-{3-[2-(3-Carbamoyl-4-hydroxy-phenoxy)-äthylamino]-2-hydroxy-propoxy}-1,2,3,4-tetrahydro-2,3-cis-naphthalindiol.

25. 1-[2-(3-Carbamoyl-4-hydroxy-phenoxy)-äthylamino]-3-(3-methyl-phenoxy)-2-propanol.

26. 1-[2-(3-Carbamoyl-4-hydroxy-phenoxy)-äthylamino]-3-(4-hydroxy-phenoxy)-2-propanol.

27. Verbindungen der Formel I gemäß Anspruch 1, oder deren Salze zur Anwendung in einem Verfahren zur therapeutischen Behandlung des menschlichen oder tierischen Körpers.

28. Verbindungen der Formel I gemäß Anspruch 1, oder deren Salze mit spezifischer Wirkung auf β-adrenerge Rezeptoren.

29. Salze von Verbindungen der Ansprüche 1 bis 26.

30. Pharmazeutisch verwendbare, nicht-toxische Säureadditionssalze von Verbindungen der Ansprüche 1—26.

31. Pharmazeutische Präparate enthaltend eine der Verbindungen der Ansprüche 1—26 und 30.

32. Verwendung von Verbindungen der Formel I gemäß Anspruch 1 und pharmazeutisch verwendbaren, nicht toxischen Säureadditionssalzen solchen Verbindungen zur Herstellung von pharmazeutischen Präparaten.

33. Verfahren zur Herstellung von Derivaten des 3-Amino-1,2-propandiols der Formel

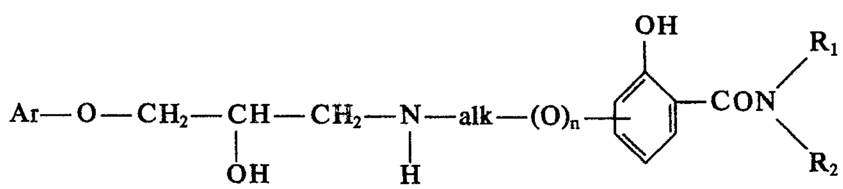

(I)

in welcher Ar gegebenenfalls substituiertes Aryl, n die Zahl 0 oder 1 und alk Alkylen mit 2—5 Kohlenstoffatomen bedeuten, wobei das Stickstoffatom und das Sauerstoffatom oder, falls n für Null steht, der Phenylrest durch mindestens zwei Kohlenstoffatome voneinander getrennt sind, und $R_1$ und $R_2$ unabhängig voneinander je Wasserstoff oder Niederalkyl mit bis zu 7 Kohlenstoffatomen, oder zusammen Niederalkylen, Oxaniederalkylen, Thianiederalkylen oder Azaniederalkylen mit jeweils bis zu 7 Kohlenstoffatomen, oder N-Niederalkyl-azaniederalkylen mit jeweils bis zu 7 Kohlenstoffatomen im Niederalkyl- bzw. Azaniederalkylenteil darstellen, dadurch gekennzeichnet, daß man

a) eine Verbindung der Formel

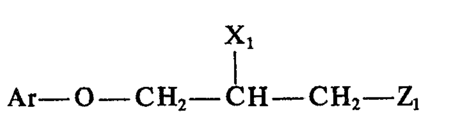

(II)

mit einer Verbindung der Formel

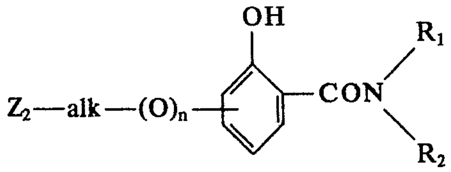
(III)

worin eine der Gruppen $Z_1$ und $Z_2$ eine reaktionsfähige veresterte Hydroxygruppe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, und Ar, n, alk, $R_1$ und $R_2$ obige Bedeutung haben, umsetzt, oder

b) in einer Verbindung der Formel

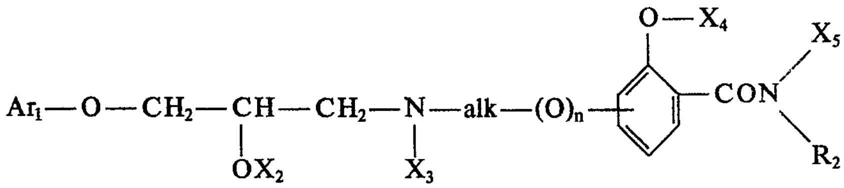
(IV)

worin $Ar_1$ die Bedeutung von Ar hat oder einen Rest Ar bedeutet, der durch 1 bis 2 in Hydroxy überführbare Gruppen substituiert ist, $X_2$, $X_3$ und $X_4$ jeweils Wasserstoff oder einen durch Wasserstoff ersetzbaren Substituenten bedeuten und $X_5$ für $R_1$ steht oder $X_2$ und $X_3$ und/oder $X_4$ und $X_5$ zusammen einen zweiwertigen, durch zwei Wasserstoffatome ersetzbaren Rest bedeuten, mit der Maßgabe, daß mindestens einer der Reste $X_2$, $X_3$ und $X_4$ von Wasserstoff verschieden ist oder mindestens $Ar_1$ einen Rest Ar bedeutet, der durch 1 bis 2 in Hydroxy überführbare Gruppen substituiert ist, oder mindestens $X_2$ und $X_3$ zusammen einen zweiwertigen, durch zwei Wasserstoffatome ersetzbaren Rest darstellen oder in einem Salz davon die von Wasserstoff verschiedenen der Gruppe $X_2$, $X_3$ und $X_4$ bzw. $X_2$ und $X_3$ zusammen oder $X_4$ und $X_5$ zusammen durch Wasserstoffatome ersetzt und/oder in einem Rest $Ar_1$ vorhandenes substituiertes Hydroxy in freis Hydroxy überführt,

c) in einer Verbindung der Formel

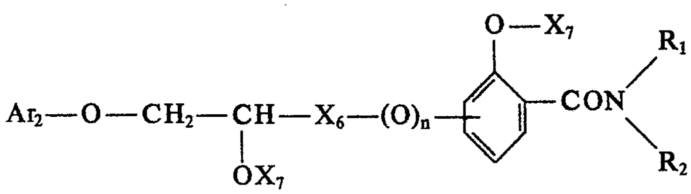
(V)

worin $X_6$ eine reduzierbare Gruppe der Formeln

$$-CH=N-alk- \quad \text{(Va)}$$

bzw.

$$-CH_2-N=alk_1- \quad \text{(Vb)}$$

ist, wobei $alk_1$ für einen dem Rest alk entsprechenden Alkylydenrest steht und $X_7$ Wasserstoff oder einen unter den Bedingungen für die Reduktion von $X_6$ durch Wasserstoff ersetzbaren Rest darstellt, $Ar_2$ einem Rest Ar entspricht, jedoch gegebenenfalls anstelle von einem oder zwei Hydroxy eine oder zwei Gruppen $OX_7$, in welchen $X_7$ die vorstehend angegebene Bedeutung hat, trägt, und n Null oder 1 bedeutet, wobei stets $X_6$ eine reduzierbare Gruppe Va oder Vb ist, diese Gruppe reduziert und gleichzeitig die von Wasserstoff verschiedene Gruppe $X_7$ durch Wasserstoff ersetzt, oder

d) eine Verbindung der Formel

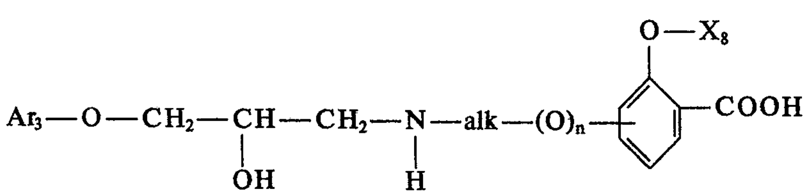
(VI)

worin $Ar_3$ die Bedeutung von Ar hat oder einen Rest Ar darstellt, der durch 1 bis 2 mittels Aminolyse in Hydroxy überführbare Gruppen substituiert ist, $X_8$ Wasserstoff oder eine mittels Aminolyse abspaltbare Gruppe bedeutet oder ein reaktionsfähiges Derivat einer der in Formel VI definierten Carbonsäuren, mit einer Verbindung der Formel

$$HNR_1R_2 \qquad \text{(VII)}$$

umsetzt und gleichzeitig gegebenenfalls vorhandene Reste $X_8$ abspaltet und durch Wasserstoff ersetzt, oder

e) in einer Verbindung der Formel

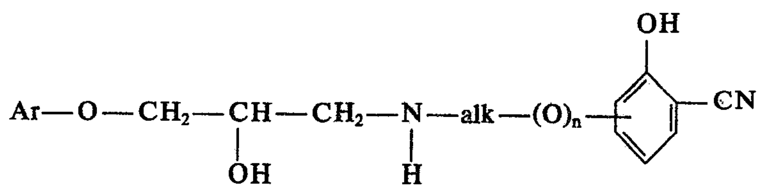

(VIII)

worin eine oder beide Hydroxygruppen und/oder im Rest Ar vorhandene Hydroxygruppen gegebenenfalls durch solche mittels Hydrolyse abspaltbaren und durch Wasserstoff ersetzbaren Gruppen geschützt sind, die unter den Bedingungen des Verfahrens abgespalten und durch Wasserstoff ersetzt werden, die Gruppe $-CN$ mittels Hydrolyse in die Gruppe $-CONH_2$ und gleichzeitig gegebenenfalls geschützte Hydroxygruppen in freie Hydroxygruppen umwandelt und, wenn erwünscht, eine so erhältliche Verbindung in eine andere Verbindung der Formel I umwandelt und/oder, wenn erwünscht, eine erhaltene freie Verbindung in ein Salz oder ein erhaltenes Salz in eine freie Verbindung überführt und/oder, wenn erwünscht, ein erhaltenes Isomerengemisch in die Isomeren oder ein erhaltenes Racemat in die Antipoden auftrennt.

34. Die nach dem Verfahren des Anspruchs 33 erhältlichen Verbindungen.

**Patentansprüche für den Vertragsstaat: AT**

1. Verfahren zur Herstellung von Derivaten des 3-Amino-1,2-propandiols der Formel

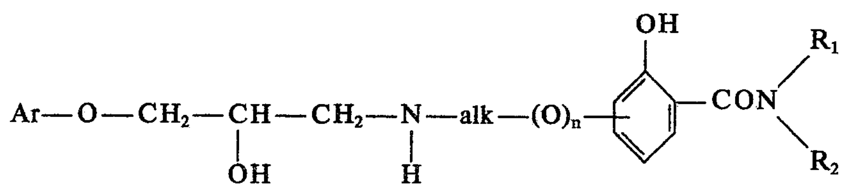

(I)

in welcher Ar gegebenenfalls substituiertes Aryl, n die Zahl 0 oder 1 und alk Alkylen mit 2—5 Kohlenstoffatomen bedeuten, wobei das Stickstoffatom und das Sauerstoffatom oder, falls n für Null steht, der Phenylrest durch mindestens zwei Kohlenstoffatome voneinander getrennt sind und $R_1$ und $R_2$ unabhängig voneinander je Wasserstoff oder Niederalkyl mit bis zu 7 Kohlenstoffatomen oder zusammen Niederalkylen, Oxaniederalkylen, Thianiederalkylen oder Azaniederalkylen mit jeweils bis zu 7 Kohlenstoffatomen oder N-Niederalkyl-azaniederalkylen mit jeweils bis zu 7 Kohlenstoffatomen im Niederalkyl- bzw. Azaniederalkylenteil darstellen, dadurch gekennzeichnet, daß man

a) eine Verbindung der Formel

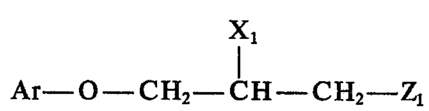

(II)

mit einer Verbindung der Formel

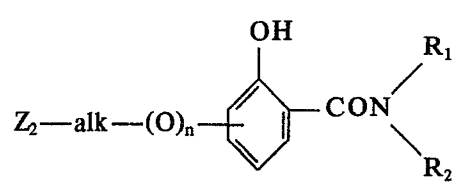

(III)

worin eine der Gruppen $Z_1$ und $Z_2$ eine reaktionsfähige veresterte Hydroxygruppe darstellt und die andere für die primäre Aminogruppe steht und $X_1$ Hydroxy bedeutet oder worin $X_1$ und $Z_1$ zusammen die Epoxygruppe bedeuten und $Z_2$ für die primäre Aminogruppe steht und Ar, n, alk, $R_1$ und $R_2$ obige Bedeutung haben, umsetzt, oder

b) in einer Verbindung der Formel

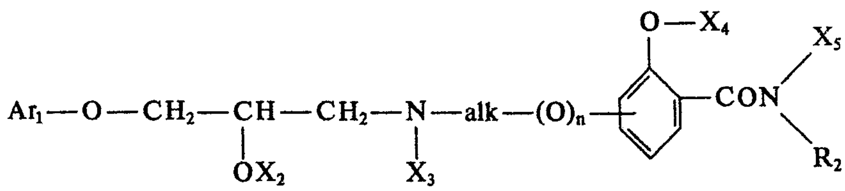

(IV)

worin $Ar_1$ die Bedeutung von Ar hat oder einen Rest Ar bedeutet, der durch 1 bis 2 in Hydroxy überführbare Gruppen substituiert ist, $X_2$, $X_3$ und $X_4$ jeweils Wasserstoff oder einen durch Wasserstoff ersetzbaren Substituenten bedeuten und $X_5$ für $R_1$ steht oder $X_2$ und $X_3$ und/oder $X_4$ und $X_5$ zusammen einen zweiwertigen, durch zwei Wasserstoffatome ersetzbaren Rest bedeuten, mit der Maßgabe, daß mindestens einer der Reste $X_2$, $X_3$ und $X_4$ von Wasserstoff verschieden ist oder mindestens $Ar_1$ einen Rest Ar bedeutet, der durch 1 bis 2 in Hydroxy überführbare Gruppen substituiert ist, oder mindestens $X_2$ und $X_3$ zusammen oder $X_4$ und $X_5$ zusammen einen zweiwertigen, durch zwei Wasserstoffatome ersetzbaren Rest darstellen oder in einem Salz davon die von Wasserstoff verschiedenen der Gruppe $X_2$, $X_3$ und $X_4$ bzw. $X_2$ uns $X_3$ zusammen oder $X_4$ und $X_5$ zusammen durch Wasserstoffatome ersetzt und/oder in einem Rest $Ar_1$ vorhandenes substituiertes Hydroxy in freies Hydroxy überführt, oder

c) in einer Verbindung der Formel

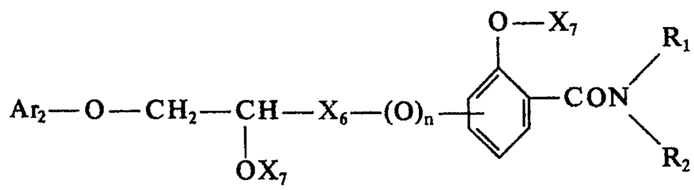

(V)

worin $X_6$ eine reduzierbare Gruppe der Formeln

$$-CH=N-alk- \qquad (Va)$$

bzw.

$$-CH_2-N=alk_1- \qquad (Vb)$$

ist, wobei $alk_1$ für einen dem Rest alk entsprechenden Alkylylidenrest steht und $X_7$ Wasserstoff oder einen unter den Bedingungen für die Reduktion von $X_6$ durch Wasserstoff ersetzbaren Rest darstellt, $Ar_2$ einem Rest Ar entspricht, jedoch gegebenenfalls anstelle von einem oder zwei Hydroxy eine oder zwei Gruppen $OX_7$, in welchen $X_7$ die vorstehend angegebene Bedeutung hat, trägt und n Null oder 1 bedeutet, wobei stets $X_6$ eine reduzierbare Gruppe Va oder Vb ist, diese Gruppe reduziert und gleichzeitig die von Wasserstoff verschiedene Gruppe $X_7$ durch Wasserstoff ersetzt, oder

d) eine Verbindung der Formel

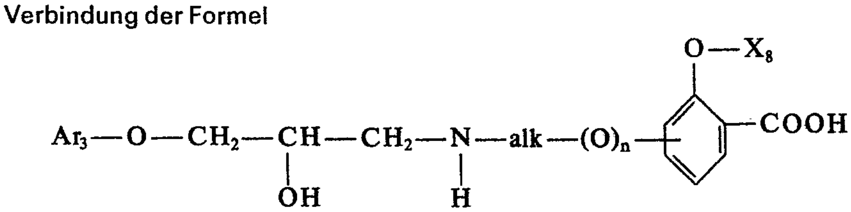

(VI)

worin $Ar_3$ die Bedeutung von Ar hat oder einen Rest Ar darstellt, der durch 1 bis 2 mittels Aminolyse in Hydroxy überführbare Gruppen substituiert ist, $X_8$ Wasserstoff oder eine mittels Aminolyse abspaltbare Gruppe bedeutet oder ein reaktonsfähiges Derivat einer der in Formel VI definierten Carbonsäuren, mit einer Verbindung der Formel

$$HNR_1R_2 \qquad (VII)$$

umsetzt und gleichzeitig gegebenenfalls vorhandene Reste $X_8$ abspaltet und durch Wasserstoff ersetzt, oder

e) in einer Verbindung der Formel

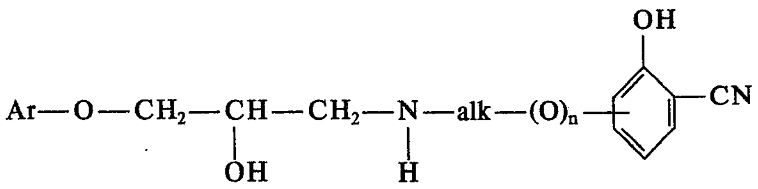

(VIII)

worin eine oder beide Hydroxygruppen und/oder im Rest Ar vorhandene Hydroxygruppen gegebenenfalls durch solche mittels Hydrolyse abspaltbaren und durch Wasserstoff ersetzbaren Gruppen geschützt sind, die unter den Bedingungen des Verfahrens abgespalten und durch Wasserstoff ersetzt werden, die Gruppe $-CN$ mittels Hydrolyse in die Gruppe $-CONH_2$ und gleichzeitig gegebenenfalls geschützte Hydroxygruppen in freie Hydroxygruppen umwandelt und, wenn erwünscht, eine so erhältliche Verbindung in eine andere Verbindung der Formel I umwandelt und/oder, wenn erwünscht, eine erhaltene freie Verbindung in ein Salz oder ein erhaltenes Salz in eine freie Verbindung überführt und/oder, wenn erwünscht, ein erhaltenes Isomerengemisch in die Isomeren oder ein erhaltenes Racemat in die Antipoden auftrennt.

2. Verfahren gemäß Anspruch 1 zur Herstellung von Verbindungen der Formel I, worin Ar mono- oder bicyclisches carbocyclisches Aryl oder mono- oder bicyclisches, über ein Ringkohlenstoffatom gebundenes, höchstens zwei Stickstoffatome und/oder ein Sauerstoff- oder Schwefelatom als Ringglieder enthaltendes Heteroaryl bedeutet, welche Reste unsubstituiert oder ein- oder mehrfach substituiert sein können, wobei als Substituenten gegebenenfalls substituiertes Niederalkyl, Niederalkenyl oder Niederalkoxy mit jeweils bis zu 7 Kohlenstoffatomen, weiter Niederalkenyloxy, Niederalkinyl, Niederalkinyloxy mit jeweils bis zu 7 Kohlenstoffatomen, Cyan und/oder Nitro und/oder direkt oder an vorgenanntes Niederalkyl, Niederalkenyl oder Niederalkoxy mit jeweils bis zu 7 Kohlenstoffatomen gebundenes Niederalkanoyl mit bis zu 7 Kohlenstoffatomen, verestertes oder amidiertes Carboxyl, Niederalkylsulfinyl, Niederalkylsulfonyl mit jeweils bis zu 7 Kohlenstoffatomen, Sulfamoyl oder Niederalkylsulfamoyl mit bis zu 7 Kohlenstoffatomen und/oder direkt oder an vorgenanntes Niederalkyl mit bis zu 7 Kohlenstoffatomen oder in höherer als der 1-Stellung an vorgenanntes Niederalkoxy mit bis zu 7 Kohlenstoffatomen gebundenes Halogen, gegebenenfalls veräthertes oder verestertes Hydroxy, wie Hydroxy, oder als nicht direkt gebundener Substituent wiederum Niederalkoxy mit bis zu 7 Kohlenstoffatomen, ferner Phenylniederalkoxy mit bis zu 7 Kohlenstoffatomen im Niederalkoxyteil oder Niederalkenyloxy mit bis zu 7 Kohlenstoffatomen, veräthertes Mercapto, gegebenenfalls substituiertes Amino, Pyrrol-1-yl, Acylamino, gegebenenfalls durch Niederalkyl oder Hydroxyniederalkyl mit jeweils bis zu 7 Kohlenstoffatomen, oder Cycloalkyl mit 5 bis 7 Ringgliedern substituiertes Ureido oder Niederalkylsulfonylamino mit bis zu 7 Kohlenstoffatomen vorliegen können und in bicyclischen Resten Ar der nicht direkt mit der Äthergruppe verbundene Ring auch mindestens partiell hydriert und in diesem Fall auch durch Oxo substituiert sein kann, und n für die Zahl 0 oder 1 steht, und alk einen Alkylenrest mit 2 bis 4 Kohlenstoffatomen bedeutet, wobei das Stickstoffatom und das Sauerstoffatom, falls n für 0 steht, der Phenylrest durch 2 bis 3 Kohlenstoffatome voneinander getrennt sind, und $R_1$ und $R_2$ die im Anspruch 1 angegebene Bedeutung haben, dadurch gekennzeichnet, daß man entsprechende Ausgangsstoffe verwendet.

3. Verfahren gemäß Anspruch 1 zur Herstellung von Verbindungen der Formel I, worin Ar mono- oder bicyclisches carbocyclisches Aryl oder mono- oder bicyclisches, über ein Ringkohlenstoffatom gebundens, höchstens zwei Stickstoffatome und/oder ein Sauerstoff- oder Schwefelatom als Ringglieder enthaltendes Heteroaryl bedeutet, welche Reste unsubstituiert oder ein- bis dreifach substituiert sein können, wobei als Substituenten gegebenenfalls in untenstehend angegebener Weise substituiertes Niederalkyl, Niederalkenyl oder Niederalkoxy mit jeweils bis zu 7 Kohlenstoffatomen, weiter Niederalkenyloxy, Niederalkinyl, Niederalkinyloxy mit jeweils bis zu 7 Kohlenstoffatomen, Cyan und/oder Nitro und/oder direkt oder an vorgenanntes Niederalkyl, Niederalkenyl oder Niederalkoxy mit jeweils bis zu 7 Kohlenstoffatomen gebundenes Niederalkanoyl mit bis zu 7 Kohlenstoffatomen, Niederalkoxycarbonyl mit bis zu 7 Kohlenstoffatomen im Niederalkoxyteil, Carbamoyl, Niederalkylcarbamoyl oder (Hydroxyniederalkyl)-carbamoyl mit jeweils bis zu 7 Kohlenstoffatomen im Niederalkyl- bzw. (Hydroxyniederalkyl)-teil, Niederalkylsulfinyl oder Niederalkylsulfonyl mit jeweils bis zu 7 Kohlenstoffatomen, Sulfamoyl oder Niederalkylsulfamoyl mit bis zu 7 Kohlenstoffatomen und/oder direkt oder an vorgenanntes Niederalkyl mit bis zu 7 Kohlenstoffatomen oder in höherer als der 1-Stellung an vorgenanntes Niederalkoxy mit bis zu 7 Kohlenstoffatomen gebundenes Halogen, Hydroxy oder, als nicht direkt gebundener Substituent, wiederum Niederalkoxy mit bis zu 7 Kohlenstoffatomen, Phenylniederalkoxy mit bis zu 7 Kohlenstoffatomen im Niederalkoxyteil, Niederalkanoyloxy oder Niederalkylthio mit jeweils bis zu 7 Kohlenstoffatomen, Amino, Niederalkylamino mit bis zu 7 Kohlenstoffatomen, Diniederalkylamino mit jeweils bis zu 7 Kohlenstoffatomen in den Niederalkylteilen, Alkylenamino oder Oxaalkylenamino, Pyrrol-1-yl, Niederalkanoylamino mit bis zu 7 Kohlenstoffatomen, oder Niederalkoxycarbonylamino mit bis zu 7 Kohlenstoffatomen im Niederalkoxyteil, gegebenenfalls durch Niederalkyl oder Hydroxyniederalkyl mit jeweils bis zu 7 Kohlenstoffatomen, oder Cycloalkyl mit 5 bis 7 Ringgliedern substituiertes Ureido, oder Niederalkylsulfonylamino mit bis zu 7 Kohlenstoffatomen vorliegen können, und in bicyclischen Resten Ar der nicht direkt mit der Äthergruppe verbundene Ring auch mindestens partiell hydriert und in diesem Fall auch durch Oxo substituiert sein kann, und n die Zahl 0 oder 1, und alk einen Alkylenrest mit 2 bis 4 Kohlenstoffatomen bedeutet, wobei das Stickstoffatom und das Sauerstoffatom, oder, falls n für 0 steht, der Phenylrest durch 2 bis 3 Kohlenstoffatomen voneinander getrennt sind, und $R_1$ und $R_2$ die im Anspruch 1 angegebene Bedeutung haben, dadurch gekennzeichnet, daß man entsprechende Ausgangsstoffe verwendet.

4. Verfahren gemäß Anspruch 1 zur Herstellung von Verbindungen der Formel I, worin Ar Phenyl, Naphthyl oder 1,2,3,4-Tetrahydro-5-naphthyl bedeutet, welche Reste unsubstituiert oder ein- bis dreifach substituiert sein können, wobei als Substituenten gegebenenfalls in untenstehend angegebener Weise substituiertes Niederalkyl, Niederalkenyl oder Niederalkoxy mit jeweils bis zu 7 Kohlenstoffatomen, weiter Niederalkenyloxy, Niederalkinyloxy mit jeweils bis zu 7 Kohlenstoffatomen und/oder Cyan und/oder direkt oder an vorgenanntes Niederalkyl Niederalkenyl oder Niederalkoxy mit jeweils bis zu 7 Kohlenstoffatomen gebundenes Niederalkanoyl mit bis zu 7 Kohlenstoffatomen, Niederalkoxycarbonyl mit bis zu 7 Kohlenstoffatomen im Niederalkoxyteil, Carbamoyl, Niederalkylcarbamoyl oder (Hydroxyniederalkyl)-carbamoyl mit jeweils bis zu 7 Kohlenstoffatomen im Niederalkyl- bzw. (Hydroxyniederalkyl)-teil, Niederalkylsulfinyl oder Niederalkylsulfonyl mit jeweils bis zu 7 Kohlenstoffatomen, Sulfamoyl oder Niederalkylsulfamoyl mit bis zu 7 Kohlenstoffatomen und/oder direkt oder an vorgenanntes Niederalkyl mit bis zu 7 Kohlenstoffatomen oder n höherer als der 1-Stellung an vorgenanntes Niederalkoxy mit bis zu 7 Kohlenstoffatomen gebundenes Halogen, Hydroxy oder, als nicht direkt gebundener Substituent, wiederum Niederalkoxy mit bis zu 7 Kohlenstoffatomen, Phenylniederalkoxy mit bis zu 7 Kohlenstoffatomen im Niederalkoxyteil, Niederalkanoyloxy oder Niederalkylthio mit jeweils bis zu 7 Kohlenstoffatomen, Amino, Niederalkylamino mit bis zu 7 Kohlenstoffatomen, Diniederalkylamino mit jeweils bis zu 7 Kohlenstoffatomen in den Niederalkylteilen, Alkylenamino oder Oxaalkylenamino, Pyrrol-1-yl, Niederalkanoylamino mit bis zu 7 Kohlenstoffatomen, Niederalkoxycarbonylamino mit bis zu 7 Kohlenstoffatomen im Niederalkoxyteil, gegebenenfalls durch Niederalkyl mit bis zu 7 Kohlenstoffatomen oder Cycloalkyl mit 5 bis 7 Ringgliedern substituiertes Ureido oder Niederalkylsulfonylamino mit bis zu 7 Kohlenstoffatomen vorliegen können, und n die Zahl 0 oder 1 und alk einen Alkylenrest mit 2 bis 4 Kohlenstoffatomen bedeutet, wobei das Stickstoffatom und das Sauerstoffatom oder, falls n für 0 steht, der Phenylrest durch 2 bis 3 Kohlenstoffatome voneinander getrennt sind und $R_1$ und $R_2$ unabhängig voneinander je Wasserstoff oder Niederalkyl mit bis zu 7 Kohlenstoffatomen darstellen oder zusammen mit dem Stickstoffatom der Amidgruppe Morpholino bilden, dadurch gekennzeichnet, daß man entsprechende Ausgangsstoffe verwendet.

5. Verfahren gemäß Anspruch 1 zur Herstellung von Verbindungen der Formel I, worin Ar Phenyl, welches unsubstituiert oder ein- bis dreifach substituiert sein kann, wobei als Substituenten gegebenenfalls in untenstehend angegebener Weise substituiertes Niederalkyl oder Niederalkoxy mit jeweils bis zu 7 Kohlenstoffatomen weiter Niederalkenyl, Niederalkinyloxy mit jeweils bis zu 7 Kohlenstoffatomen, und/oder Cyan und/oder direkt oder an vorgenanntes Niederalkyl oder Niederalkoxy mit jeweils bis zu 7 Kohlenstoffatomen gebundenes Niederalkanoyl mit bis zu 7 Kohlenstoffatomen, Niederalkoxycarbonyl mit bis zu 7 Kohlenstoffatomen im Niederalkoxyteil, Carbamoyl, Niederalkylcarbamoyl oder (Hydroxyniederalkyl)-carbamoyl mit jeweils bis zu 7 Kohlenstoffatomen im Niederalkyl- bzw. (Hydroxyniederalkyl)-teil, Niederalkylsulfinyl oder Niederalkylsulfonyl mit jeweils bis zu 7 Kohlenstoffatomen, und/oder direkt oder an vorgenanntes Niederalkyl mit bis zu 7 Kohlenstoffatomen oder in höherer als der 1-Stellung an vorgenanntes Niederalkoxy mit bis zu 7 Kohlenstoffatomen gebundenes Halogen, Hydroxy oder, als nicht direkt gebundener Substituent, wiederum Niederalkoxy mit bis zu 7 Kohlenstoffatomen, ferner Phenylniederalkoxy mit bis zu 7 Kohlenstoffatomen im Niederalkoxyteil, Niederalkylthio mit bis zu 7 Kohlenstoffatomen, Alkylen- oder Oxaalkylenamino, Pyrrol-1-yl, Niederalkanoylamino mit bis zu 7 Kohlenstoffatomen, Niederalkoxycarbonylamino mit bis zu 7 Kohlenstoffatomen im Niederalkoxyteil oder gegebenenfalls durch Niederalkyl mit bis zu 7 Kohlenstoffatomen substituiertes Ureido vorliegen können, und n die Zahl 0 oder 1 und alk einen Alkylenrest mit 2 bis 4 Kohlenstoffatomen bedeutet, wobei das Stickstoffatom und das Sauerstoffatom oder, falls n für 0 steht, der Phenylrest durch 2 bis 3 Kohlenstoffatome voneinander getrennt sind, und $R_1$ und $R_2$ unabhängig je Wasserstoff oder Niederalkyl mit bis zu 7 Kohlenstoffatomen darstellen, dadurch gekennzeichnet, daß man entsprechende Ausgangsstoffe verwendet.

6. Verfahren gemäß Anspruch 1 zur Herstellung von Verbindungen der Formel I, worin Ar Phenyl, welches unsubstituiert oder ein- bis dreifach substituiert sein kann, wobei als Substituenten gegebenenfalls in untenstehend angegebener Weise substituiertes Niederalkyl oder Niederalkoxy mit jeweils bis zu 7 Kohlenstoffatomen, weiter Niederalkenyl, Niederalkenyloxy oder Niederalkinyloxy mit jeweils bis zu 7 Kohlenstoffatomen und/oder Cyan und/oder direkt oder an vorgenanntes Niederalkyl oder Niederalkoxy mit jeweils bis zu 7 Kohlenstoffatomen gebundenes Niederalkanoyl mit bis zu 7 Kohlenstoffatomen, Carbamoyl, Niederalkylcarbamoyl oder (Hydroxyniederalkyl)-carbamoyl mit jeweils bis

zu 7 Kohlenstoffatomen im Niederalkyl- bzw. (Hydroxyniederalkyl)-teil, Niederalkylsulfinyl oder Niederalkylsulfonyl mit jeweils bis zu 7 Kohlenstoffatomen, und/oder direkt oder an vorgenanntes Niederalkyl mit bis zu 7 Kohlenstoffatomen oder in höherer als der 1-Stellung an vorgenanntes Niederalkoxy mit bis zu 7 Kohlenstoffatomen gebundenes Fluor oder Chlor, Hydroxy oder, als nicht direkt gebundener Substituent, wiederum Niederalkoxy mit bis zu 7 Kohlenstoffatomen, ferner Phenylniederalkoxy mit bis zu 7 Kohlenstoffatomen im Niederalkoxyteil, Niederalkylthio mit bis zu 7 Kohlenstoffatomen, Pyrrol-1-yl, Niederalkanoylamino mit bis zu 7 Kohlenstoffatomen, Niederalkoxycarbonylamino mit bis zu 7 Kohlenstoffatomen im Niederalkoxyteil oder Ureido vorliegen können und n die Zahl 1 und alk einen Alkylenrest mit 2 bis 4 Kohlenstoffatomen bedeutet, wobei das Stickstoffatom und das Sauerstoffatom durch 2 Kohlenstoffatome voneinander getrennt sind, und $R_1$ und $R_2$ Wasserstoff darstellen, dadurch gekennzeichnet, daß man entsprechende Ausgangsstoffe verwendet.

7. Verfahren gemäß Anspruch 1 zur Herstellung von Verbindungen der Formel I, worin der die Amid- und die Hydroxygruppe tragende Phenylrest vorzugsweise in seiner 4-Stellung und vor allem in seiner 5-Stellung mit dem restlichen Molekül verbunden ist, dadurch gekennzeichnet, daß man entsprechende Ausgangsstoffe verwendet.

8 . Verfahren zur Herstellung von 1-[2-(3-Carbamoyl-4-hydroxyphenoxy)-äthylamino]-3-[4-(2-methoxy-äthoxy)-phenoxy]-2-propanol nach Anspruch 1, dadurch gekennzeichnet, daß man 1-{[2-(Carbamoyl-4-hydroxy-phenoxy)-äthyl]-benzylamino}-3-[4-(2-methoxyäthoxy)-phenoxy]-2-propanol als Ausgangsstoff verwendet.

9. Verfahren zur Herstellung von 1-[2-(3-Carbamoyl-4-hydroxy-phenoxy)-äthylamino]-3-[4-(2-methoxyäthoxy)-phenoxy]-2-propanol nach Anspruch 1, dadurch gekennzeichnet, daß man 1-[4-(2-methoxyäthoxy)-phenoxy]-3-amino-2-propanol und 5-(2-Bromäthoxy)-salicylamid als Ausgangsstoffe verwendet.

10. Verfahren zur Herstellung von 1-[2-(3-Carbamoyl-4-hydroxy-phenoxy)-äthylamino]-3-[4-(2-methoxyäthoxy)-phenoxy]-2-propanol nach Anspruch 1, dadurch gekennnzeichnet, daß man (2,3-Dihydro-2,2-dimethyl-4H-1,3-benzoxazin-4-on-6-yloxy)-acetaldehyd und 1-Amino-3-[4-(2-methoxyäthoxy)-phenoxy]-2-propanol als Ausgangsstoffe verwendet.

11. Verfahren zur Herstellung von 1-[2-(3-Carbamoyl-4-hydroxy-phenoxy)-äthylamino]-3-[4-(2-methoxy-äthoxy)-phenoxy]-2-propanol nach Anspruch 1, dadurch gekennzeichnet, daß man 1-[2-(3-Cyano-4-hydroxy-phenoxy)-äthylamino]-3-[4-(2-methoxy-äthoxy)-phenoxy]-2-propanol als Ausgangsstoff verwendet.

12. Verfahren zur Herstellung von 1-(2-Allyloxyphenoxy)-3-[2-(3-carbamoyl-4-hydroxy-phenoxy)-äthylamino]-2-propanol nach Anspruch 1, dadurch gekennzeichnet, daß man 1-(2-Allyloxy-phenoxy)-3-amino-2-propanol und 6-(2-Bromäthoxy)-2,3-dihydro-2,2-dimethyl-4H-1,3-benzoxazin-4-on als Ausgangsstoffe verwendet.

13. Verfahren zur Herstellung von 1-(2-Allyloxy-phenoxy)-3-[2-(3-carbamoyl-4-hydroxy-phenoxy)-äthylamino]-2-propanol nach Anspruch 1, dadurch gekennzeichnet, daß man 1-(2-Allyloxy-phenoxy)-3-amino-2-propanol und 5-(2-Bromäthoxy)-salicylamid als Ausgangsstoffe verwendet.

14. Verfahren zur Herstellung von 1-(2-Allyloxy-phenoxy)-3-[2-(3-carbamoyl-4-hydroxy-phenoxy)-äthylamino]-2-propanol nach Anspruch 1, dadurch gekennzeichnet, daß man [2-(2,3-Epoxy-propoxy)-phenyl]-2-allyläther und 5-(2-Amino-äthoxy)-salicylamid als Ausgangsstoffe verwendet.

15. Verfahren zur Herstellung von 1-[2-(3-Carbamoyl-4-hydroxy-phenoxy)-äthylamino]-3-(4-hydroxy-phenoxy)-2-propanol nach Anspruch 1, dadurch gekennzeichnet, daß man 1-{N-[2-(3-Carbamoyl-4-hydroxy-phenoxy)-äthyl]-benzylamino}-3-(4-benzyloxy-phenoxy)-2-propanol als Ausgangsstoff verwendet.

16. Verfahren zur Herstellung von 4-{2-Hydroxy-3-[(3-carbamoyl-4-hydroxy-phenoxy)-äthylamino]-propoxy}-phenyl-acetamid nach Anspruch 1, dadurch gekennzeichnet, daß man 1-{N-[2-(3-Carbamoyl-4-hydroxy-phenoxy)-äthyl]-benzylamino}-3-(4-carbamoylmethyl-phenoxy)-2-propanol als Ausgangsstoff verwendet.

17. Verfahren zur Herstellung von 1-[2-(3-Carbamoyl-4-hydroxy-phenoxy)-äthylamino]-3-(4-carbamoylmethyl-phenoxy)-2-propanol nach Anspruch 1, dadurch gekennzeichnet, daß man (4-Carbamoylmethyl-phenoxy)-2,3-epoxypropan und 5-(2-Aminoäthoxy)-salicylamid als Ausgangsstoffe verwendet.

18. Verfahren zur Herstellung von 1-[2-(3-Carbamoyl-4-hydroxy-phenoxy)-äthylamino]-3-[2-(pyrrol-1-yl)-phenoxy]-2-propanol nach Anspruch 1, dadurch gekennzeichnet, daß man 1-{N-[2-(3-Carbamoyl-4-hydroxy-phenoxy)-äthyl]-benzylamino}-3-[2-(pyrrol-1-yl)-phenoxy]-2-propanol als Ausgangsstoff verwendet.

19. Verfahren zur Herstellung von 1-[2-(3-Carbamoyl-4-hydroxy-phenoxy)-äthylamino]-3-{4-[2-(methoxycarbonylamino)-äthoxy]-phenoxy}-2-propanol nach Anspruch 1, dadurch gekennzeichnet, daß man 1-{N-[2-(3-Carbamoyl-4-hydroxy-phenoxy)-äthyl]-benzylamino}-3-[4-(2-methoxycarbonylamino-äthoxy)-phenoxy]-2-propanol als Ausgangsstoff verwendet.

20. Verfahren zur Herstellung von 1-[2-(3-Carbamoyl-4-hydroxy-phenoxy)-äthylamino]-3-(2-methyl-indol-4-yloxy)-2-propanol nach Anspruch 1, dadurch gekennzeichnet, daß man 1-{N-[2-(3-Carbamoyl-4-hydroxy-phenoxy)-äthyl]-benzylamino}-3-(2-methyl-indol-4-yloxy)-2-propanol als Ausgangsstoff verwendet.

21. Verfahren zur Herstellung von 4-{3-[2-(3-Carbamoyl-4-hydroxy-phenoxy)-äthylamino]-2-hydroxy-

propoxy]-benzimidazol-2-on nach Anspruch 1, dadurch gekennzeichnet, daß man N-[2-(4-Hydroxy-3-carbamoyl-phenoxy)-äthyl]-N-[3-(2-oxo-benzimidazol-4-yloxy)-2-hydroxypropyl]-N-benzylamin als Ausgangsstoff verwendet.

22. Verfahren nach einem der Ansprüche 1—21, dadurch gekennzeichnet, daß man eine erhaltene Verbindung der Formel I in ein pharmazeutisch verwendbares, nicht-toxisches Säureadditionssalz überführt.

**Claims for the Contracting States BE, CH, DE, FR, GB, IT, LU, SE, NL**

1. A derivative of 3-amino-1,2-propanediol of the formula

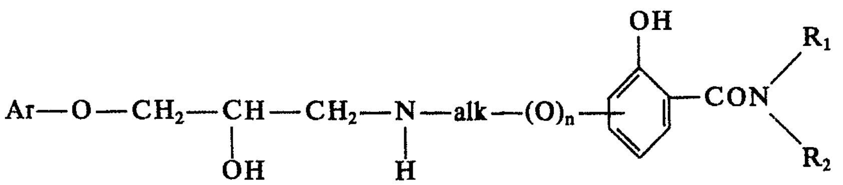

(I)

wherein Ar is unsubstituted or substituted aryl, n is 0 or 1, and alk is $C_2—C_5$alkylene, and the nitrogen atom and the oxygen atom or, if n is 0, the phenyl radical, are separated from each other by at least 2 carbon atoms, and each of $R_1$ and $R_2$ independently is hydrogen or lower alkyl of 1 to 7 carbon atoms, or together are lower alkylene, oxa-lower alkylene, thia-lower alkylene or aza-lower alkylene, each of 1 to 7 carbon atoms, or N-lower alkyl-aza-lower alkylene containing 1 to 7 carbon atoms in the lower alkyl and aza-lower alkylene moiety, resprectively.

2. A compound of the formula I according to claim 1, wherein Ar is mono- or bicyclic carbocyclic aryl or mono- or bicyclic heteroaryl which is bonded through a ring carbon atom and contains not more than two nitrogen atoms and/or an oxygen or sulfur atom as ring members, which radicals may be substituted or substituted, possible substituents being unsubstituted or substituted lower alkyl, lower alkenyl or lower alkoxy, each of 1 to 7 carbon atoms, and lower alkenyloxy, lower alkynyl, lower alkynyloxy, each of 1 to 7 carbon atoms, cyano and/or nitro, and/or as substituents bonded direct or bonded to the above mentioned lower alkyl, lower alkenyl or lower alkoxy, each of 1 to 7 carbon atoms, one or more of lower alkanoyl, or esterified or amidated carboxyl, lower alkylsulfinyl, lower alkynsulfonyl, each of 1 to 7 carbon atoms, sulfamoyl or lower alkylsulfamoyl of 1 to 7 carbon atoms, and/or halogen bonded direct or to above mentioned lower alkyl of 1 to 7 carbon atoms or, in a position higher than in 1-position, to above mentioned lower alkoxy of 1 to 7 carbon atoms, free or etherified or esterified hydroxyl such as hydroxyl, or, as substituent which is not bonded direct, in turn lower alkoxy of 1 to 7 carbon atoms, and phenyl-lower alkoxy containing 1 to 7 carbon atoms in the lower alkoxy moiety, or lower alkenyloxy of 1 to 7 carbon atoms, etherified mercapto, unsubstituted or substituted amino, pyrrol-1-yl, acylamino, ureido which is unsubstituted or substituted by lower alkyl or hydroxy-lower alkyl, each of 1 to 7 carbon atoms, or by cycloalkyl containing 5 to 7 ring members, or lower alkylsulfonylamino of 1 to 7 carbon atoms, and, in bicyclic radicals Ar the ring which is not attached direct to the ether group may also be at least partially hydrogenated and, in this case, may also be substituted by oxo, and n is 0 or 1 and alk is a $C_2—C_4$alkylene radical, and the nitrogen atom and the oxygen atom or, if n is 0, the phenyl radical, are separated from each other by 2 to 3 carbon atoms, and $R_1$ and $R_2$ are as defined in claim 1.

3. A compound of the formula I according to claim 1, wherein Ar is monocyclic or bicyclic carbocyclic aryl or monocyclic or bicyclic heteroaryl bonded through a ring carbon atom and containing as ring members not more than two nitrogen atoms and/or one oxygen or sulfur atom, which radicals may be unsubstituted or mono- to trisubstituted, suitable substituents being unsubstituted or substituted in the manner specified hereinafter and selected from lower alkyl, lower alkenyl or lower alkoxy, each of 1 to 7 carbon atoms, cyano and/or nitro and/or, as substituents bonded direct to or to the above mentioned lower alkyl, lower alkenyl or lower alkoxy, each of 1 to 7 carbon atoms, one or more of lower alkanoyl, lower alkoxycarbonyl of 1 to 7 carbon atoms, carbamoyl, lower alkylcarbamoyl or (hydroxy)-lower alkyl carbamoyl, each containing 1 to 7 carbon atoms in the lower alkyl and lower hydroxyalkyl moiety respectively, lower alkylsulfinyl or lower alkylsulfonyl, each of 1 to 7 carbon atoms, sulfamoyl or lower alkylsulfamoyl, each of 1 to 7 carbon atoms; and/or halogen bonded direct or halogen bonded to the above mentioned lower alkyl of 1 to 7 carbon atoms or, in a position higher than the 1-position, halogen bonded to the above mentioned lower alkoxy of 1 to 7 carbon atoms; hydroxyl; or, as substituent not bonded direct, in turn lower alkoxy of 1 to 7 carbon atoms, hydroxyl or, as substituent not bonded direct, in turn lower alkoxy of 1 to 7 carbon atoms, phenyl-lower alkoxy containing 1 to 7 carbon atoms in the lower alkoxy moiety, lower alkanoyloxy, or lower alkylthio, each of 1 to 7 carbon atoms, amino, lower alkylamino of 1 to 7 carbon atoms, di-lower alkylamino containing 1 to 7 carbon atoms in each alkyl moiety, alkyleneamino or oxaalkyleneamino, pyrrol-1-yl, lower alkanoylamino of 1 to 7 carbon atoms, or lower alkoxycarbonylamino containing 1 to 7 carbon atoms in

the lower alkoxy moiety, ureido which is unsubstituted or substituted by lower alkyl or hydroxy-lower alkyl, each of 1 to 7 carbon atoms, or by cycloalkyl containing 5 to 7 ring members, or lower alkylsulfonylamino of 1 to 7 carbon atoms, and in bicyclic radicals Ar the ring which is not attached direct to the ether group may also be partially hydrogenated, and in this case may also be substituted by oxo, and n is 0 or 1 and alk is a $C_2$—$C_4$alkylene radical, and the nitrogen atom and the oxygen atom or, if n is 0, the phenyl radical, are separated from each other by 2 to 3 carbon atoms, and $R_1$ and $R_2$ are as defined in claim 1.

4. A compound of the formula I according to claim 1, wherein Ar is phenyl, naphthyl or 1,2,3,4-tetra-5-naphthyl, which radicals may be unsubstituted or mono- to trisubstituted, suitable substituents being unsubstituted or substituted in the manner specified hereinafter and selected from lower alkyl, lower alkenyl or lower alkoxy, each of 1 to 7 carbon atoms, and lower alkenyloxy, lower alkynyloxy, each of 1 to 7 carbon atoms, and/or cyano, and/or, as substituents bonded direct or to above mentioned lower alkyl, lower alkenyl or lower alkoxy of 1 to 7 carbon atoms, one or more of lower alkanoyl of 1 to 7 carbon atoms, lower alkoxycarbonyl containing to 1 to 7 carbon atoms in the alkoxy moiety, carbamoyl, lower alkylcarbamoyl or (hydroxy-lower alkyl)carbamoyl, each containing 1 to 7 carbon atoms in the lower alkyl or hydroxy-lower alkyl moiety respectively, lower alkylsulfinyl or lower alkylsulfonyl, each of 1 to 7 carbon atoms, sulfamoyl or lower alkylsulfamoyl of 1 to 7 carbon atoms, and/or as halogen bonded direct or to above mentioned lower alkyl of 1 to 7 carbon atoms, or to above mentioned lower alkoxy of 1 to 7 carbon atoms in a position higher than the 1-position, hydroxy or, as substituent not bonded direct, in turn lower alkoxy of 1 to 7 carbon atoms, phenyl-lower alkoxy containing 1 to 7 carbon atoms in the alkoxy moiety, lower alkanoyloxy or lower alkylthio, each of 1 to 7 carbon atoms, amino, lower alkylamino of 1 to 7 carbon atoms, di-lower alkylamino containing 1 to 7 carbon atoms in each alkyl moiety, alkyleneamino or oxaalkyleneamino, pyrrol-1-yl, lower alkylanoylamino of 1 to 7 carbon atoms, lower alkoxycarbonylamino containing 1 to 7 carbon atoms in the lower alkoxy moiety, ureido which is unsubstituted or substituted by lower alkyl of 1 to 7 carbon atoms or cycloalkyl containing 5 to 7 ring members, or lower alkylsulfonylamino of 1 to 7 carbon atoms, and n is 0 or 1 and alk is a $C_2$—$C_4$alkylene radical, and the nitrogen atom and the oxygen atom or, if n is 0, the phenyl radical, are separated from each other by 2 to 3 carbon atoms, and $R_1$ and $R_2$ are each independently hydrogen or lower alkyl of 1 to 7 carbon atoms, or together with the nitrogen atom of the amide group form morpholino.

5. A compound of the formula I according to claim 1, wherein Ar is phenyl which may be unsubstituted or mono- to trisubstituted, suitable substituents being unsubstituted or substituted in the manner specified hereinafter and selected from lower alkyl or lower alkoxy, each of 1 to 7 carbon atoms, and lower alkenyl, lower alkenyloxy or lower alkynyloxy, each of 1 to 7 carbon atoms, and/or cyano, and/or as substituents bonded direct or to the above mentioned lower alkyl or lower alkoxy, each of 1 to 7 carbon atoms, lower alkanoyl of 1 to 7 carbon atoms, lower alkoxycarbonyl containing 1 to 7 carbon atoms in the lower alkoxy moiety, carbamoyl, lower alkylcarbamoyl or (hydroxy-lower alkyl)carbamoyl, each containing 1 to 7 carbon atoms in the lower alkyl or hydroxy-lower moiety respectively, lower alkylsulfinyl or lower alkylsulfonyl, each of 1 to 7 carbon atoms, and/or halogen bonded direct or to above mentioned lower alkyl of 1 to 7 carbon atoms or to above mentioned lower alkoxy of 1 to 7 carbon atoms in a position higher than the 1-position, hydroxy or, as substituent not bonded direct, in turn lower alkoxy of 1 to 7 carbon atoms, and also phenyl-lower alkoxy containing 1 to 7 carbon atoms in the lower alkoxy moiety, lower alkylthio of 1 to 7 carbon atoms, alkyleneamino or oxaalkyleneamino, pyrrol-1-yl, lower alkanoylamino of 1 to 7 carbon atoms, lower alkoxycarbonylamino containing 1 to 7 carbon atoms in the lower alkoxy moiety, or ureido which is unsubstituted or substituted by lower alkyl of 1 to 7 carbon atoms, and n is 0 or 1 and alk is a $C_2$—$C_4$alkylene radical, and the nitrogen atom and the oxygen atom or, if n is 0, the phenyl radical, are separated from each other by 2 to 3 carbon atoms, and $R_1$ and $R_2$ are each independently hydrogen or lower alkyl of 1 to 7 carbon atoms.

6. A compound of the formula I according to claim 1, wherein Ar is phenyl which may be unsubstituted or mono- to trisubstituted, suitable substituents being unsubstituted or substituted in the manner specified hereinafter and selected from lower alkyl or lower alkoxy, each of 1 to 7 carbon atoms, and lower alkenyl, lower alkenyloxy or lower alkynyloxy, each of 1 to 7 carbon atoms, and/or cyano, and/or as substituents bonded direct or to the above mentioned lower alkyl or lower alkoxy, each of 1 to 7 carbon atoms, lower alkanoyl of 1 to 7 carbon atoms, carbamoyl, lower alkylcarbamoyl or (hydroxy-lower alkyl)carbamoyl, each containing 1 to 7 carbon atoms in the lower alkyl or hydroxy-lower moiety respectively, lower alkylsulfinyl or lower alkylsulfonyl, each of 1 to 7 carbon atoms, and/or fluorine or chlorine bonded direct or to above mentioned lower alkyl of 1 to 7 carbon atoms or to above mentioned lower alkoxy of 1 to 7 carbon atoms in a position higher than the 1-position, hydroxy or, as substituent not bonded direct, in turn lower alkoxy of 1 to 7 carbon atoms, and also phenyl-lower alkoxy containing 1 to 7 carbon atoms in the lower alkoxy moiety, lower alkylthio of 1 to 7 carbon atoms, pyrrol-1-yl, lower alkanoylamino containing 1 to 7 carbon atoms, lower alkoxycarbonylamino containing 1 to 7 carbon atoms in the lower alkoxy moiety, or ureido, and n is 1 or and alk is a $C_2$—$C_4$alkylene radical, and the nitrogen atom and the oxygen atom or, if n is 0, the phenyl radical, are separated from each other by 2 carbon atoms, and $R_1$ and $R_2$ are hydrogen.

7. A compound of the formula I according to claim 1, wherein the phenyl radical carrying the amido

and hydroxyl group is attached to the remainder of the molecule preferably in its 4-position and, most preferably, in its 5-position.

8. 1-[2-(3-Carbamoyl-4-hydroxyphenoxy)ethylamino]-3-[4-(2-methoxyethoxy)-phenoxy]-2-propanol.

9. 1-(4-Hydroxyphenoxy)-3-[2-(3-carbamoyl-4-hydroxyphenoxy)-1-methylethylamino]-2-propanol.

10. 1-(2-Allyloxyphenoxy)-3-[2-(3-carbamoyl-4-hydroxyphenoxy)-ethylamino]-2-propanol.

11. 1-[4-[2-(Acetamido)ethoxy]phenoxy]-3-[2-(3-carbamoyl-4-hydroxyphenoxy)ethylamino]-2-propanol.

12. 1-[2-(3-Carbamoyl-4-hydroxyphenoxy)ethylamino]-3-[4-(2-oxopropoxy)phenoxy]-2-propanol.

13. 1-[2-(4-Carbamoyl-3-hydroxyphenoxy)-1-methylethylamino]-3-[4-(2-methoxyethoxy)phenoxy]-2-propanol.

14. 4-[2-Hydroxy-3-[3-carbamoyl-4-hydroxyphenoxy)ethylaminopropoxy]-phenylacetamide.

15. 1-[2-(3-Carbamoyl-4-hydroxyphenoxy)ethylamino]-3-[4-[2-(methoxycarbonylamino)ethoxy]-phenoxy]-2-propanol.

16. 1-[2-(3-Carbamoyl-4-hydroxyphenoxy)ethylamino]-3-(3-methyl-4-methylsulfinylphenoxy)-2-propanol.

17. 1-[2-(3-Carbamoyl-4-hydroxyphenoxy)ethylamino]-3-(2-cyanophenoxy)-2-propanol.

18. 1-[2-(3-Carbamoyl-4-hydroxyphenoxy)ethylamino]-3-(2-propoxyphenoxy)-2-propanol.

19. 1-[2-(3-Carbamoyl-4-hydroxyphenoxy)ethylamino]-3-[2-(pyrrol-1-yl)-phenoxy]-2-propanol.

20. 1-[2-(3-Carbamoyl-4-hydroxyphenoxy)ethylamino]-3-[2-(2-propinyloxy)phenoxy]-2-propanol.

21. 1-[2-(3-Carbamoyl-4-hydroxyphenoxy)ethylamino]-3-(2-methoxyphenoxy)-2-propanol.

22. 1-[2-(3-Carbamoyl-4-hydroxyphenoxy)ethylamino]-3-(2-methylindol-4-yloxy)-2-propanol.

23. 1-(2-Acetylphenoxy)-3-[2-(3-Carbamoyl-4-hydroxyphenoxy)ethylamino]-2-propanol.

24. 5-[3-[2-(3-Carbamoyl-4-hydroxyphenoxy)ethylamino]-2-hydroxypropoxy]-1,2,3,4-tetrahydroxy-2,3-cis-naphthalenediol.

25. 1-[2-(3-Carbamoyl-4-hydroxyphenoxy)ethylamino]-3-(3-methylphenoxy)-2-propanol.

26. 1-[2-(3-Carbamoyl-4-hydroxy-phenoxy)ethylamino]-3-(4-hydroxyphenoxy)-2-propanol.

27. A compound of the formula I according to claim 1, or a salt thereof, for use in a therapeutic method of treating the human or animal body.

28. A compound of the formula I according to claim 1, or a salt thereof, which specifically acts on $\beta$-adrenergic receptors.

29. A salt of a compound of any one of claims 1 to 26.

30. A pharmaceutically acceptable non-toxic acid addition salt of a compound as claimed in any one of claims 1 to 26.

31. A pharmaceutical preparation containing a compound of any one of claims 1 to 26 and 30.

32. Use of a compound of the formula I according to claim 1, or of a pharmaceutically acceptable non-toxic acid addition salt thereof, for the preparation of a pharmaceutical preparation.

33. A process for the preparation of a derivative of 3-amino-1,2-propanediol of the formula

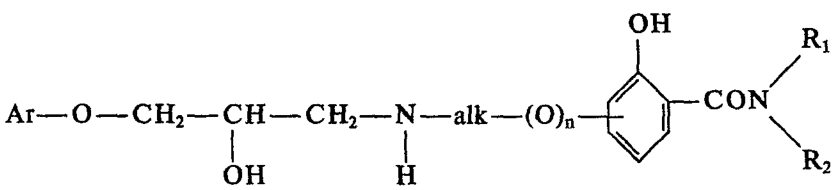

(I)

wherein Ar is unsubstituted or substituted aryl, n is 0 or 1, and alk is $C_2-C_5$alkylene, and the nitrogen atom and the oxygen atom or, if n is 0, the phenyl radical, are separated from each other by at least 2 carbon atoms, and each of $R_1$ and $R_2$ independently is hydrogen or lower alkyl of 1 to 7 carbon atoms, or together are lower alkylene, oxa-lower alkylene, thia-lower alkylene or aza-lower alkylene, each of 1 to 7 carbon atoms, or N-lower alkyl-aza-lower alkylene containing 1 to 7 carbon atoms in the lower alkyl and aza-lower alkylene moiety respectively, which process comprises

a) reacting a compound of the formula

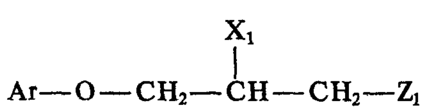

(II)

with a compound of the formula

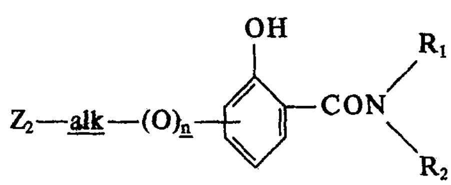

(III)

wherein one of the groups $Z_1$ and $Z_2$ is a reactive esterified hydroxyl group and the other ist the primary amino group, and $X_1$ is hydroxy, or in which $X_1$ and $Z_1$ together are the epoxy group and $Z_2$ is the primary amino group, and Ar, n, alk, $R_1$ and $R_2$ have the meanings given above or

b) in a compound of the formula

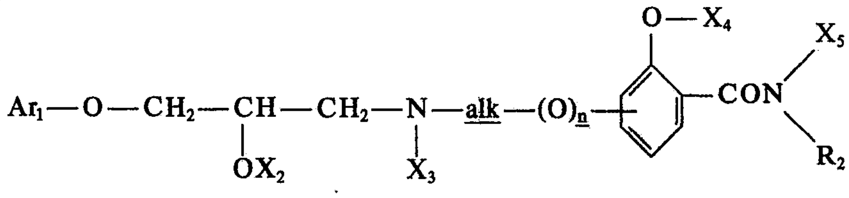

(IV)

wherein $Ar_1$ has the same meaning as Ar or is a radical Ar which is substituted by 1 or 2 groups which may be converted into hydroxy, $X_2$, $X_3$ and $X_4$ are each hydrogen or a substituent which may be replaced by hydrogen, and $X_5$ is $R_1$, or $X_2$ and $X_3$ and/or $X_4$ and $X_5$ together are a divalent radical which may be replaced by two hydrogen atoms, with the proviso that at least one of the radicals $X_2$, $X_3$ and $X_4$ is different from hydrogen, or at least $Ar_1$ is a radical Ar which is substituted by 1 or 2 groups which may be converted into hydroxy, or at least $X_2$ and $X_3$ together or $X_4$ and $X_5$ together are a divalent radical which may be replaced by two hydrogen atoms, or in a salt thereof those of the groups $X_2$, $X_3$ and $X_4$, or $X_2$ and $X_3$ together, or $X_4$ and $X_5$ together, which are different from hydrogen are replaced by hydrogen atoms, and/or substituted hydroxy present in a radical $Ar_1$ is converted into free hydroxy, or

c) in a compound of the formula

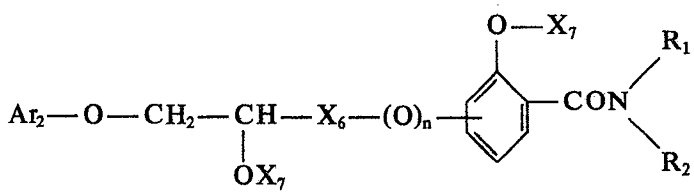

(V)

wherein $X_6$ is a reducible group of the formula

$$-CH=N-\underline{alk}- \qquad (V\underline{a})$$

or

$$-CH_2-N=\underline{alk}_1- \qquad (V\underline{b})$$

in which $\underline{alk}_1$ is an alkylylidene radical corresponding to the radical alk, and $X_7$ is hydrogen or a radical which may be replaced by hydrogen under the conditions for the reduction of $X_6$, $Ar_2$ corresponds to a radical Ar, but optionally, instead of carrying one or two hydroxy groups carries one or two $OX_7$ groups in which $X_7$ has the meaning given above, and n is 0 or 1, wherein $X_6$ is always a reducible group Va or Vb, the $X_6$ group is reduced, and simultaneously the $X_7$ group different from hydrogen is replaced by hydrogen, or

d) reacting a compound of the formula

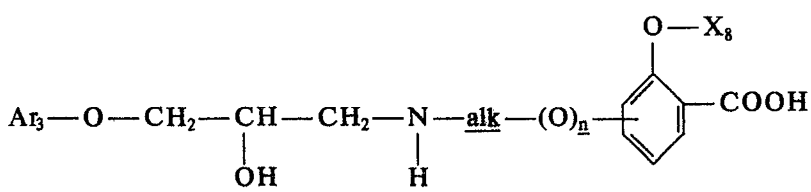

(VI)

wherein $Ar_3$ has the same meaning as Ar, or is a radical Ar which is substituted by 1 or 2 groups which may be converted into hydroxy by aminolysis, $X_8$ is hydrogen or a group which can be split off by aminolysis, or a reactive derivative of one of the carboxylic acids defined in formula VI, with a compound of the formula

$$HNR_1R_2 \qquad (VII)$$

and at the same time splitting off optionally present $X_8$ radicals and replacing them by hydrogen, or

e) in a compound of the formula

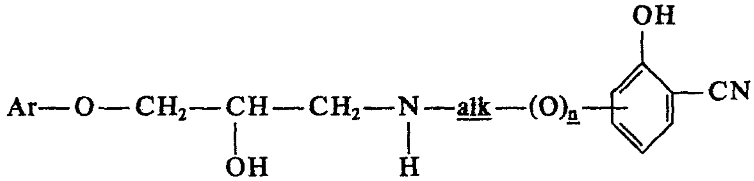

(VIII)

wherein one or both hydroxy groups and/or hydroxy groups optionally present in the radical Ar are protected by those groups that can be split off by hydrolysis and replaced by hydrogen which groups are split off under the conditions of the process and are replaced by hydrogen, the $-CN$ group is converted by hydrolysis into the $-CONH_2$ group and, at the same time, optionally protected hydroxy groups are converted into free hydroxy groups, and, if desired, converting a resultant free compound into a salt or a resultant salt into a free compound, and/or, if desired, separating a mixture of isomers into the individual isomers or resolving a racemate into antipodes.

34. A compound obtainable according to claim 33.

**Claims for the Contracting State: AT**

1. A process for the preparation of a derivative of 3-amino-1,2-propanediol of the formula

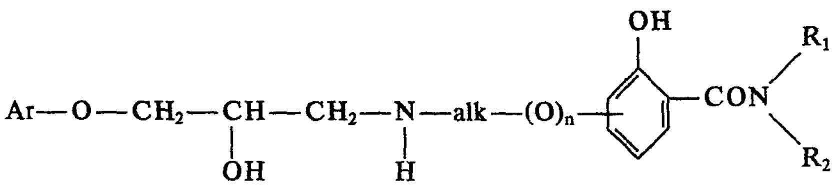

(I)

wherein Ar is unsubstituted or substituted aryl, n is 0 or 1, and alk is $C_2-C_5$alkylene, and the nitrogen atom and the oxygen atom or, if n is 0, the phenyl radical, are separated from each other by at least 2 carbon atoms, and each of $R_1$ and $R_2$ independently is hydrogen or lower alkyl of 1 to 7 carbon atoms, or together are lower alkylene, oxa-lower alkylene, thia-lower alkylene or aza-lower alkylene, each of 1 to 7 carbon atoms, or N-lower alkyl-aza-lower alkylene containing 1 to 7 carbon atoms in the lower alkyl and aza-lower alkylene moiety respectively, which process comprises

a) reacting a compound of the formula

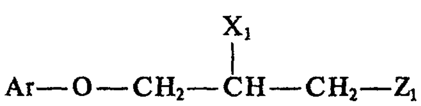

(II)

with a compound of the formula

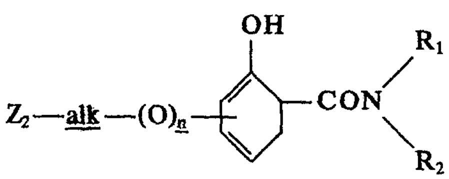

(III)

wherein one of the groups $Z_1$ and $Z_2$ is a reactive esterified hydroxyl group and the other is the primary amino group, and $X_1$ is hydroxy, or in which $X_1$ and $Z_1$ together are the epoxy group and $Z_2$ is the primary amino group, and Ar, n, alk, $R_1$ and $R_2$ have the meanings given above or

b) in a compound of the formula

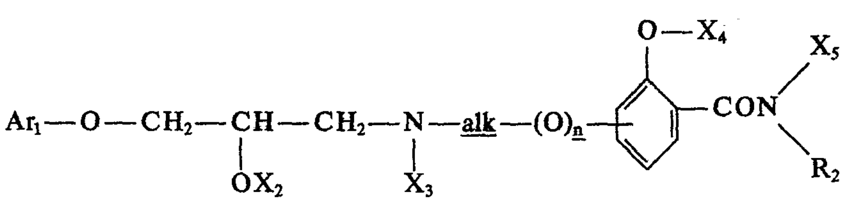

(IV)

wherein $Ar_1$ has the same meaning as Ar or is a radical Ar which is substituted by 1 or 2 groups

which may be converted into hydroxy, $X_2$, $X_3$ and $X_4$ are each hydrogen or a substituent which may be replaced by hydrogen, and $X_5$ is $R_1$, or $X_2$ and $X_3$ and/or $X_4$ and $X_5$ together are a divalent radical which may be replaced by two hydrogen atoms, with the proviso that at least one of the radicals $X_2$, $X_3$ and $X_4$ is different from hydrogen, or at least $Ar_1$ is a radical Ar which is substituted by 1 or 2 groups which may be converted into hydroxy, or at least $X_2$ and $X_3$ together or $X_4$ and $X_5$ together are a divalent radical which may be replaced by two hydrogen atoms, or in a salt thereof those of the groups $X_2$, $X_3$ and $X_4$, or $X_2$ and $X_3$ together, or $X_4$ and $X_5$ together, which are different from hydrogen are replaced by hydrogen atoms, and/or substituted hydroxy present in a radical $Ar_1$ is converted into free hydroxy, or

c) in a compound of the formula

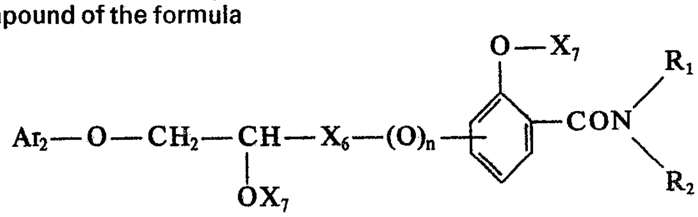

(V)

wherein $X_6$ is a reducible group of the formula

$-CH=N-\underline{alk}$ (V<u>a</u>)

or

$-CH_2-N=\underline{alk}_1-$ (V<u>b</u>)

in which $\underline{alk}_1$ is an alkylidene radical corresponding to the radical <u>alk</u>, and $X_7$ is hydrogen or a radical which may be replaced by hydrogen under the conditions for the reduction of $X_6$, $Ar_2$ corresponds to a radical Ar, but optionally, instead of carrying one or two hydroxy groups carries one or two $OX_7$ groups in which $X_7$ has the meaning given above, and <u>n</u> is 0 or 1, wherein $X_6$ is always a reducible group V<u>a</u> or V<u>b</u>, the $X_6$ group is reduced, and simultaneously the $X_7$ group different from hydrogen is replaced by hydrogen, or

d) reacting a compound of the formula

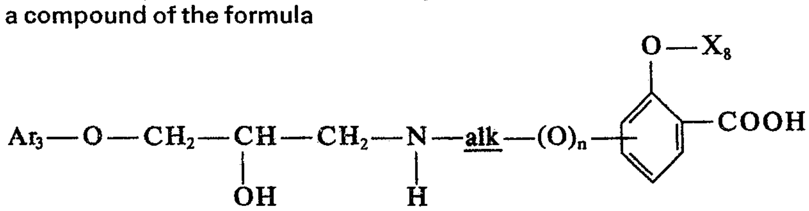

(VI)

wherein $Ar_3$ has the same meaning as Ar, or is a radical Ar which is substituted by 1 or 2 groups which may be converted into hydroxy by aminolysis, $X_8$ is hydrogen or a group which can be split off by aminolysis, or a reactive derivative of one of the carboxylic acids defined in formula VI, with a compound of the formula

$HNR_1R_2$ (VII)

and at the same time splitting off optionally present $X_8$ radicals and replacing them by hydrogen, or

e) in a compound of the formula

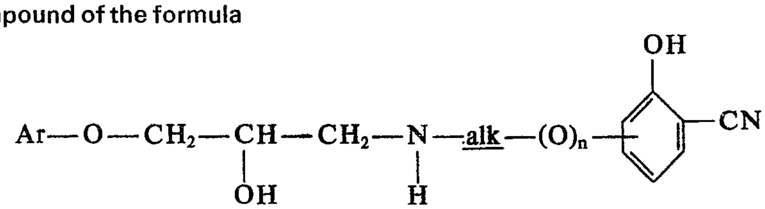

(VIII)

wherein one or both hydroxy groups and/or hydroxy groups optionally present in the radical Ar are protected by those groups that can be split off by hydrolysis and replaced by hydrogen, which groups are split off under the conditions of the process and are replaced by hydrogen, the $-CN$ group is converted by hydrolysis into the $-CONH_2$ group and, at the same time, optionally protected hydroxy groups are converted into free hydroxy groups, and, if desired, converting a resultant free compound into a salt or a resultant salt into a free compound, and/or, if desired,

separating a mixture of isomers into the individual isomers or resolving a racemate into the antipodes.

2. A process according to claim 1 for the preparation of a compound of the formula I, wherein Ar is mono- or bicyclic carbocyclic aryl or mono- or bicyclic heteroaryl which is bonded through a ring carbon atom and contains not more than two nitrogen atoms and/or an oxygen or sulfur atom as ring members, which radicals may be substituted or substituted, possible substituents being unsubstituted or substituted lower alkyl and selected from lower alkenyl or lower alkoxy, each of 1 to 7 carbon atoms, and lower alkenyloxy, lower alkynyl, lower alkynyloxy, each of 1 to 7 carbon atoms, cyano and/or nitro, and/or as substituents bonded direct or bonded to the above mentioned lower alkyl, lower alkenyl or lower alkoxy, each of 1 to 7 carbon atoms, one or more of lower alkanoyl, or esterified or amidated carboxyl, lower alkylsulfinyl, lower alkynsulfonyl, each of 1 to 7 carbon atoms, sulfamoyl or lower alkylsulfamoyl of 1 to 7 carbon atoms, and/or halogen bonded direct or to above mentioned lower alkyl of 1 to 7 carbon atoms or, in a position higher than in 1-position, to above mentioned lower alkoxy of 1 to 7 carbon atoms, free or etherified or esterified hydroxyl such as hydroxyl, or, as substituent which is not bonded direct, in turn lower alkoxy of 1 to 7 carbon atoms, and phenyl-lower alkoxy containing 1 to 7 carbon atoms in the lower alkoxy moiety, or lower alkenyloxy of 1 to 7 carbon atoms, etherified mercapto, unsubstituted or substituted amino, pyrrol-1-yl, acylamino, ureido which is unsubstituted or substituted by lower alkyl or hydroxy-lower alkyl, each of 1 to 7 carbon atoms, or by cycloalkyl containing 5 to 7 ring members, or lower alkylsulfonylamino of 1 to 7 carbon atoms, and, in bicyclic radicals Ar the ring which is not attached direct to the ether group may also be at least partially hydrogenated and, in this case, may also be substituted by oxo, and n is 0 or 1 and alk is a $C_2—C_4$-alkylene radical, and the nitrogen atom and the oxygen atom or, if n is 0, the phenyl radical, are separated from each other by 2 to 3 carbon atoms, and $R_1$ and $R_2$ are as defined in claim 1, wherein corresponding starting materials are used.

3. A process according to claim 1 for the preparation of a compound of the formula I, wherein Ar is monocyclic or bicyclic carbocyclic aryl or monocyclic or bicyclic heteroaryl bonded through a ring carbon atom and containing as ring members not more than two nitrogen atoms and/or one oxygen or sulfur atom, which radicals may be unsubstituted or mono- to trisubstituted, suitable substituents being unsubstituted or substituted in the manner specified hereinafter and selected from lower alkyl, lower alkenyl or lower alkoxy, each of 1 to 7 carbon atoms, cyano and/or nitro and/or, as substituents bonded direct to or to the above mentioned lower alkyl, lower alkenyl or lower alkoxy, each of 1 to 7 carbon atoms, one or more of lower alkanoyl, lower alkoxycarbonyl of 1 to 7 carbon atoms, carbamoyl, lower alkylcarbamoyl or (hydroxy)-lower alkyl)carbamoyl, each containing 1 to 7 carbon atoms in the lower alkyl and lower hydroxyalkyl moiety respectively, lower alkylsulfinyl or lower alkylsulfonyl, each of 1 to 7 carbon atoms, sulfamoyl or lower alkylsulfamoyl, each of 1 to 7 carbon atoms; and/or halogen bonded direct or halogen bonded to the above mentioned lower alkyl of 1 to 7 carbon atoms or, in a position higher than the 1-position, halogen bonded to the above mentioned lower alkoxy of 1 to 7 carbon atoms; hydroxyl; or, as substituent not bonded direct, in turn lower alkoxy of 1 to 7 carbon atoms, hydroxyl or, as substituent not bonded direct, in turn lower alkoxy of 1 to 7 carbon atoms, phenyl-lower alkoxy containing 1 to 7 carbon atoms in the lower alkoxy moiety, lower alkanoyloxy, or lower alkylthio, each of 1 to 7 carbon atoms, amino, lower alkylamino of 1 to 7 carbon atoms, di-lower alkylamino containing 1 to 7 carbon atoms in each alkyl moiety, alkyleneamino or oxaalkyleneamino, pyrro-1-yl, lower alkanoylamino of 1 to 7 carbon atoms, or lower alkoxycarbonylamino copntaining 1 to 7 carbon atoms in the lower alkoxy moiety, ureido which is unsubstituted or substituted by lower alkyl or hydroxy-lower alkyl, each of 1 to 7 carbon atoms, or by cycloalkyl containing 5 to 7 ring members, or lower alkylsulfonylamino of 1 to 7 carbon atoms, and in bicyclic radicals Ar the ring which ist not attached direct to the ether group may also be partially hydrogenated and, in this, may also be substituted by oxo. and n is 0 or 1 and alk is a $C_2—C_4$alkylene radical, and the nitrogen atom and the oxygen atom or, if n is 0, the phenyl radical, are separated from each other by 2 to 3 carbon atoms, and $R_1$ and $R_2$ are as defined in claim 1, wherein corresponding starting materials are used.

4. A process according to claim 1 for the preparation of a compound of the formula I, wherein Ar is phenyl, naphthyl or 1,2,3,4-tetra-5-naphthyl, which radicals may be unsubstituted or mono- to trisubstituted, suitable substituents being, unsubstituted or substituted in the manner specified hereinafter and selected from lower alkyl, lower alkenyl or lower alkoxy, each of 1 to 7 carbon atoms, and lower alkenyloxy, lower alkynyloxy, each of 1 to 7 carbon atoms, and/or cyano, and/or, as substituents bonded direct or to above mentioned lower alkyl, lower alkenyl or lower alkoxy of 1 to 7 carbon atoms, one or more of lower alkanoyl of 1 to 7 carbon atoms, lower alkoxycarbonyl containing 1 to 7 carbon atoms in the alkoxy moiety, carbamoyl, lower alkylcarbamoyl or (hydroxy-lower alkyl)-carbamoyl, each containing 1 to 7 carbon atoms in the lower alkyl or hydroxy-lower alkyl moiety respectively, lower alkylsulfinyl or lower alkylsulfonyl, each of 1 to 7 carbon atoms, sulfamoyl or lower alkylsulfamoyl of 1 to 7 carbon atoms, and/or as halogen bonded direct or to above mentioned lower alkyl of 1 to 7 carbon atoms, or to above mentioned lower alkoxy of 1 to 7 carbon atoms in a position higher than the 1-position, hydroxy or, as substituent not bonded direct, in turn lower alkoxy of 1 to 7 carbon atoms, phenyl-lower alkoxy containing 1 to 7 carbon atoms in the alkoxy moiety, lower alkanoyloxy or lower

alkylthio, each of 1 to 7 carbon atoms, amino, lower alkylamino of 1 to 7 carbon atoms, di-lower alkylamino containing 1 to 7 carbon atoms in each alkyl moiety, alkyleneamino or oxaalkyleneamino, pyrrol-1-yl, lower alkylanoylamino of 1 to 7 carbon atoms, lower alkoxycarbonylamino containing 1 to 7 carbon atoms in the lower alkoxy moiety, ureido which is unsubstituted or substituted by lower alkyl of 1 to 7 carbon atoms or cycloalkyl containing 5 to 7 ring members, or lower alkylsulfonylamino of 1 to 7 carbon atoms, and n is 0 or 1 and alk is a $C_2$—$C_4$alkylene radical, and the nitrogen atom and the oxygen atom or, if n is 0, the phenyl radical, are separated from each other by 2 to 3 carbon atoms, and $R_1$ and $R_2$ are each independently hydrogen or lower alkyl of 1 to 7 carbon atoms, or together with the nitrogen atom of the amide group form morpholino, wherein corresponding starting materials are used.

5. A process according to claim 1 for the preparation of a compound of the formula I, wherein Ar is phenyl which may be unsubstituted or mono- to trisubstituted, suitable substituents being, unsubstituted or substituted in the manner specified hereinafter and selected from lower alkyl or lower alkoxy, each of 1 to 7 carbon atoms, and lower alkenyl, lower alkenyloxy or lower alkynyloxy, each of 1 to 7 carbon atoms, and/or cyano, and/or as substituents bonded direct or to the above mentioned lower alkyl or lower alkoxy, each of 1 to 7 carbon atoms, lower alkanoyl of 1 to 7 carbon atoms, lower alkoxycarbonyl containing 1 to 7 carbon atoms in the lower alkoxy moiety, carbamoyl, lower alkylcarbamoyl or (hydroxy-lower alkyl)-carbamoyl, each containing 1 to 7 carbon atoms in the lower alkyl or hydroxy-lower moiety respectively, lower alkylsulfinyl or lower alkylsulfonyl, each of 1 to 7 carbon atoms, and/or halogen bonded direct or to above mentioned lower alkyl of 1 to 7 carbon atoms or to above mentioned lower alkoxy of 1 to 7 carbon atoms in a position higher than the 1-position, hydroxy or, as substituent not bonded direct, in turn lower alkoxy of 1 to 7 carbon atoms, and also phenyl-lower alkoxy containing 1 to 7 carbon atoms in the lower alkoxy moiety, lower alkylthio of 1 to 7 carbon atoms, alkyleneamino or oxaalkyleneamino, pyrrol-1-yl, lower alkanoylamino of 1 to 7 carbon atoms, lower alkoxycarbonyl amino containing 1 to 7 carbon atoms in the lower alkoxy moiety, or ureido which is unsubstituted or substituted by lower alkyl of 1 to 7 carbon atoms, and n is 0 or 1 and alk is a $C_2$—$C_4$alkylene radical, and the nitrogen atom and the oxygen atom or, if n is 0, the phenyl radical, are separated from each other by 2 to 3 carbon atoms, and $R_1$ and $R_2$ are each independently hydrogen or lower alkyl of 1 to 7 carbon atoms, wherein corresponding starting materials are used.

6. A process according to claim 1 for the preparation of a compound of the formula I, wherein Ar is phenyl which may be unsubstituted or mono- to trisubstituted, suitable substituents being, unsubstituted or substituted in the manner specified hereinafter and selected from lower alkyl or lower alkoxy, each of 1 to 7 carbon atoms, and lower alkenyl, lower alkenyloxy or lower alkynyloxy, each of 1 to 7 carbon atoms, and/or cyano, and/or as substituents bonded direct or the above mentioned lower alkyl or lower alkoxy, each of 1 to 7 carbon atoms, lower alkanoyl of 1 to 7 carbon atoms, lower alkoxycarbonyl containing 1 to 7 carbon atoms in the lower alkoxy moiety, carbamoyl, lower alkylcarbamoyl or (hydroxy-lower alkyl)-carbamoyl, each containing 1 to 7 carbon atoms in the lower alkyl or hydroxy-lower moiety respectively, lower alkylsulfinyl or lower alkylsulfonyl, each of 1 to 7 carbon atoms, and/or halogen bonded direct orto above mentioned lower alkyl of 1 to 7 carbon atoms or to above mentioned lower alkoxy of 1 to 7 carbon atoms in a position higher than the 1-position, hydroxy or, as substituent not bonded direct, in turn lower alkoxy of 1 to 7 carbon atoms, and also phenyl-lower alkoxy containing 1 to 7 carbon atoms in the lower alkoxy moiety, lower alkylthio of 1 to 7 carbon atoms, alkyleneamino or oxaalkyleneamino, pyrrol-1-yl, lower alkanoylamino of 1 to 7 carbon atoms, lower alkoxycarbonylamino containing 1 to 7 carbon atoms in the lower alkoxy moiety, or ureido which is unsubstituted or substituted by lower alkyl of 1 to 7 carbon atoms, and n is 0 or 1 and alk is a $C_2$—$C_4$alkylene radical, and the nitrogen atom and the oxygen atom or, if n is 0, the phenyl radical, are separated from each other by 2 to 3 carbon atoms, and $R_1$ and $R_2$ are each independently hydrogen or lower alkyl of 1 to 7 carbon atoms, wherein corresponding starting materials are used.

7. A process according to claim 1 for the preparation of a compound of the formula I, wherein the phenyl radical carrying the amido and hydroxyl group is attached to the remainder of the molecule preferably in its 4-position and, most preferably, in its 5-position, wherein corresponding starting materials are used.

8. A process for the preparation of 1-[2-(3-carbamoyl-4-hydroxyphenoxy)ethylamino]-3-[4-(2-methoxyethoxy)phenoxy]-2-propanol according to claim 1, wherein 1-[N-[2-(3-carbamoyl-4-hydroxyphenoxy)ethyl]benzylamino]-3-[4-(2-methoxyethoxy)phenoxy]-2-propanol is used as starting material.

9. A process for the preparation of 1-[2-(3-carbamoyl-4-hydroxyphenoxy)ethylamino]-3-[4-(2-methoxyethoxy)phenoxy]-2-propanol according to claim 1, wherein 1-[4[(2-methoxyethoxy)phenoxy]-3-amino-2-propanol ist used as starting material.

10. A process for the preparation of 1-[2-(3-carbamoyl-4-hydroxyphenoxy)ethylamino]-3-[4-(2-methoxyethoxy)phenoxy]-2-propanol according to claim 1, wherein (2,3-dihydro-2,2-dimethyl-4H-1,3-benzoxazin-4-on-6-yloxy) acetyldehyde and 1-amino-3-[4-(2-methoxyethoxy)phenoxy]-2-propanol are used as starting materials.

11. A process for the preparation of 1-[2-(3-carbamoyl-4-hydroxyphenoxy)ethylamino]-3-[4-(2-methoxyethoxy)phenoxy]-2-propanol according to claim 1, wherein 1-[2-(3-cyano-4-hydroxyphenoxy)ethylamino]-3-[4-(2-methoxyethoxy)phenoxy]-2-propanol is used starting material.

12. A process for the preparation of 1-(2-allyloxyphenoxy)-3-[2-(3-carbamoyl-4-hydroxyphenoxy)ethylamino]-2-propanol according to claim 1, wherein 1-(2-allyloxyphenoxy)-3-amino-2-propanol and 6-(2-bromoethoxy)-2,3-dihydro[2,2-dimethyl-4H-1,3-benzoxazin-4-one are used as starting materials.

13. A process for the preparation of 1-(2-allyloxyphenoxy)-3-[2-(3-carbamoyl-4-hydroxyphenoxy)ethylamino]-2-propanol according to claim 1, wherein 1-(2-alkyloxyphenoxy)-3-amino-2-propanol and 5-(2-bromoethoxy)salicylamide are used as starting materials.

14. A process for the preparation of 1-(2-allyloxyphenoxy)-3-[2-(3-carbamoyl-4-hydroxyphenoxy)ethylamino]-2-propanol according to claim 1, wherein [2-(2,3-epoxypropoxy)phenyl]-2-allyl ether and 5-(2-aminoethoxy)salicylamide are used as starting materials.

15. A process for the preparation of 1-[2-(3-carbamoyl-4-hydroxyphenoxy)ethylamino]-3-[4-hydroxyphenoxy]-2-propanol according to claim 1, wherein 1-[N-[2-(3-carbamoyl-4-hydroxyphenoxy)-ethyl]benzylamino]-3-(4-benzyloxyphenoxy)-2-propanol is used as starting material.

16. A process for the preparation of 4-[2-hydroxy-3-[3-carbamoyl-4-hydroxyphenoxy)ethylamino]propoxy]phenylacetatamide according to claim 1, wherein 1-[N-[2-(3-carbamoyl-4-hydroxyphenoxy)ethyl]benzylamino]-3-(4-carbamoylmethylphenoxy)-2-propanol is used as starting material.

17. A process for the preparation of 1-[2-(3-carbamoyl-4-hydroxyphenoxy)ethylamino]-3-(4-carbamoylmethylphenoxy)-2-propanol according to claim 1, wherein (4-carbamoylmethylphenoxy)-2,3-epoxypropane and 5-(2-aminoethoxy)salicylamide are used as starting materials.

18. A process for the preparation of 1-[2-(3-carbamoyl-4-hydroxyphenoxy)ethylamino]-3-[2-(pyrrol-1-yl)phenoxy]-2-propanol according to claim 1, wherein 1-[N-2-(3-carbamoyl-4-hydroxyphenoxy)ethylbenzylamino]-3-[2-(pyrrol-1-yl)phenoxy]-2-propanol is used as starting material.

19. A process for the preparation of 1-[2-(3-carbamoyl-4-hydroxyphenoxy)ethylamino]-3-[4-(2-methoxycarbonylamino)ethoxy]-phenoxy-2-propanol according to claim 1, wherein 1-N-[2-(3-carbamoyl-4-hydroxyphenoxy)ethyl]benzylamino]-3-[4-2-(methoxycarbonylamino)-ethoxy)-phenoxy]-2-propanol is used as starting material.

20. A process for the preparation of 1-[2-(3-carbamoyl-4-hydroxyphenoxy)ethylamino]-3-(2-methylindol-4-yloxy)-2-propanol according to claim 1, wherein 1-{N-[2-(3-carbamoyl-4-hydroxyphenoxy)ethyl]-benzylamino}-3-(2-methylindol-4-yloxy)-2-propanol is used as starting material.

21. A process for the preparation of 4-[3-[2-carbamoyl-4-hydroxyphenoxy)ethylamino]-2 hydroxypropoxy]benzimidazol-2-one according to claim 1, wherein N-[2-(4-hydroxy-3-carbamoylphenoxy)ethyl]-N-[3-(2-oxobenzimidazol-4-yloxy)-2-hydroxypropyl]-N-benzylamine is used as starting material.

22. A process according to any one of claims 1 to 21, wherein a resultant compound of the formula I is converted into a pharmaceutically acceptable non-toxic addition salt.

**Revendications pour les Etats contractants: BE, CH, DE, FR, GB, IT, LU, SE, NL**

1. Dérivés du 3-amino-1,2-propanediol de formule

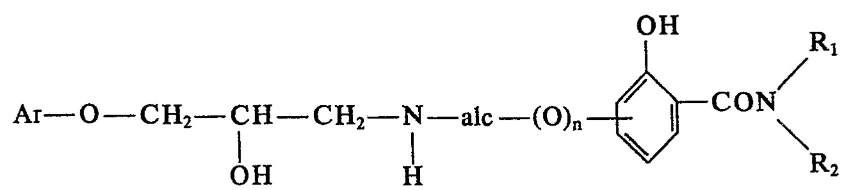

où Ar représente un aryle éventuellement substitué, n le nombre 0 ou 1 et Alc un alcoylène en $C_2$ à $C_5$, où l'atome d'atome d'oxygène ou, si n vaut zéro, le radical phényle sont séparés l'un de l'autre par au moins deux atomes de carbone, et $R_1$ et $R_2$ représentent chacun indépendamment l'un de l'autre un hydrogène ou un alcoyle inférieur ayant jusqu'à 7 atomes de carbone, ou représentent ensemble un alcoylène inférieur, un oxa-alcoylène inférieur, un thiaalcoylène inférieur ou un aza-alcoylène inférieur avec à chaque fois jusqu'à 7 atomes de carbone, ou un N-alcoyle inférieur-aza-alcoylène inférieur avec à chaque fois jusqu'à 7 atomes de carbone dans la fraction alcoyle inférieur ou selon les cas aza-alcoylèneinférieur.

2. Composés de formule I selon la revendication 1, où Ar représente un aryle carbocyclique mono- ou bicyclique ou un hétéroaryle lié par l'intermédiaire d'un atome de carbone du noyau, contenant comme chaînon au plus 2 atomes d'azote et/ou un atome d'oxygène ou un atome de soufre, radicaux qui peuvent être non substitués ou mono- ou poly-substitués, où l'on peut avoir comme substituants un alcoyle inférieur éventuellement substitué, un alcényle inférieur ou un alcoxy inférieur avec à chaqué fois jusqu'à 7 atomes de carbone, ou encore un alcényloxy inférieur, un alcynyle inférieur, un alcynyloxy inférieur avec à chaque fois jusqu'à 7 atomes de carbone, un cyano et/ou un nitro, et/ou un alcanoyle inférieur ayant jusqu'à 7 atomes de carbone lié directement ou sur l'alcoyle inférieur, l'alcényle inférieur ou l'alcoxy inférieur ayant chacun jusqu'à 7 atomes de carbone et mentionnés

ci-dessus, un carboxyle estérifié ou amidé, un alcoyle inférieursulfinyle, un alcoyle inférieur-sulfonyle ayant à chaque fois jusqu'à 7 atomes de carbone, un sulfamoyle ou un alcoyle inférieur-sulfamoyle ayant jusqu'à 7 atomes de carbone, et/ou un halogène lié, directement ou sur l'alcoyle inférieur ayant jusqu'à 7 atomes de carbone montionné ci-dessus ou sur l'alcoxy inférieur ayant jusqu'à 7 atomes de carbone mentionné ci-dessus, en position supérieure à la position 1, un hydroxy éventuellement éthérifié ou estérifié, comme un hydroxy, ou comme substituant non directement lié, à nouveau un alcoxy inférieur ayant jusqu'à 7 atomes de carbone, ou encore un phényl-alcoxy inférieur ayant jusqu'à 7 atomes de carbone dans la fraction alcoxy inférieur, ou un alcényloxy inférieur ayant jusqu'à 7 atomes de carbone, un mercapto éthérifié, un amino éventuellement substitué, un pyrrol-1-yle, un acylamino, un uréido éventuellement substitué par un alcoyle inférieur ou un hydroxy-alcoyle inférieur ayant à chaque fois jusqu'à 7 atomes de carbone, ou un cycloalcoyle ayant de 5 à 7 chaînons, ou un alcoyle inférieur-sulfonylamino ayant jusqu'à 7 atomes de carbone, et dans les radicaux bicycliques Ar le noyau non lié directement par le groupe éther peut également être au moins partiellement hydrogéné et dans ce cas également substitué par un oxo, et n représente le nombre zéro ou 1, et alc représente un radical alcoylène en $C_2$ à $C_4$, où l'atome d'azote et l'atome d'oxygène ou, si n vaut zéro, le radical phényle sont séparés l'un de l'autre par 2 à 3 atomes de carbone, et $R_1$ et $R_2$ ont la signification donnée dans la revendication 1.

3. Composés de formule I selon la revendication 1 où Ar représente un aryle carbocyclique mono- ou bicyclique ou un hétéro-aryle mono- ou bicyclique, lié par l'intermédiaire d'un atome de carbone du noyau, contenant au plus deux atomes d'azote et/ou un atome d'oxygène ou un atome de soufre comme chaînons, radicaux qui peuvent être non substitues ou mono- à tri-substitués, où l'on peut avoir comme substituants un alcoyle inférieur, un alcényle inférieur ou un alcoxy inférieur ayant à chaque fois jusqu'à 7 atomes de carbone et éventuellement substitué de la manière indiquée ci-dessous, ou encore un alcényloxy inférieur, un alcynyle inférieur, un alcynyloxy inférieur ayant à chaque fois jusqu'à 7 atomes de carbone, un cyano et/ou un nitro et/ou un alcanoyle inférieur ayant jusqu'à 7 atomes de carbone lié directement sur l'alcoyle inférieur, l'alcényle inférieur ou l'alcoxy inférieur mentionné ci-dessus ayant chacun jusqu'à 7 atomes de carbone, un alcoxy inférieur-carbonyle ayant jusqu'à 7 atomes de carbone dans la fraction alcoxy inférieur, un carbamoyle, un alcoyle inférieur-carbamoyle ou un (hydroxyalcoyle inférieur)-carbamoyle ayant à chaque fois jusqu'à 7 atomes de carbone dans la fraction alcoyle inférieur ou selon les cas (hydroxyalcoyle inférieur), un alcoyle inférieur-sulfinyle ou un alcoyle inférieur-sulfonyle ayant à chaque fois jusqu'à 7 atomes de carbone, un sulfamoyle ou un alcoyle inférieur-sulfamoyle ayant jusqu'à 7 atomes decarbone, et/ou un halogène lié directement ou sur l'alcoyle inférieur ayant jusqu'à 7 atomes de carbone mentionné ci-dessus, ou sur l'alcoxy inférieur ayant jusqu'à 7 atomes de carbone mentionné ci-dessus, en position supérieure à la position 1, un hydroxy ou, comme substituant non directement lié, à nouveau un alcoxy inférieur ayant jusqu'à 7 atomes de carbone, un phényl-alcoxy inférieur ayant jusqu'à 7 atomes de carbone dans la fraction alcocy inférieur, un alcanoyloxy inférieur, ou un alcoyle inférieur-thio ayant à chaque fois jusqu'à 7 atomes de carbone, un amino, un alcoyle inférieur-amino ayant jusqu'à 7 atomes de carbone, un dialcoyle inférieur-amino ayant à chaque fois jusqu'à 7 atomes de carbone dans les fractions alcoyle inférieur, un alcoylèneamino ou un oxaalcoylèneamino, un pyrrol-1-yle, un alcanoyle inférieur-amino ayant jusqu'à 7 atomes de carbone, ou un alcoxy inférieur-carbonylamino ayant jusqu'à 7 atomes de carbone dans la fraction alcoxy inférieur, un uréido éventuellement substitué par un alcoyle inférieur ou un hydroxyalcoyle inférieur ayant à chaque fois jusqu'à 7 atomes de carbone, ou un cycloalcoyle ayant de 5 à 7 chaînons, ou un alcoyle inférieur-sulfonylamino ayant jusqu'à 7 atomes de carbone, et dans les radicaux bicycliques Ar le noyau non directement lié par le groupe éther peut également être au moins partiellement hydrogéné et dans ce cas également substitué par un oxo, et n représente le nombre 0 ou 1, et alc représente un radical alcoylène en $C_2$ à $C_4$, où l'atome d'azote et l'atome d'oxygène ou, si n vaut zéro, le radical phényle sont séparés l'un de l'autre par 2 à 3 atomes de carbone, et $R_1$ et $R_2$ ont la signification donnée dans la revendication 1.

4. Composés de formule I selon la revendication 1, où Ar représente un phényle, un naphtyle ou un 1,2,3,4-tétrahydro-5-naphtyle, radicaux qui peuvent être non substitués ou mono- à tri-substitués, où l'on peut avoir comme substituants un alcoyle inférieur, un alcényle inférieur ou un alcoxy inférieur ayant chacun jusqu'à 7 atomes de carbone et éventuellement substutué de la manière indiquée ci-dessous, ou encore un alcényloxy inférieur, un alcynyloxy inférieur ayant à chaque fois jusqu'à 7 atomes de carbone et/ou un cyano et/ou alcanoyle inférieur ayant jusqu'à 7 atomes de carbone lié directement ou sur l'alcoyle inférieur, l'alcényle inférieur, ou l'alcoxy inférieur mentionné ci-dessus ayant à chaque fois jusqu'à 7 atomes de carbone, un alcoxy inférieur-carbonyle ayant jusqu'à 7 atomes de carbone dans la fraction alcoxy inférieur, un carbamoyle, un alcoyle inférieur-carbamoyle ou un (hydroxyalcoyle inférieur)-carbamoyle ayant à chaque fois jusqu'à 7 atomes de carbone dans la fraction alcoyle inférieur ou selon les cas (hydroxyalcoyle inférieur), un alcoyle inférieur-sulfinyle ou un alcoyle inférieursulfonyle ayant à chaque fois jusqu'à 7 atomes de carbone, un sulfamoyle ou un alcoyle inférieur-sulfamoyle ayant jusqu'à 7 atomes de carbone, et/ou un halogène lié directement ou sur l'alcoyle inférieur ayant jusqu'à 7 atomes de carbone mentionné ci-dessus ou sur l'alcoxy inférieur ayant jusqu'à 7 atomes de carbone mentionné ci-dessus en position supérieure à la position 1, un hydroxy ou, comme substituant non directement lié, à nouveau un alcoxy inférieur ayant jusqu'à 7

atomes carbone, un phényl-alcoxy inférieur ayant jusqu'à 7 atomes de carbone dans la fraction alcoxy inférieur, un alcanoyloxy inférieur ou un alcoyle inférieur-thio ayant à chaque fois jusqu'à 7 atomes de carbone, un amino, un alcoyle inférieur-amino ayant jusqu'à atomes de carbone, un dialcoyle inférieuramino ayant à chaque fois jusqu'à 7 atomes de carbone dans les fractions alcoyle inférieur, un alcoylèneamino ou un oxalacoylèneamino, un pyrrol-1-yle, un alcanoyle inférieur-amino ayant jusqu'à 7 atomes de carbone, un alcoxy inférieur-carbonylamino ayant jusqu'à 7 atomes de carbone dans la fraction alcoxy inférieur, un uréido éventuellement substitué par un alcoyle inférieur ayant jusqu'à 7 atomes de carbone ou un cycloalcoyle en $C_5$ à $C_7$, ou un alcoyle inférieur-sulfonylamino ayant jusqu'à 7 atomes de carbone, et n représente le nombre 0 ou 1 et alc représente un radical alcoylène en $C_2$ à $C_4$, où l'atome d'azote et l'atome d'oxygène ou, si n vaut zéro, le radical phénylène sont séparés l'un de l'autre par 2 à 3 atomes de carbone et $R_1$ et $R_2$ représent chacun indépendamment l'un de l'autre un hydrogène ou un alcoyle inférieur ayant jusqu'à 7 atomes de carbone, ou forment ensemble avec l'atome d'azote du groupe amide un morpholino.

5. Composés de formule I selon la revendication 1, où Ar représente un phényle, qui peut être non substitué ou mono- à tri-substitué, où l'on peut avoir comme substituants un alcoyle inférieur ou un alcoxy inférieur ayant chacun jusqu'à 7 atomes de carbone et éventuellement substitué de la manière indiquée cidessouss, ou encore un alcényle inférieur, un alcényloxy inférieur ou un alcynyloxy inférieur ayant chacun jusqu'à 7 atomes carbone, et/ou un cyano, et/ou un alcanoyle inférieur ayant jusqu'à 7 atomes de carbone lié directement ou sur l'alcoyle inférieur ou l'alcoxy inférieur ayant chacun jusqu'à 7 atomes de carbone et mentionné ci-dessus, un alcoxy inférieur-carbonyle ayant jusqu'à 7 atomes de carbone dans la fraction alcoxy inférieur, un carbamoyle, un alcoyle inférieurcarbamoyle ou un (hydroxy-alcoyle inférieur)-carbamoyle ayant chacun jusqu'à 7 atomes de carbone dans la fraction alcoyle inférieur ou selon les cas (hydroxyalcoyle inférieur), un alcoyle inférieur-sulfinyle ou un alcoyle inférieur-sulfonyle ayant à chaque fois jusqu'à 7 atomes de carbone, et/ou un halogène lié directement ou sur l'alcoyle inférieur ayant jusqu'à 7 atomes de carbone mentionné ci-dessus ou sur l'alcoxy inférieur ayant jusqu'à 7 atomes de carbone mentionné ci-dessus en position supérieure à la position 1, un hydroxy ou, comme substituant non directement lié, à nouveau un alcoxy inférieur ayant jusqu'à 7 atomes de carbone, ou encore un phényl-alcoxy inférieur ayant jusqu'à 7 atomes de carbone dans la fraction alcoxy inférieur, un alcoyle inférieur thio ayant jusqu'à 7 atomes de carbone, un alcoylène- ou un oxaalcoylèneamino, un pyrrol-1-yle, un alcanoyle inférieur-amino ayant jusqu'à 7 atomes de carbone, un alcoxy inférieurcarbonylamino ayant jusqu'à 7 atomes de carbone dans la fraction alcoxy inférieur, ou un uréido éventuellement substitué par un alcoyle inférieur ayant jusqu'à 7 atomes de carbone, et n représente le nombre zéro ou 1, et alc représente un radical alcoylène en $C_2$ à $C_4$, où l'atome d'azote et l'atome d'oxygène ou, si n vaut zéro, le radical phényle sont sépares l'un de l'autre par 2 à 3 atomes de carbone, et $R_1$ et $R_2$ représentent chanun indépendamment l'un de l'autre un hydrogène ou un alcoyle inférieur ayant jusqu'à 7 atomes de carbone.

6. Composés de formule I selon la revendication 1, où Ar représente un phényle, qui peut être non substitué ou mono- à tri-substitué, et l'on peut avoir comme substituants un alcoyle inférieur ou un alcoxy inférieur ayant chacun jusqu'à 7 atomes de carbone éventuellement substitué de la manière indiquée ci-dessous, ou encore un alcényle inférieur, un alcényloxy inférieur ou un alcynyloxy inférieur avec à chaque fois jusqu'à 7 atomes de carbone, et/ou un cyano, et/ou un alcanoyle inférieur ayant jusqu'à 7 atomes de carbone lié directement ou sur l'alcoyle inférieur ou l'alcoxy inférieur ayant à chaque fois jusqu'à 7 atomes de carbone mentionné ci-dessus, un carbamoyle, un alcoyle inférieur-carbamoyle ou un (hydroxyalcoyle inférieur)-carbamoyle avec à chaque fois jusqu'à 7 atomes de carbone dans la fraction alcoyle inférieur ou selon les cas (hydroxyalcoyle inférieur), un alcoyle inférieur-sulfinyle ou un alcoyle inférieur-sulfonyle avec à chaque fois jusqu'à 7 atomes de carbone, et/ou un fluor ou un chlore lié directement ou sur l'alcoyle inférieur ayant jusqu'à 7 atomes de carbone mentionné ci-dessus, ou sur l'alcoxy inférieur ayant jusqu'à 7 atomes de carbone mentionné ci-dessus en position supérieure à la position 1, un hydroxy ou, comme substituant non directement lié, à nouveau un alcoxy inférieur ayant jusqu'à 7 atomes de carbone, ou encore un phényl-alcoxy inférieur ayant jusqu'à 7 atomes de carbone dans la fraction alcoxy inférieur, un alcoyle inférieur-thio ayant jusqu'à 7 atomes de carbone, un pyrrol-1-yle, un alcanoyle inférieur-amino ayant jusqu'à 7 atomes de carbone, un alcoxy inférieur-carbonylamino ayant jusqu'à 7 atomes de carbone dans la fraction alcoxy inférieur, ou un uréido, et n représente le nombre 1, et alc représente un radical alcoylène en $C_2$ à $C_4$, où l'atome d'azote et l'atome d'oxygène sont séparés l'un de l'autre par 2 atomes de carbone, et $R_1$ et $R_2$ représentent un hydrogène.

7. Composés de formule I selon la revendication 1, où le radical phényle portant le groupe amide et le groupe hydroxy est lié de préférence dans sa position 4 et surtout dans sa position 5 avec le reste de la molécule.

8. 1-[2-(3-carbamoyl-4-hydroxy-phénoxy)-éthylamino]-3-[4-(2-méthoxyéthoxy)-phénoxy]-2-propanol.

9. 1-(4-hydroxy-phénoxy)-3-[2-(3-carbamoyl-4-hydroxyphénoxy)-1-méthyléthylamino]-2-propanol.

10. 1-(2-allyloxy-phénoxy)-3-[2-(3-carbamoyl-4-hydroxyphénoxy)-éthylamino]-2-propanol.

11. 1-{4-[2-(acétamido)-éthoxy]-phénoxy}-3-[2-(3-carbamoyl-4-hydroxy-phénoxy)-éthylamino]-2-propanol.

12. 1-[2-(3-carbamoyl-4-hydroxy-phénoxy)-éthylamino]-3-[4-(2-oxopropoxy)-phénoxy]-2-propanol.

13. 1-[2-(4-carbamoyl-3-hydroxy-phénoxy]-1-méthyléthylamino]-3-[4-(2-méthoxy-éthoxy)-phénoxy]-2-propanol.

14. 4-[2-hydroxy-3-[(3-carbamoyl-4-hydroxy-phénoxy)-éthylamino-propoxy]-phénylacétamide.

15. 1-[2-(3-carbamoyl-4-hydroxy-phénoxy)-éthylamino]-3-{4-[2-(méthoxycarbonylamino)-éthoxy]-phenoxy}-2-propanol.

16. 1-[2-(3-carbamoyl-4-hydroxy-phénoxy)-éthylamino]-3-(3-méthyl-e-méthylsulfinyl-phénoxy)-2-propanol.

17. 1-[2-(3-carbamoyl-4-hydroxy-phénoxy)-éthylamino]-3-(2-cyano-phénoxy)-2-propanol.

18. 1-[2-(3-carbamoyl-4-hydroxy-phénoxy)-éthylamino]-3-(2-propyloxy-phénoxy)-2-propanol.

19. 1-[2-(3-carbamoyl-4-hydroxy-phénoxy)-éthylamino]-3-[2-(pyrrol-1-yl)-phénoxy]-2-propanol.

20. 1-(2-(3-carbamoyl-4-hydroxy-phénoxy)-éthylamino]-3-[2-(2-propynyloxy)-phénoxy]-2-propanol.

21. 1-[2-(3-carbamoyl-4-hydroxy-phénoxy)-éthylamino]-3-(2-méthoxy-phénoxy)-2-propanol.

22. 1-[2-(3-carbamoyl-4-hydroxy-phénoxy)-éthylamino]-3-(2-méthyl-indol-4-yloxy)-2-propanol.

23. 1-(2-acétyl-phénoxy)-3-[2-(3-carbamoyl-4-hydroxyphénoxy)-éthylamino]-2-propanol.

24. 5-{3-[2-(3-carbamoyl-4-hydroxy-phénoxy)-éthylamino]-2-hydroxy-propoxy}-1,2,3,4-tétrahydro--2,3-cis-naphthalène-diol.

25. 1-[2-(3-carbamoyl-4-hydroxy-phénoxy)-éethylamino]-3-(3-méthyl-phénoxy)-2-propanol.

26. 1-[2-(3-carbamoyl-4-hydroxy-phénoxy)-éthylamino]-3-(3-méthyl-phénoxy)-2-propanol.

27. Composés de formule I selon la revendication 1, ou leurs sels aux fins d'application dans un procédé de traitement thérapeutique du corps humain ou animal.

28. Composés de formule I selon la revendication 1, ou leurs sels, à action spécifique sur les récepteurs $\beta$-adrénergiques.

29. Sels de composés des revendications 1 à 26.

30. Sels d'addition d'acides non toxiques pharmaceutiquement acceptables de composés des revendications 1 à 26.

31. Préparations pharmaceutiques contenant l'un des composés des revendication 1 à 26 et 30.

32. Application de composés de formule I selon la revendication 1 et de sels d'addition d'acides non toxiques pharmaceutiquement acceptables, de tels composés aux fins de préparation de préparations pharmaceutiques.

33. Procédé de préparation de dérivés du 3-amino-1,2-propanediol de formule

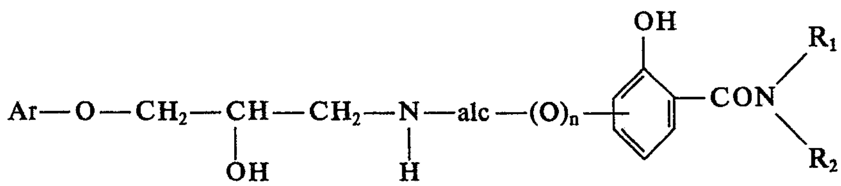

(I)

où Ar représente un aryle éventuellement substitué, n représente le nombre 0 ou 1 et alc un alcoylène en $C_2$ à $C_5$, où l'atome d'azote et l'atome d'oxygène ou, si n vaut zéro, le radical phényle sont séparés l'un de l'autre par au moins deux atomes de carbone, et $R_1$ et $R_2$ représentent chacun indépendamment l'un de l'autre un hydrogène ou un alcoyle inférieur ayant jusqu'à 7 atomes de carbone, ou représentent ensemble un alcoylène inférieur, un oxa-alcoylène inférieur, un thia-alcoylène inférieur, ou un aza-alcoylène inféreiur avec à chaque fois jusqu'à 7 atomes de carbone, ou un N-alcoyle inférieur-aza-alcoylène inférieur avec à chaque fois jusqu'à 7 atomes de carbone dans la fraction alcoyle inférieur ou selon les cas aza-alcoylène inférieur, caractérisé en ce que

a) on fait réagir un composé de formule

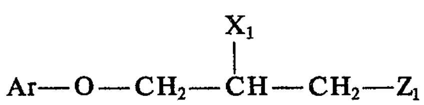

(II)

avec un composé de formule

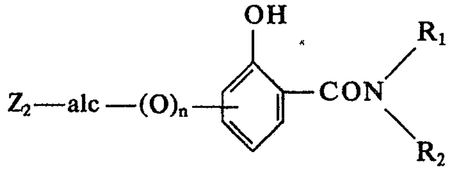

(III)

où l'un des groupes $Z_1$ et $Z_2$ représente un groupe hydroxy estérifié réactif et l'autre représente le groupe amino primaire, et $X_1$ représente un hydroxy, ou bien où $X_1$ et $Z_1$ représentent ensemble le

groupe époxy et $Z_2$ représente le groupe amino primaire, et Ar, n, alc, $R_1$ et $R_2$ ont la signification donnée ci-dessus, ou

b) dans un composé de formule

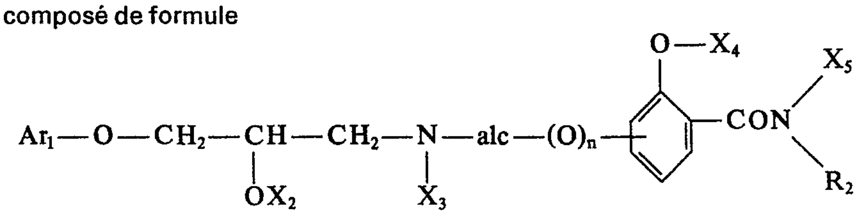

(IV)

où $Ar_1$ a la signification de Ar ou représente un radical Ar qui est substitué par 1 à 2 groupes transformables en hydroxy, $X_2$, $X_3$ et $X_4$ représentent à chaque fois un hydrogène ou un substituant remplaçable par un hydrogène et $X_5$ représente $R_1$, ou $X_2$ et $X_3$ et/ou $X_4$ et $X_5$ représentent ensemble un radical bivalent remplaçable par deux atomes d'hydrogène, avec cette précision qu'au moins l'un des radicaux $X_2$, $X_3$ et $X_4$ est différent d'un hydrogène, ou au moins $Ar_1$ représente un radical Ar qui est substitué par 1 à 2 groupes transformables en hydroxy, ou au moins $X_2$ et $X_3$ représentent ensemble et $X_4$ et $X_5$ ensemble un radical bivalent remplaçable par deux atomes d'hydrogène, ou dans un de ses sels, on remplace par des atomes d'hydrogène ceux des groupes $X_2$, $X_3$ et $X_4$ ou selon les cas $X_2$ et $X_3$ ensemble ou $X_4$ et $X_5$ ensemble qui sont différents de l'hydrogène, et/ou dans un radical $Ar_1$ on transforme un hydroxy substitué présent en un hydroxy libre, ou

c) dans un composé de formule

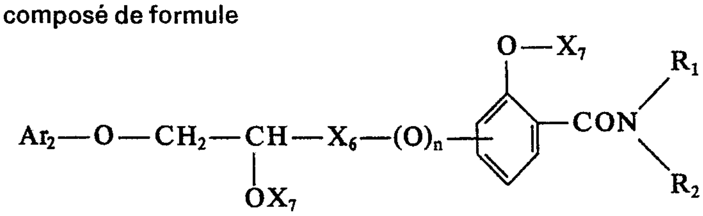

(V)

où $X_6$ est un groupe réductible de formules

$$-CH=N-alc- \qquad (Va)$$

ou selon les cas

$$-CH_2-N=alc_1- \qquad (Vb)$$

où $alc_1$ représente un radical alcoylidène correspondant au radical alc et $X_7$ représente un hydrogène ou un radical remplaçable par un hydrogène dans les conditions de la réduction de $X_6$, $Ar_2$ correspond à un radical Ar, mais porte le cas échéant à la place d'un ou deux hydroxy un ou deux groupes $OX_7$ où $X_7$ a la signification donnée ci-dessus, et n vaut 0 ou 1, ou $X_6$ est toujours un groupe réductible Va ou Vb, on réduit ce groupe et simultanément on remplace par un hydrogène le groupe $X_7$ différent d'un hydrogène, ou

d) on fait réagir un composé de formule

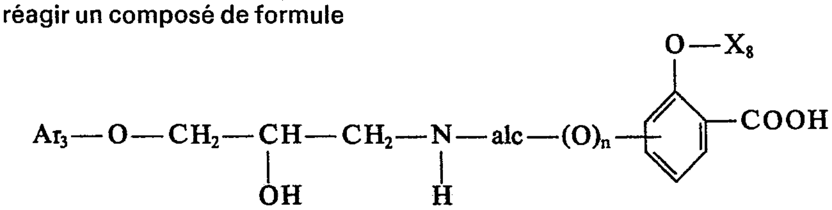

(VI)

où $Ar_3$ a la signification de Ar, ou représente un radical Ar qui est substitué par 1 à 2 groupes transformables en hydroxy au moyen d'une aminolyse, $X_8$ représente un hydrogène ou un groupe séparable au moyen d'une aminolyse, ou un dérivé réactif d'un des acides carboxyliques définis dans la formule VI, avec un composé de formule

$$HNR_1R_2 \qquad (VII)$$

et simultanément on sépare les radicaux $X_8$ éventuellement présents et on les remplace par de l'hydrogène, ou

e) dans un composé de formule

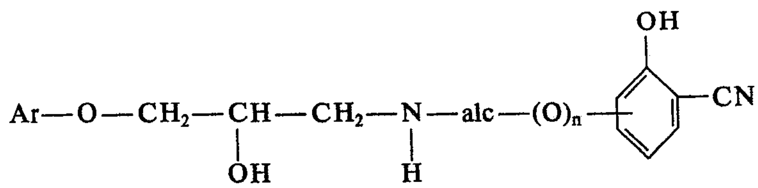

(VIII)

où un ou deux groupes hydroxy et/ou groupes hydroxy présents dans le radical Ar sont éventuellement protégés par des groupes de ce genre séparables par hydrolyse et remplaçables par de l'hydrogène, lesquels groupes sont séparés dans les conditions du procédé et remplacés par de l'hydrogène, on transforme le groupe $-CN$ au moyen d'une hydrolyse en le groupe $-CONH_2$ et simultanément on transforme les groupes hydroxy éventuellement protégés en groupes hydroxy libres, et, si on le désire, on transforme un composé ainsi obtenu en un autre composé de formule I et/ou, si on le désire, on transforme un composé libre obtenu en un sel ou un sel obtenu en un composé libre, et/ou, si on le désire, on sépare un mélange d'isomeres obtenu pour donner les isomères ou un racémate obtenu pour donner les antipodes.

34. Les composés que l'on peut obtenir selon le procédé de la revendication 33.

**Revendications pour l'Etat contractant: AT**

1. Procédé de préparation de dérivés du 3-amino-1,2-propanediol de formule

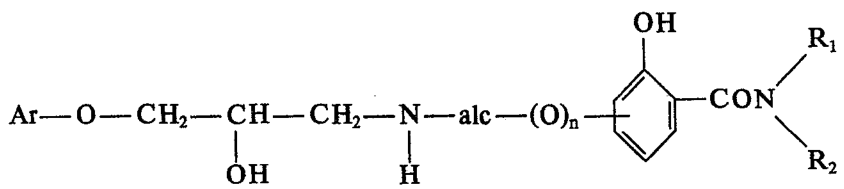

(I)

où Ar représente un aryle éventuellement substitué, n représente le nombre 0 ou 1 et alc un alcoylène en $C_2$ à $C_5$, où l'atome d'azote et l'atome d'oxygène ou, si n vaut zéro, le radical phenyle sont séparés l'un de l'autre par au moins deux atomes de carbone, et $R_1$ et $R_2$ représentent chacun indépendamment l'un de l'autre un hydrogène ou un alcoyle inférieur ayant jusqu'à 7 atomes de carbone, ou représentent ensemble un alcoylène inférieur, un oxa-alcoylène inférieur, un thia-alcoylène inférieur, ou un aza-alcoylène inférieur avec à chaque fois jusqu'à 7 atomes de carbone, ou un N-alcoyle inférieur-aza-alcoylène inférieur avec à chaque fois jusqu'à 7 atomes de carbone dans la fraction alcoyle inférieur ou selon les cas aza-alcoylène inférieur, caractérisé en ce que

a) on fait réagir un composé de formule

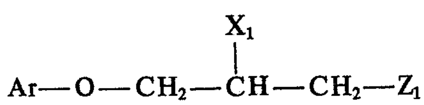

(II)

avec un composé de formule

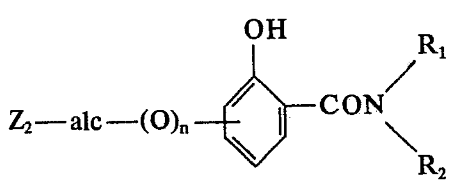

(III)

où l'un des groupes $Z_1$ et $Z_2$ représente un groupe hydroxy estérifié réactif et l'autre représente le groupe amino primaire, et $X_1$ représente un hydroxy, ou bien où $X_1$ et $Z_1$ représentent ensemble le groupe époxy et $Z_2$ représente le groupe amino primaire, et Ar, n, alc, $R_1$ et $R_2$ ont la signification donnée ci-dessus, ou

b) dans un composé de formule

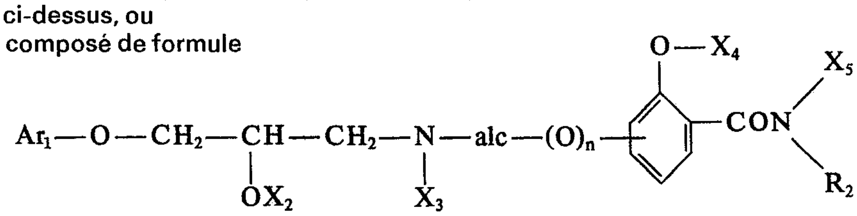

(IV)

où $Ar_1$ a la signification de Ar ou représente un radical Ar qui est substitué par 1 à 2 groupes transformables en hydroxy, $X_2$, $X_3$ et $X_4$ représentent à chaque fois un hydrogène ou un substituant remplaçable par un hydrogène et $X_5$ représente $R_1$, ou $X_2$ et $X_3$ et/ou $X_4$ et $X_5$ représentent ensemble un radical bivalent remplaçable par deux atomes d'hydrogène, avec cette précision qu'au moins l'un des radicaux $X_2$, $X_3$ et $X_4$ est différent d'un hydrogène, ou au moins $Ar_1$ représente un radical Ar qui est substitué par 1 à 2 groupes transformables en hydroxy, ou au moins $X_2$ et $X_3$ représentent ensemble et $X_4$ et $X_5$ ensemble un radical bivalent remplaçable par deux atomes d'hydrogène, ou dans un de ses sels, on remplace par des atomes d'hydrogène ceux des groupes $X_2$, $X_3$ et $X_4$ ou selon les cas $X_2$ et $X_3$ ensemble ou $X_4$ et $X_5$ ensemble qui sont différents de l'hydrogène, et/ou dans un radical $Ar_1$ on transforme un hydroxy substitué présent en un hydroxy libre, ou

c) dans un composé de formule

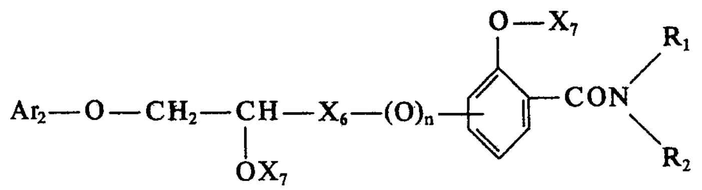
(V)

où $X_6$ est un groupe réductible de formules

$$-CH=N-alc- \qquad (Va)$$

ou selon les cas

$$-CH_2-N=alc_1- \qquad (Vb)$$

où $alc_1$ représente un radical alcoylidène correspondant au radical alc et $X_7$ représente un hydrogène ou un radical remplaçable par un hydrogène dans les conditions de la réduction de $X_6$, $Ar_2$ correspond à un radical Ar, mais porte le cas échéant à la place d'un ou deux hydroxy un ou deux groupes $OX_7$ où $X_7$ a la signification donnée ci-dessus, et n vaut 0 ou 1, ou $X_6$ est toujours un groupe réductible Va ou Vb, on réduit ce groupe et simultanément on remplace par un hydrogène le groupe $X_7$ différent d'un hydrogène, ou

d) on fait réagir un composé de formule

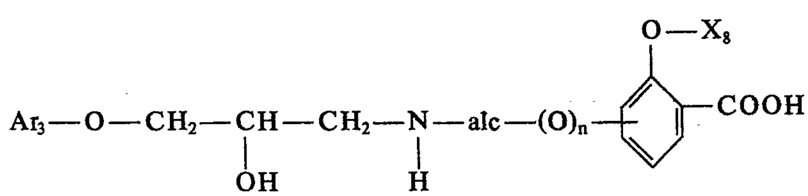
(VI)

où $Ar_3$ a la signification de Ar, ou représente un radical Ar qui est substitué par 1 à 2 groupes transformables en hydroxy au moyen d'une aminolyse, $X_8$ représente un hydrogène ou un groupe séparable au moyen d'une aminolyse, ou un dérivé réactif d'un des acides carboxyliques définis dans la formule VI, avec un composé de formule

$$HNR_1R_2 \qquad (VII)$$

et simultanément on sépare les radicaux $X_8$ éventuellement présents et on les remplace par de l'hydrogène, ou

e) dans un composé de formule

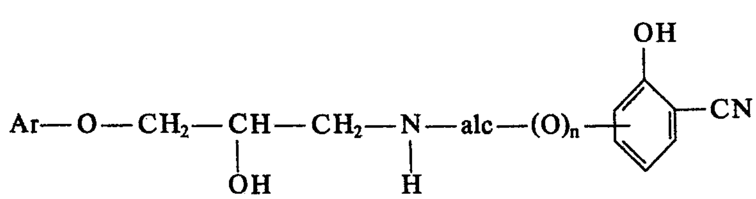
(VIII)

où un ou deux groupes hydroxy et/ou groupes hydroxy présents dans le radical Ar sont éventuellement protégés par des groupes de ce genre séparables par hydrolyse et remplaçables par de l'hydrogène, lesquels groupes sont séparés dans les conditions du procédé et remplacés par de l'hydrogène, on transforme le groupe —CN au moyen d'une hydrolyse en le groupe $-CONH_2$ et simultanément on transforme les groupes hydroxy éventuellement protégés en groupes hydroxy libres, et, si on le désire, on transforme un composé ainsi obtenu en un autre composé de formule I et/ou, si on le désire, on transforme un composé libre obtenu en un sel ou un sel obtenu en un composé libre, et/ou, si on le désire, on sépare un mélange d'isomères obtenu pour donner les isomères ou un racémate obtenu pour donner les antipodes.

2. Procédé selon la revendication 1 de préparation de composés de formule I, où Ar représente un aryle carbocyclique mono- ou bicyclique ou un hétéroaryle lié par l'intermédiaire d'un atome de carbone du noyau, contenant comme chaînon au plus 2 atomes d'azote et/ou un atome d'oxygène ou un atome de soufre, radicaux qui peuvent être non substitués ou mono- ou poly-substitués, où l'on peut avoir comme substituants un alcoyle inférieur éventuellement substitué, un alcényle inférieur ou un alcoxy inférieur avec à chaque fois jusqu'à 7 atomes de carbone, ou encore un alcényloxy inférieur, un alcynyle inférieur, un alcynyloxy inférieur avec à chaque fois jusqu'à 7 atomes de carbone, un cyano et/ou un nitro, et/ou un alcanoyle inférieur ayant jusqu'à 7 atomes de carbone lié directement ou sur l'alcoyle inférieur, l'alcényle inférieur ou l'alcoxy inférieur ayant chacun jusqu'à 7 atomes de carbone et mentionnés ci-dessus, un carboxyle estérifié ou amidé, un alcoyle inférieur-sulfinyle, un alcoyle inférieur-sulfonyle ayant à chaque fois jusqu'à 7 atomes de carbone, un sulfamoyle ou un alcoyle inférieur-sulfamoyle ayant jusqu'à 7 atomes de carbone, et/ou un halogène lié, directement ou sur l'alcoyle inférieur ayant jusqu'à 7 atomes de carbone mentionné ci-dessus ou sur l'alcoxy inférieur ayant jusqu'à 7 atomes de carbone mentionné ci-dessus, en position supérieure à la position 1, un hydroxy éventuellement éthérifié ou estérifié, comme un hydroxy, ou comme substituant non directement lié, à nouveau un alcoxy inférieur ayant jusqu'à 7 atomes de carbone, ou encore un phényl-alcoxy inférieur ayant jusqu'à 7 atomes de carbone dans la fraction alcoxy inférieur, ou un alcényloxy inférieur ayant jusqu'à 7 atomes de carbone, un mercapto éthérifié, un amino éventuellement substitué, un pyrrol-1-yle, un acylamino, un uréido éventuellement substitué par un alcoyle inférieur ou un hydroxy-alcoyle inférieur ayant à chaque fois jusqu'à 7 atomes de carbone, ou un cycloalcoyle ayant de 5 à 7 chaînons, ou un alcoyle inférieur-sulfonylamino ayant jusqu'à 7 atomes de carbone, et dans les radicaux bicycliques Ar le noyau non lié directement par le groupe éther peut également être au moins partiellement hydrogéné et dans ce cas également substitué par un oxo, et n représente le nombre zéro ou 1, et alc représente un radical alcoylène en $C_2$ à $C_4$, où l'atome d'azote et l'atome d'oxygène ou, si n vaut zéro, le radical phényle sont séparés l'un de l'autre par 2 à 3 atomes de carbone, et $R_1$ et $R_2$ ont la signification donnée dans la revendication 1, caractérisé en ce qu'on utilise des produits de départ correspondants.

3. Procédé selon la revendication 1 de préparation de composés de formule I, où Ar représente un aryle carbocyclique mono- ou bicyclique ou un hétéro-aryle mono- ou bicyclique, lié par l'intermédiaire d'un atome de carbone du noyau, contenant au plus deux atomes d'azote et/ou un atome d'oxygène ou un atome de soufre comme chaînons, radicaux qui peuvent être non substitués ou mono- à tri-substitués, où l'on peut avoir comme substituants un alcoyle inférieur, un alcényle inférieur ou un alcoxy inférieur ayant à chaque fois jusqu'à 7 atomes de carbone et éventuellement substitué de la manière indiquée ci-dessous, ou encore un alcényloxy inférieur, un alcynyle inférieur, un alcynyloxy inférieur ayant à chaque fois jusqu'à 7 atomes de carbone, un cyano et/ou un nitro et/ou un alcanoyle inférieur ayant jusqu'à 7 atomes de carbone lié directement sur l'alcoyle inférieur, l'alcényle inférieur ou l'alcoxy inférieur mentionné ci-dessus ayant chacun jusqu'à 7 atomes de carbone, un alcoxy inférieur-carbonyle ayant jusqu'à 7 atomes de carbone dans la fraction alcoxy inférieur, un carbamoyle, un alcoyle inférieur-carbamoyle ou un (hydroxyalcoyle inférieur)-carbamoyle ayant à chaque fois jusqu'à 7 atomes de carbone dans la fraction alcoyle inférieur ou selon les cas (hydroxyalcoyle inférieur), un alcoyle inférieur-sulfinyle ou un alcoyle inférieur-sulfonyle ayant à chaque fois jusqu'à 7 atomes de carbone, un sulfamoyle ou un alcoyle inférieur-sulfamoyle ayant jusqu'à 7 atomes de carbone, et/ou un halogène lié directement ou sur l'alcoyle inférieur ayant jusqu'à 7 atomes de carbone mentionné ci-dessus, ou sur l'alcoxy inférieur ayant jusqu'à 7 atomes de carbone mentionné ci-dessus, en position supérieure à la position 1, un hydroxy ou, comme substituant non directement lié, à nouveau un alcoxy inférieur ayant jusqu'à 7 atomes de carbone, un phényl-alcoxy inférieur ayant jusqu'à 7 atomes de carbone dans la fraction alcoxy inférieur, un alcanoyloxy inférieur, ou un alcoyle inférieur-thio ayant à chaque fois jusqu'à 7 atomes de carbone, un amino, un alcoyle inférieur-amino ayant jusqu'à 7 atomes de carbone, un dialcoyle inférieur-amino ayant à chaque fois jusqu'à 7 atomes de carbone dans les fractions alcoyle inférieur, un alcoylèneamino ou un oxaalcoylèneamino, un pyrrol-1-yle, un alcanoyle inférieur-amino ayant jusqu'à 7 atomes de carbone, ou un alcoxy inférieur-carbonylamino ayant jusqu'à 7 atomes de carbone dans la fraction alcoxy inférieur, un uréido éventuellement substiué par un alcoyle inférieur ou un hydroxyalcoyle inférieur ayant à chaque fois jusqu'à 7 atomes de carbone, ou un cycloalcoyle ayant de 5 à 7 chaînons, ou un alcoyle inférieur-sulfonylamino ayant jusqu'à 7 atomes de carbone, et dans les radicaux bicycliques Ar le noyau non directement lié par le groupe éther peut également être au moins partiellement hydrogéné et dans ce cas également

substitué par un oxo, et n représente le nombre 0 ou 1, et alc représente un radical alcoylène en $C_2$ à $C_4$, où l'atome d'azote et l'atome d'oxygène ou, si n vaut zéro, le radical phényle sont séparés l'un de l'autre par 2 à 3 atomes de carbone, et $R_1$ et $R_2$ ont la signification donnée dans la revendication 1.

4. Procédé selon la revendication 1 de préparation de composés de formule I, où Ar représente un phényle, un naphtyle ou un 1,2,3,4-tétrahydro-5naphtyle radicaux qui peuvent être non substitués ou mono- à tri-substitués où l'on peut avoir comme substituants un alcoyle inférieur, un alcényle inférieur ou un alcoxy inférieur ayant chacun jusqu'à 7 atomes de carbone et éventuellement substitué de la manière indiquée ci-dessous, ou encore un alcényloxy inférieur, un alcynyloxy inférieur ayant à chaque fois jusqu'à 7 atomes de carbone et/ou un cyano et/ou un alcanoyle inférieur ayant jusqu'à 7 atomes de carbone lié directement ou sur l'alcoyle inférieur, l'alcényle inférieur, ou l'alcoxy inférieur mentionné ci-dessus ayant à chaque fois jusqu'à 7 atomes de carbone, un alcoxy inférieur-carbonyle ayant jusqu'à 7 atomes de carbone dans la fraction alcoxy inférieur, un carbamoyle, un alcoyle inférieur-carbamoyle ou un (hydroxyalcoyle inférieur)-carbamoyle ayant à chaque fois jusqu'à 7 atomes de carbone dans la fraction alcoyle inférieur ou selon les cas (hydroxyalcoyle inférieur), un alcoyle inférieur-sulfinyle ou un alcoyle inférieur-sulfonyle ayant à chaque fois jusqu'à 7 atomes de carbone, un sulfamoyle ou un alcoyle inférieur-sulfamoyle ayant jusqu'à 7 atomes de carbone, et/ou un halogène lié directement ou sur l'alcoyle inférieur ayant jusqu'à 7 atomes de carbone mentionné ci-dessus ou sur l'alcoxy inférieur ayant jusqu'à 7 atomes de carbone mentionné ci-dessus en position supérieure à la position 1, un hydroxy ou, comme substituant non directement lié, à nouveau un alcoxy inférieur ayant jusqu'à 7 atomes de carbone, un phényl-alcoxy inférieur ayant jusqu'à 7 atomes de carbone dans la fraction alcoxy inférieur, un alcanoyloxy inférieur ou un alcoyle inférieur-thio ayant à chaque fois jusqu'à 7 atomes de carbone, un amino, un alcoyle inférieur-amino ayant jusqu'à 7 atomes de carbone, un dialcoyle inférieur-amino ayant à chaque fois jusqu'à 7 atomes de carbone dans les fractions alcoyle inférieur, un alcoylèneamino ou un oxalacoylèneamino, un pyrrol-1-yle, un alcanoyle inférieur-amino ayant jusqu'à 7 atomes de carbone, un alcoxy inférieur-carbonylamino ayant jusqu'à 7 atomes de carbone dans la fraction alcoxy inférieur, un uréido éventuellement substitué par un alcoyle inférieur ayant jusqu'à 7 atomes de carbone ou un cycloalcoyle en $C_5$ à $C_7$, ou un alcoyle inférieur-sulfonylamino ayant jusqu'à 7 atomes de carbone, et n représente le nombre 0 ou 1 et alc représente un radical alcoylène en $C_2$ à $C_4$, où l'atome d'azote et l'atome d'oxygène ou, si n vaut zéro, le radical phénylène sont séparés l'un de l'autre par 2 à 3 atomes de carbone et $R_1$ et $R_2$ représent chacun indépendamment l'un de l'autre un hydrogène ou un alcoyle inférieur ayant jusqu'à 7 atomes de carbone, ou forment ensemble avec l'atome d'azote du groupe amide un morpholino.

5. Procédé selon la revendication 1 de préparation de composés de formule I, où Ar représente un phényle, qui peut être non substitué ou mono- à tri-substitué, où l'on peut avoir comme substituants un alcoyle inférieur ou un alcoxy inférieur ayant chacun jusqu'à 7 atomes de carbone et éventuellement substitué de la manière indiquée ci-dessous, ou encore un alcényle inférieur, un alcényloxy inférieur ou un alcynyloxy inférieur ayant chacun jusqu'à 7 atomes de carbone, et/ou un cyano, et/ou un alcanoyle inférieur ayant jusqu'à 7 atomes de carbone lié directement ou sur l'alcoyle inférieur ou l'alcoxy inférieur ayant chacun jusqu'à 7 atomes de carbone et mentionné ci-dessus, un alcoxy inférieur-carbonyle ayant jusqu'à 7 atomes de carbone dans la fraction alcoxy inférieur, un carbamoyle, un alcoyle inférieur-carbamoyle ou un (hydroxy-alcoyle inférieur)-carbamoyle ayant chacun jusqu'à 7 atomes de carbone dans la fraction alcoyle inférieur ou selon les cas (hydroxy-alcoyle inférieur), un alcoyle inférieur-sulfinyle ou un alcoyle inférieur-sulfonyle ayant à chaque fois jusqu'à 7 atomes de carbone, et/ou un halogène lié directement ou sur l'alcoyle inférieur ayant jusqu'à 7 atomes de carbone mentionné ci-dessus ou sur l'alcoxy inférieur ayant jusqu'à 7 atomes de carbone mentionné ci-dessus en position supérieure à la position 1, un hydroxy ou, comme substituant non directement lié, à nouveau un alcoxy inférieur ayant jusqu'à 7 atomes de carbone, ou encore un phényl-alcoxy inférieur ayant jusqu'à 7 atomes de carbone dans la fraction alcoxy inféreiur, un alcoyle inférieur thio ayant jusqu'à 7 atomes de carbone, un alcoylène- ou un oxaalcoylène-amino, un pyrrol-1-yle, un alcanoyle inférieur-amino ayant jusqu'à 7 atomes de carbone, un alcoxy inférieur-carbonylamino ayant jusqu'à 7 atomes de carbone dans la fraction alcoxy inférieur, ou un uréido éventuellement substitué par un alcoyle inférieur ayant jusqu'à 7 atomes de carbone, et n représente le nombre zéro ou 1, et alc représente un radical alcoylène en $C_2$ à $C_4$, où l'atome d'azote et l'atome d'oxygène ou, si n vaut zéro, le radical phényle sont séparés l'un de l'autre par 2 à 3 atomes de carbone, et $R_1$ et $R_2$ représentent chacun indépendamment l'un de l'autre un hydrogène ou un alcoyle inférieur ayant jusqu'à 7 atomes de carbone.

6. Procédé selon la revendication 1 de préparation de composés de formule I, ou Ar représente un phényle, qui peut être non substitué ou mono- à tri-substitué, et l'on peut avoir comme substituants un alcoyle inférieur ou un alcoxy inférieur ayant jusqu'à 7 atomes de carbone éventuellement substitué de la manière indiquée ci-dessous, ou encore un alcényle inférieur, un alcényloxy inférieur ou un alcynyloxy inférieur avec à chaque fois jusqu'à 7 atomes de carbone, et/ou un cyano, et/ou un alcanoyle inférieur ayant jusqu'à 7 atomes de carbone lié directement ou sur l'alcoyle inférieur ou l'alcoxy inférieur ayant à chaque fois jusqu'à 7 atomes de carbone mentionné ci-dessus, un carbamoyle, un alcoyle inférieur-carbamoyle ou un (hydroxyalcoyle inférieur)-carbamoyle avec à chaque fois jusqu'à 7 atomes de carbone dans la fraction alcoyle inférieur ou selon les cas (hydroxyalcoyle

inférieur), un alcoyle inférieur-sulfinyle ou un alcoyle inférieur-sulfonyle avec à chaque fois jusqu'à 7 atomes de carbone, et/ou un fluor ou un chlore lié directement ou sur l'alcoyle inférieur ayant jusqu'à 7 atomes de carbone mentionné ci-dessus, ou sur l'alcoxy inférieur ayant jusqu'à 7 atomes de carbone mentionné ci-dessus en position supérieure à la position 1, un hydroxy ou, comme substituant non directement lié, à noveau un alcoxy inférieur ayant jusqu'à 7 atomes de carbone, ou encore un phényl-alcoxy inférieur ayant jusqu'à 7 atomes de carbone dans la fraction alcoxy inférieur, un alcoyle inférieur-thio ayant jusqu'à 7 atomes de carbone, un pyrrol-1-yle, un alcanoyle inférieur-amino ayant jusqu'à 7 atomes de carbone, un alcoxy inférieur-carbonylamino ayant jusqu'à 7 atomes de carbone dans la fraction alcoxy inférieur, ou un uréido, et n représente le nombre 1, et alc représente un radical alcoylène en $C_2$ à $C_4$, où l'atome d'azote et l'atome d'oxygène sont séparés l'un de l'autre par 2 atomes de carbone, et $R_1$ et $R_2$ représentent un hydrogène, caractérisé en ce qu'on utilise des produits de départ correspondants.

7. Procédé selon la revendication 1 de préparation de composés de formule I, où le radical phényle portant le groupe amide et le groupe hydroxy est lié de préférence dans sa position 4 et surtout dans sa position 5 avec le reste de la molécule, caractérisé en ce qu'on utilise des produits de départ correspondants.

8. Procédé de préparation de 1-[2-(3-carbamoyl-4-hydroxyphénoxy)-éthylamino]-3-[4-(2-méthoxy-éthoxy)-phénoxy]-2-propanol selon la revendication 1, caractérisé en ce qu'on utilise le 1-{N-[2-(3-carbamoyl-4-hydroxy-phénoxy)-éthyl]-benzylamino}-3-[4-(2-méthoxyéthoxy)-phénoxy]-2-propanol comme produit de départ.

9. Procédé de préparation de 1-[2-(3-carbamoyl-4-hydroxy-phénoxy)-éthylamino]-3-[4-(2-méthoxyéthoxy)-phénoxy]-2-propanol selon la revendication 1, caractérisé en ce qu'on utilise le 1-[4-(2-méthoxyéthoxy)-phénoxy]-3-amino-2-propanol et le 5-(2-brométhoxy)-salicylamide comme produits de départ.

10. Procédé de préparation de 1-[2-(3-carbamoyl-4-hydroxy-phénoxy)-éthylamino]-3-[4-(2-méthoxyéthoxy)-phénoxy]-2-propanol selon la revendication 1, caractérisé en ce qu'on utilise le (2,3-dihydro-2,2-diméthyl-4H-1,3-benzoxazin-4-on-6-yloxy)-acétaldéhyde et le 1-amino-3-[4-(2-méthoxyéthoxy)-phénoxy]-2-propanol comme produits de départ.

11. Procédé de préparation de 1-[2-(3-carbamoyl-4-hydroxy-phénoxy)-éthylamino]-3-]4-(2-méthoxy-éthoxy)-phénoxy]-2-propanol selon la revendication 1, caractérisé en ce qu'on utilise le 1-[2-(3-cyano-4-hydroxy-phénoxy)-éthyl-amino]-3-[4-(2-méthoxy-éthoxy)-phénoxy]-2-propanol comme produit de départ.

12. Procédé de préparation de 1-(2-allyloxyphénoxy)-3-[2-(3-carbamoyl-4-hydroxy-phénoxy)-éthylamino]-2-propanol selon la revendication 1, caractérisé en ce qu'on utilise le 1-(2-allyloxy-phénoxy)-3-amino-2-propanol et la 6-(2-brométhoxy)-2,3-dihydro-2,2-diméthyl-4H-1,3-benzoxazin-4-one comme produits de départ.

13. Procédé de préparation de 1-(2-allyloxy-phénoxy)-3-[2-(3-carbamoyl-4-hydroxy-phénoxy)-éthylamino]-2-propanol selon la revendication 1, caractérisé en ce qu'on utilise le 1-(2-allyloxy-phénoxy)-3-amino-2-propanol et le 5-(2-brométhoxy)-salicylamide comme produits de départ.

14. Procédé de préparation de 1-(2-allyloxy-phénoxy)-3-[2-(3-carbamoyl-4-hydroxy-phénoxy)-éthylamino]-2-propanol selon la revendication 1, caractérisé en ce qu'on utilise le [2-(3-époxy-propoxy)-phényl]-2-allyléther et le 5-(2-amino-éthoxy)-salicylamide comme produits de départ.

15. Procédé de préparation de 1-[2-(3-carbamoyl-4-hydroxy-phénoxy)-éthylamino]-3-(4-hydroxy-phénoxy)-2-propanol selon la revendication 1, caractérisé en ce qu'on utilise le 1-{N-[2-(3-carbamoyl-4-hydroxy-phénoxy)-éthyl]-benzyl-amino}-3-(4-benzyloxy-phénoxy)-2-propanol comme produit de départ.

16. Procédé de préparation de 4-{2-hydroxy-3-[3-carbamoyl-4-hydroxy-phénoxy)-éthylamino]-propoxy}-phényl-acétamide selon la revendication 1, caractérisé en ce qu'on utilise le 1-{N-[2-(3-carbamoyl-4-hydroxy-phénoxy)-éthyl]-benzylamino}-3-(4-carbamoylméthyl-phénoxy)-2-propanol comme produit de départ.

17. Procédé de préparation de 1-[2-(3-carbamoyl-4-hydroxy-phénoxy)-éthylamino]-3-(4-carbamoylméthyl-phénoxy)-2-propanol selon la revendication 1, caractérisé en ce qu'on utilise le (4-carbamoylméthyl-phénoxy)-2,3-époxy-propane et le 5-(2-aminoéthoxy)-salicylamide comme produits de départ.

18. Procédé de préparation de 1-[2-(3-carbamoyl-4-hydroxy-phénoxy)-éthylamino]-3-[2-pyrrol-1-yl)-phénoxy]-2-propanol selon la revendication 1, caractérisé en ce qu'on utilise le 1-{N-[2-(3-carbamoyl-4-hydroxy-phénoxy)-éthyl]-benzylamino}-3-[2-(pyrrol-1-yl)-phénoxy]-2-propanol comme produit de départ.

19. Procédé de préparation de 1-[2-(3-carbamoyl-4-hydroxy-phénoxy)-éthylamino]-3-{4-[2-(méthoxycarbonylamino)-éthoxy]-phénoxy]-2-propanol selon la revendication 1, caractérisé en ce qu'on utilise le 1-{N-[2-(3-carbamoyl-4-hydroxy-phénoxy)-éthyl]-benzylamino}-3-[4-(2-méthoxycarbonylamino)-éthoxy]-phénoxy}-2-propanol comme produit de départ.

20. Procédé de préparation de 1-[2-(3-carbamoyl-4-hydroxy-phénoxy)-éthylamino]-3-(2-méthyl-indol-4-yloxy)-2-propanol selon la revendication 1, caractérisé en ce qu'on utilise le 1-{N-[2-(3-carbamoyl-4-hydroxy-phénoxy)-éthyl]-benzylamino}-3-(2-méthyl-indol-4-yloxy)-2-propanol comme produit de départ.

21. Procédé de préparation de 4-{3-[2-(3-carbamoyl-4-hydroxy-phénoxy)-éthylamino]-2-hydroxy-propoxy}-benzimidazol-2-one selon la revendication 1, caractérisé en ce qu'on utilise la N-[2-(4-hydroxy-3-carbamoyl-phénoxy)-éthyl]-N-[3-(2-oxo-benzimidazol-4-yloxy)-2-hydroxypropyl]-N-benzylamine comme produit de départ.

22. Procédé selon l'une des rev. 1—21, caractérisé en ce qu'on transforme un composé de formule I obtenu en un sel d'addition d'acide non toxique pharmaceutiquement acceptable.